(19) Europäisches Patentamt
European Patent Office
Office européen des brevets

(11) EP 2 683 714 B1

(12) **EUROPEAN PATENT SPECIFICATION**

(45) Date of publication and mention of the grant of the patent:
**09.01.2019 Bulletin 2019/02**

(21) Application number: **12710465.1**

(22) Date of filing: **09.03.2012**

(51) Int Cl.:
***C07D 413/14*** (2006.01) ***C07D 498/04*** (2006.01)

(86) International application number:
**PCT/EP2012/054181**

(87) International publication number:
**WO 2012/120141 (13.09.2012 Gazette 2012/37)**

(54) **II (PI)-CONJUGATED FLUOROIONOPHORES AND METHOD FOR DETERMINING AN ALKALI ION**

II (PI)-KONJUGIERTE FLUOROIONOPHOREN UND VERFAHREN ZUR BESTIMMUNG EINES ALKALIIONS

FLUOROIONOPHORES II (PI)-CONJUGUÉS ET MÉTHODE DE DOSAGE D’UN ION ALCALIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2011 EP 11157413**
**18.03.2011 EP 11158899**

(43) Date of publication of application:
**15.01.2014 Bulletin 2014/03**

(73) Proprietor: **Universität Potsdam**
**14469 Potsdam (DE)**

(72) Inventors:
- **AST, Sandra**
**14471 Potsdam (DE)**
- **HOLDT, Hans-Jürgen**
**19412 Jülchendorf (DE)**

(74) Representative: **Schubert, Klemens et al**
**Müller & Schubert Patentanwälte**
**Schlüterstrasse 37**
**10629 Berlin (DE)**

(56) References cited:
**WO-A2-2007/044866**

- **CARPENTER, RICHARD D. ET AL: "Function-Oriented Synthesis of a Didesmethyl Triazacryptand Analogue for Fluorescent Potassium Ion Sensing", EUROPEAN JOURNAL OF ORGANIC CHEMISTRY , (7), 1242-1248, S1242/1-S1242/18 CODEN: EJOCFK; ISSN: 1434-193X, 2011, XP002674354,**
- **NAZAROV, V. B. ET AL: "Complex formation of 2,2-diphenyl-2H-benzo[f]chromene containing the aza-18-crown-6-ether fragment in the polymeric layer", RUSSIAN CHEMICAL BULLETIN (TRANSLATION OF IZVESTIYA AKADEMII NAUK, SERIYA KHIMICHESKAYA) , 52(12), 2661-2667 CODEN: RCBUEY; ISSN: 1066-5285, 2003, XP002674355,**
- **MAGZOUB, MAZIN ET AL: "Millisecond Association Kinetics of K+ with Triazacryptand-Based K+ Indicators Measured by Fluorescence Correlation Spectroscopy", JOURNAL OF PHYSICAL CHEMISTRY B , 110(42), 21216-21221 CODEN: JPCBFK; ISSN: 1520-6106, 2006, XP002674356,**
- **NAMKUNG, WAN ET AL: "In Situ Measurement of Airway Surface Liquid [K+] Using a Ratioable K+-sensitive Fluorescent Dye", JOURNAL OF BIOLOGICAL CHEMISTRY , 284(23), 15916-15926 CODEN: JBCHA3; ISSN: 0021-9258, 2009, XP002674357,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to π-conjugated fluoroionophores, methods for their preparation, and their use and a method for determining an alkali ion.

## Background of the invention

EP 0 881 487 A2 discloses a method for the determination of an alkali ion in biological samples like blood. The determination is performed by using a compound having a luminophoric and an ionophoric moiety. WO 2007/0044866 A2 describes chromoionophoric compounds comprising a triazaeryptand (TAC) $K^+$ ionophore conjugated to at least a first chromophoric moiety. These water-soluble fluorescent compounds are used for the detection of potassium. US 6, 211,359 B1 discloses a triaza-cryptand useful as a luminescence indicator for alkali ions. US 2007/0259443 A1 and US 2007/0259444 A1 disclose chromoionophores and a method of determining potassium ions.

Recently 1,2,3-triazol-1,4-diyl fluoroionophores for $Zn^{2+}$,[1] $Ni^{2+}$,[2] $Cu^{2+}$,[3] $Hg^{2+}$,[4] $Ag^+$,[4] and $Al^{3+}$,[5] were generated by Cu(I) catalyzed reaction between an azide and an alkyne (CuAAC). In these fluoroionophores the 1,2,3-triazol-1,4-diyls serve in addition to the conventional function as covalent linkers as both, chelating ligand of the metal ions and as electronic transmitter of a coordination event to the fluorophore.[1a] Only $Zn^{2+}$ and $Al^{3+}$ could be detected by fluorescent enhancement, whereas the other metal ions were analysed through fluorescence quenching. In these known 1,2,3-triazol-1,4-diyl fluoroionophores, no electronically conjugated signal transduction chain: ionophore - 1,2,3-triazole - fluorophore, is used. Either receptor and 1,2,3-triazole are connected through a deconjugated linker[1,2] or the triazole and the fluorophor-ic group are deconjugated.[3,4,5]

In a systematic investigation Diederich et al. showed the capacity of the 1,2,3-triazol-diyls as active π-linkers in Charge Transfer (CT) chromophores.[6] Such push-pull chromophores are only weak or even non fluorescent.

The compounds and methods known show several disadvantages when used for the determination of potassium especially in biological samples like blood. A disadvantage of the known methods is that the cations can only be measured within a small concentration range. Further these methods also lack sensitivity and selectivity especially in the presence of sodium.

Therefore, it is an object of the present invention to provide a method for the determination of alkali metal cations in biological samples within a broad concentration range, and with improved sensitivity and specificity.

## Brief description of the invention

The inventors found that in simple CuAAC-generated systems the electronic conjugation of an alkali metal ion selective receptor which could be a *N*-phenylaza-crown ether or a *N*-phenylaza-cryptand, and a fluorophore through a 1,2,3-triazol π-linker results in a perfect signal transduction chain for a simultaneous fluorescence quenching *via* both, an internal charge transfer (ICT) and a photoinduced electron transfer (PET). The fluorescence of the original fluorophore is basically nullified. Coordination of an alkali metal ion to the anilino-donor of the ionophoric unit interrupts this π-conjugation as well as the PET, resulting in a revival of the fluorescence, proportional to the metal ion concentration. This concentration dependant fluorescence enhancement allows the sensing of $K^+$ and $Na^+$ under simulated physiological conditions.

The problem of the invention is solved by the development of novel triazole-based fluoroionophores which show significant selectivity for $Na^+$ and $K^+$ in water though bearing a simple crown-ether-receptor or cryptand-receptor unit.

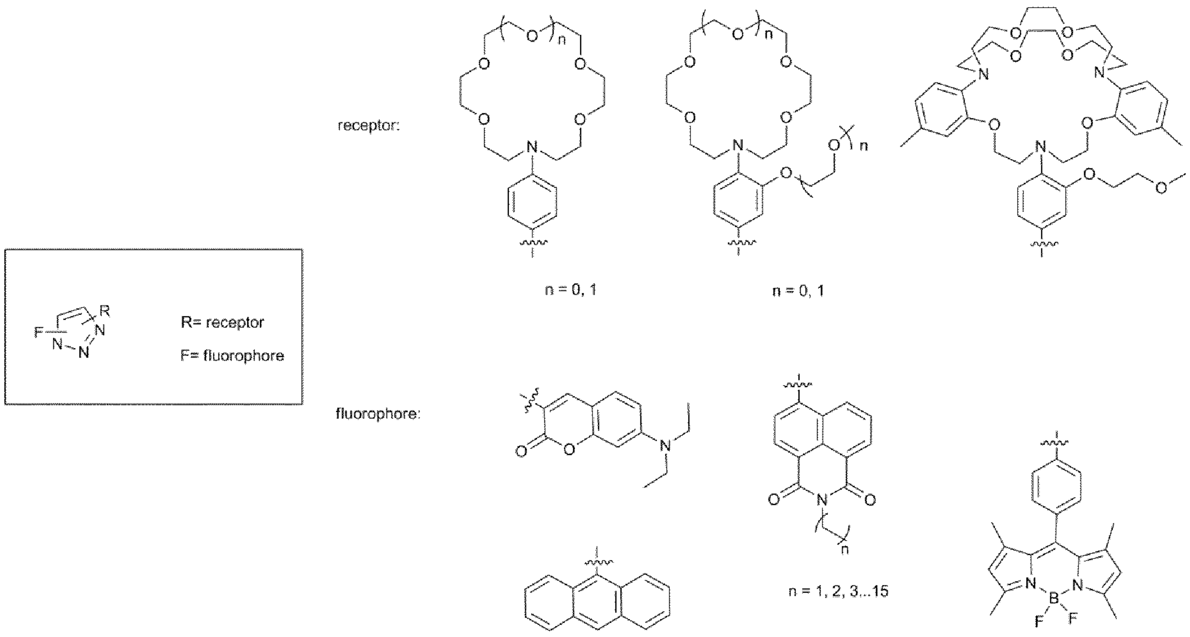


**Scheme 1** Π-conjugated 1,2,3-triazole-based fluoroionophores

To the best of the inventors knowledge no 1,2,3-triazole-based linker between receptor and fluorophore, giving a conjugated system exists to now.

Variation of the ionophoric unit offers a broad spectrum of detectable $K^+$ and $Na^+$ -concentrations, ranging from high concentration around 800 mM down to very low concentrations around 3 mM.

The new alkali metal ion 1,2,3-triazole-based fluoroionophores have great potential for application in fluorescent optode systems based blood analyzing equipment for methods and kits for the determination of $K^+$ and $Na^+$ concentrations in biological systems, either in vitro or in vivo, using embodiments of the fluoroionophores according to the present invention.

The problem is solved according to the invention by providing a fluoroiono-phoric compound of the general formula I

**Ionophore – π-Linker - Fluorophore (I)**

wherein

the Ionophore is an anilino containing crown ether or cryptand with one or more anilino donor moieties as electron donors, forming a stable complex with an alkali metal ion

the π-Linker is a heteroaromatic conjugative linking moiety, and is selected from the group consisting of isomeric disubstituted 1,2,3-triazoles, preferably:

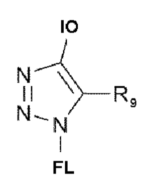 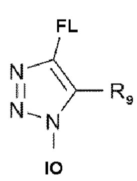 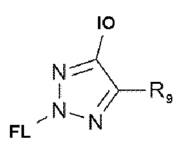 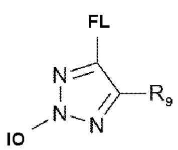 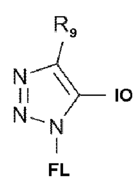 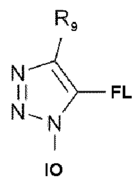

wherein
IO is the ionophore;
FI is the fluorophore;
$R_9$ is selected from hydrogen, halogen, nitro, amino, hydroxyl, lower alkyl, selected from methyl, ethyl, propyl, isopropyl, butyl, sec-butyl, tert-butyl, pentyl, isopentyl, neopentyl, hexyl, isohexyl or neohexyl and lower alkoxy,

selected from -O-methyl, -O-ethyl, -O-propyl, -O-isopropyl, -O-butyl, -O-sec-butyl, -O-tert-butyl, -O-pentyl, -O-isopentyl, -O-neopentyl, -O-hexyl, -O-isohexyl or -O-neohexyl, optionally substituted with halogen, nitro, amino, hydroxyl or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl,-O-butyl, -O-sec-butyl, -O-tert-butyl, -O-pentyl, -O-isopentyl, -O-neopentyl, -O-hexyl, -O-isohexyl or -O-neohexyl, and
the Fluorophore is an electron acceptor moiety.

Preferred are compounds according to the invention, wherein the ionophore is selected from the group consisting of

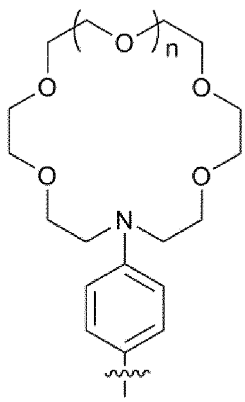

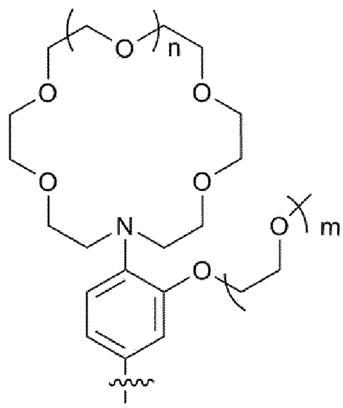

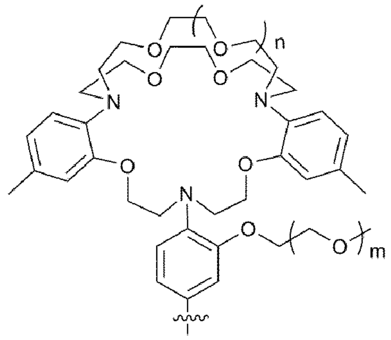

wherein

n is a number selected from 0 and 1,
m is a number selected from 0, 1, and 2, and
wherein the phenyl ring is optionally substituted with halogen, nitro, amino, hydroxyl, lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl, - O-butyl, -O-sec-butyl, -O-tert-butyl, -O-pentyl, -O-isopentyl, -O-neopentyl, -O-hexyl, -O-isohexyl or -O-neohexyl, wherein the lower alkyl or lower alkoxy are optionally substituted with halogen, nitro, amino, hydroxyl or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl, -O-butyl, -O-sec-butyl, -O-tert-butyl, -O-pentyl, -O-isopentyl, -O-neopentyl, -O-hexyl, -O-isohexyl or -O-neohexyl, and
wherein the phenyl ring may optionally be a part of a condensed aromatic system, that is optionally substituted with halogen, nitro, amino, hydroxyl, or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl, -O-butyl,-O-sec-butyl, -O-tert-butyl, -O-pentyl, -O-isopentyl, -O-neopentyl, -O-hexyl,-O-isohexyl or -O-neohexyl, or phenyl, optionally substituted with halogen, nitro, amino, hydroxyl or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl, -O-butyl, -O-sec-butyl, -O-tert-butyl, -O-pentyl, -O-isopentyl, -O-neopentyl, -O-hexyl, -O-isohexyl or -O-neohexyl.

Very specially preferred are compounds according to the invention, wherein the π-Linker is selected from the group consisting of substituted 1,4-triazoles, namely

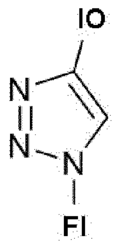

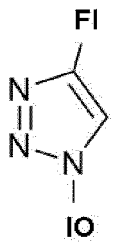

wherein IO is the ionophore and FI is the fluorophore.

Preferred are compounds according to the invention, wherein the fluorophore moiety is represented by the formula

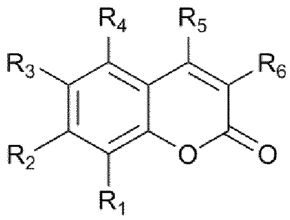

wherein

$R_1$, $R_4$, $R_5$ = H, lower alkyl, selected from methyl, ethyl, propyl, isopropyl, butyl, sec-butyl, tert-butyl, pentyl, isopentyl, neopentyl, hexyl, isohexyl or neohexyl, $CF_3$, MeO, halogen, $NO_2$, CN,

$R_2$, $R_3$ = H, $NH_2$, N(lower alkyl)$_2$, lower alkyl, selected from methyl, ethyl, propyl, isopropyl, butyl, sec-butyl, tert-butyl, pentyl, isopentyl, neopentyl, hexyl, isohexyl or neohexyl, optionally substituted with carboxyl or carbonyl, diethyl amino

$R_6$ = alkinyl, azide.

Preferred are also compounds according to the invention, wherein the fluorophore moiety is selected from the group consisting of

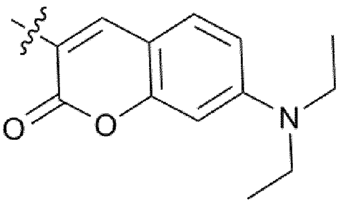

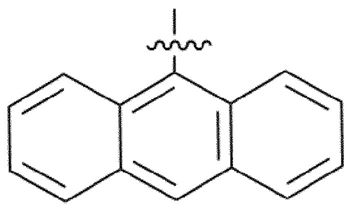

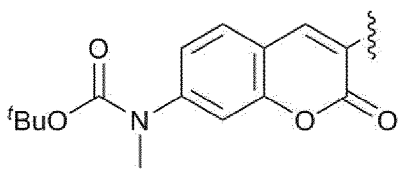

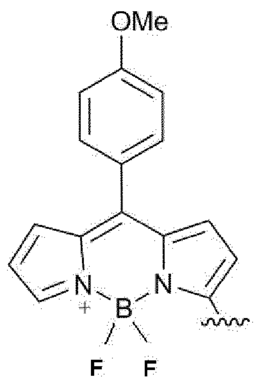

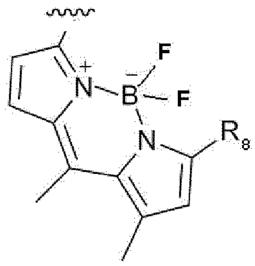

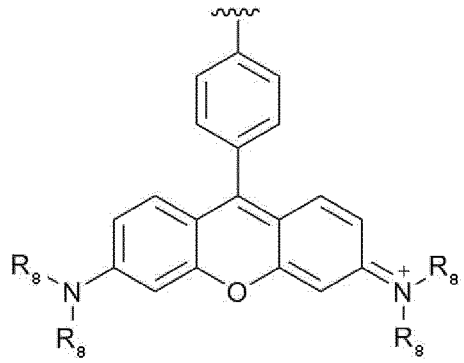

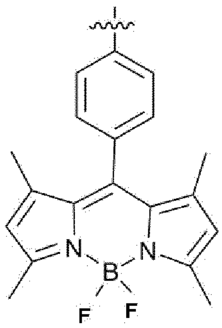

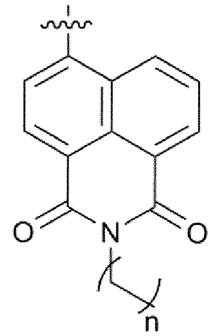

wherein

n is an integer ranging from 0 to 15;
$R_8$ is selected from hydrogen or lower alkyl, selected from methyl, ethyl, propyl, isopropyl, butyl, sec-butyl, tert-butyl, pentyl, isopentyl, neopentyl, hexyl, isohexyl or neohexyl.

Especially preferred are the following compounds of the invention according to general Formula (I), namely

**Coumarin fluoroionophores**

Potassium selective

3-{4-[4-(1,4,7,10,13-pentaoxa-16-azacyclooctadecan-16-yl)phenyl]-1H-1,2,3-triazol-1-yl}-7-(diethylamino)-2H-chromen-2-one

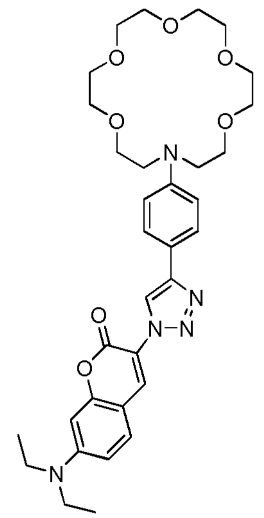

3-{1-[4-(1,4,7,10,13-pentaoxa-16-azacyclooctadecan-16-yl)phenyl]-1H-1,2,3-triazol-4-1-7-(diethylamino)-2H-chromen-2-one

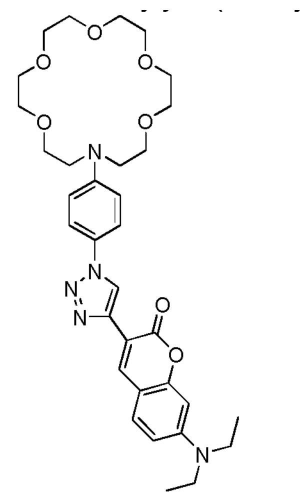

7-(diethylamino)-3-{1-[3-(2-methoxyethoxy)-4-(1,4,7,10,13-pentaoxa-16-azacyclooctadecan-16-yl)phenyl]-1H-1,2,3-triazol-4-l-2H-chromen-2-one

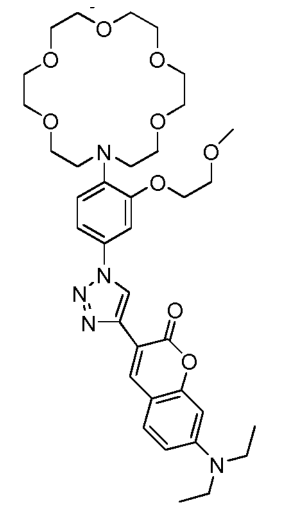

7-(diethylamino)-3-{4-[3-(2-methoxyethoxy)-4-(1,4,7,10,13-pentaoxa-16-azacyclooctadecan-16-yl)phenyl]-1H-1,2,3-triazol-1-yl}-2H-chromen-2-one

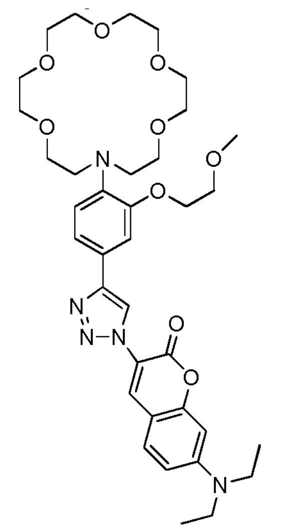

7-(diethylamino)-3-{4-3-[2-methoxyethoxy)-4{bis(4-methylbenzo[5,6,17, 18](O,N,N',O')-]-1,7,16,-triaza-cryptand-[3,2, 1]-phen-4-yl]-1H-1.2.3-triazol-1yl}-2H-chromen-2-one

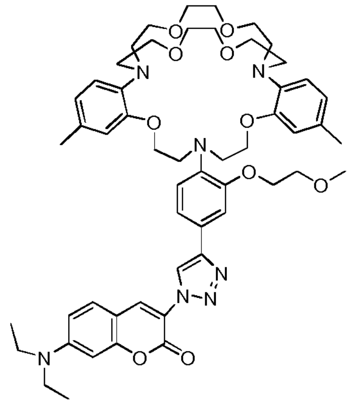

7-(diethylamino)-3-{4-[3-(2-methoxyethoxy]-4{bis(4-methylbenzo[5, 6, 17, 18](O,N, N', O')-]-1,7, 16, -triaza-cryptand-[3, 2, 1]-phen-4-yl]-1H-1.2.3-triazol-11-2H-chromen-2-one

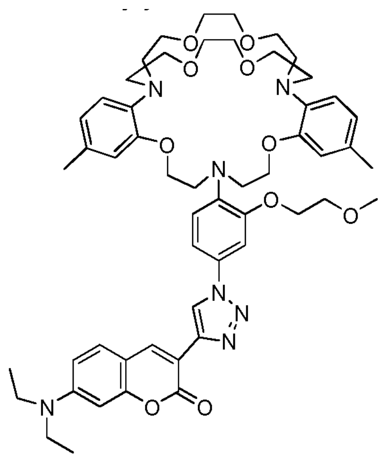

Sodium selective

3-{4-[4-(1,4,7,10-tetraoxa-13-azacyclopentadecan-13-yl)phenyl]-1H-1,2,3-triazol-1-yl}-7-(diethylamino)-2H-chromen-2-one

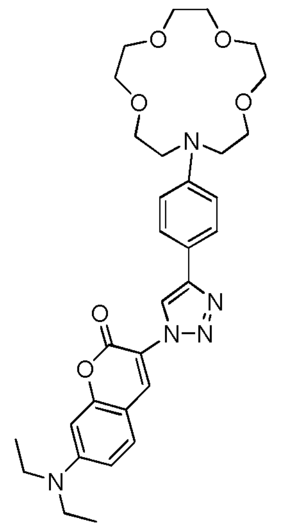

3-{1-[4-(1,4,7,10-tetraoxa-13-azacyclopentadecan-13-yl)phenyl]-1H-1,2,3-triazol-4-yl}-7-(diethylamino)-2H-chromen-2-one

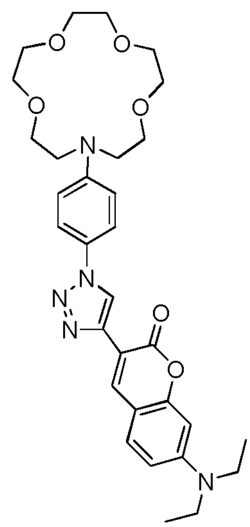

7-(diethylamino)-3-{4-[3-methoxy-4-(1,4,7,10-tetraoxa-13-azacyclopentadecan-13-yl)phenyl]-1H-1,2,3-triazol-1-yl}-yl}-2H-chromen-2-one

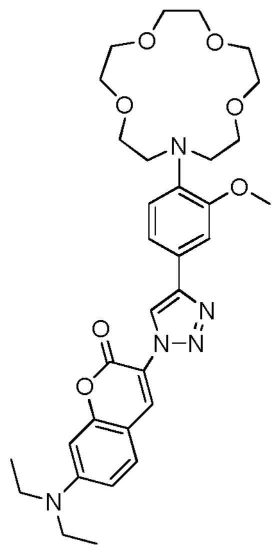

7-(diethylamino)-3-{1-[3-methoxy-4-(1,4,7,10-tetraoxa-13-azacyclopentadecan-13-yl)phenyl]-1H-1,2,3-triazol-4-yl}-2H-chromen-2-one

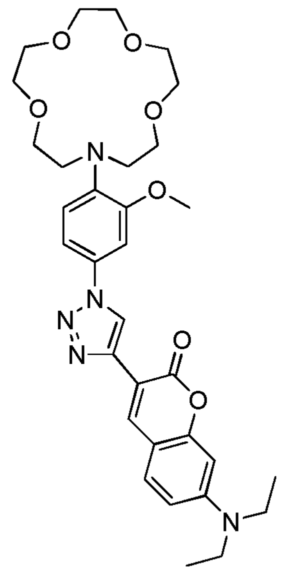

7-(diethylamino)-3-{4-[3-methoxy-4{bis(4-methylbenzo[5, 6, 17, 18](O,N, N', O')-]-1,7,16,-triaza-cryptand-[3,2,1]-phen-4-yl]-1H-1.2.3-triazol-1yl}-2H-chromen-2-one

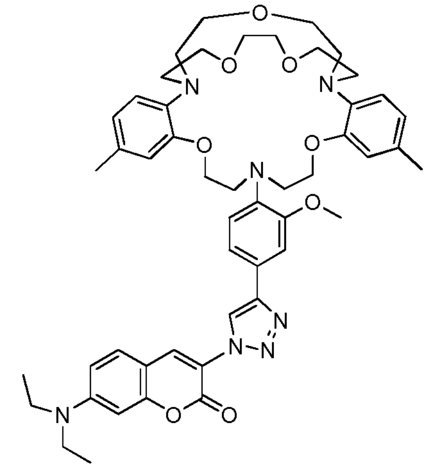

7-(diethylamino)-3-{1-[3methoxy-4{bis(4-methylbenzo[5, 6, 17, 18](O,N, N', O')-]-1,7,16,-triaza-cryptand-[3,2,1]-phen-1-yl]-1H-1,2.3-triazol-1yl}-2H-chromen-2-one

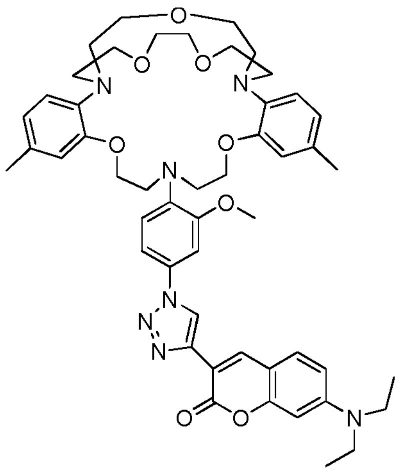

**Naphthalimid fluoroionophores**

Potassium selective

6-{4-[4-(1,4,7,10,13-pentaoxa-16-azacyclooctadecan-16-yl)phenyl]-1H-1,2,3-triazol-1-yl}-2-ethyl-1H-benzo[de]isoquinoline-1,3(2H)-dione

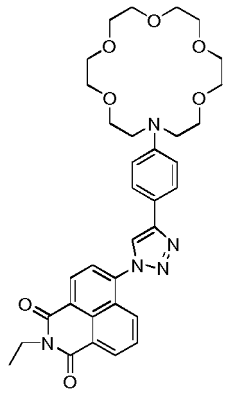

6-{1-[4-(1,4,7,10,13-pentaoxa-16-azacyclooctadecan-16-yl)phenyl]-1H-1,2,3-triazol-4-yl}-2-ethyl-1H-benzo[de]isoquinoline-1,3(2H)-dione

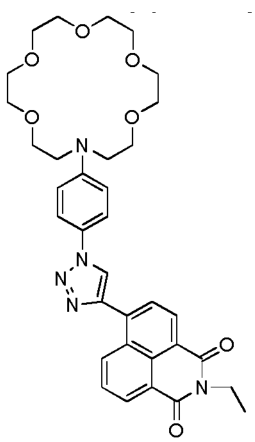

2-ethyl-6-{4-[3-(2-methoxyethoxy)-4-(1,4,7,10,13-pentaoxa-16-azacyclooctadecan-16-yl)phenyl]-1H-1,2,3-triazol-1-yl}-yl}-1H-benzodeisouinoline-1,32H-dione

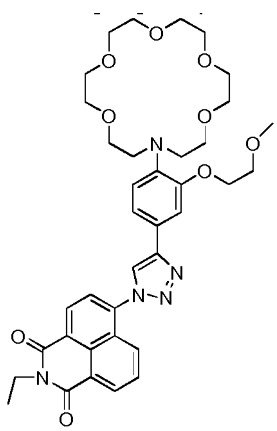

2-ethyl-6-{1-[3-(2-methoxyethoxy)-4-(1,4,7,10,13-pentaoxa-16-azacyclooctadecan-16-yl)phenyl]-1H-1,2,3-triazol-4-yl}-1H-benzo[de]isoquinoline-1,3(2H)-dione

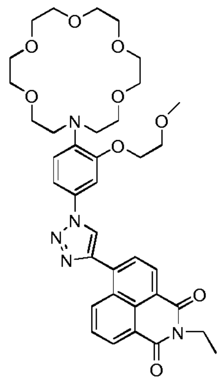

2-ethyl-6-{4-[3-(2-methoxyethoxy)-4{bis(4-methylbenzo[5, 6, 17, 18](O,N, N', O')-]-1,7,16,-triaza-cryptand-[3,2,2]-phen-1-yl]-1H-1.2.3-triazol 1-yl}-1H-benzo[de]isoquinoline-1,3(2H)-dione

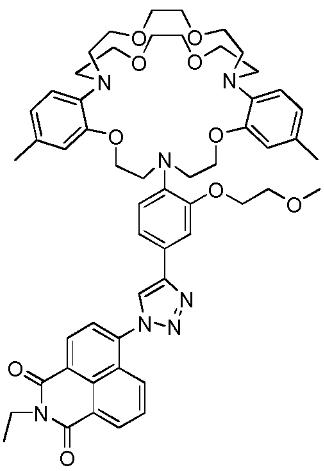

2-ethyl-6-{1-[3-(2-methoxyethoxy)-4{bis(4-methylbenzo[5, 6, 17, 18](O,N, N', O')-]-1,7,16,-triaza-cryptand-[3,2,2]-phen-4-yl]-1H-1.2.3-triazol1-yl}-1H-benzo[de]isoquinoline-1,3(2H)-dione

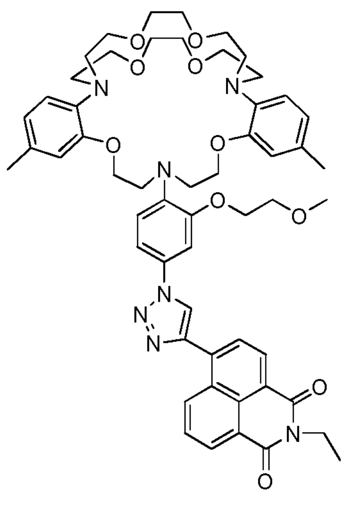

Sodium selective

6-{4-[4-(1,4,7,10-tetraoxa-13-azacyclopentadecan-13-yl)phenyl]-1H-1,2,3-triazol-1-yl}-2-ethyl-1H-benzo[de]isoquinoline-1,3(2H)-dione

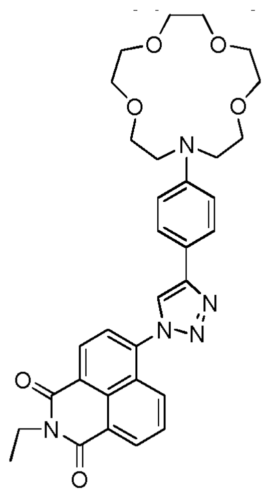

6-{1-[4-(1,4,7,10-tetraoxa-13-azacyclopentadecan-13-yl)phenyl]-1H-1,2,3-triazol-4-yl}-2-ethyl-1H-benzo[de]isoquinoline-1,3(2H)-dione

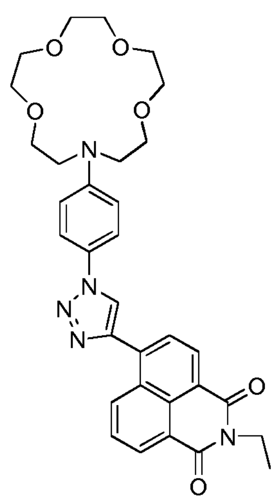

2-ethyl-6-{4-[3-methoxy-4-(1,4,7,10-tetraoxa-13-azacyclopentadecan-13-yl)phenyl]-1H-1,2,3-triazol-1-yl}-1H-benzo[de]isoquinoline-1,3(2H)-dione

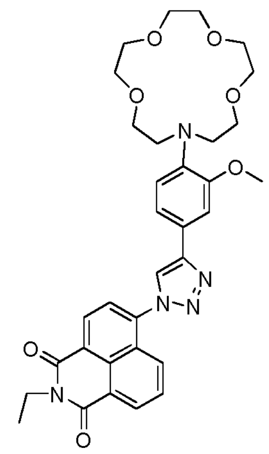

2-ethyl-6-{1-[3-methoxy-4-(1,4,7,10-tetraoxa-13-azacyclopentadecan-13-yl)phenyl]-1H-1,2,3-triazol-4-yl}-1H-benzo[de]isoquinoline-1,3(2H)-dione

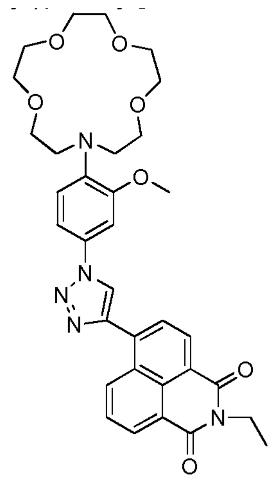

2-ethyl-6-{4-[3-methoxy-4{bis(4-methylbenzo[5, 6, 17, 18](O,N, N', O')-]-1,7, 16,-triaza-cryptand-[3,2,1]-phen-1-yl]-1H-1.2.3-triazol1-yl}-1H-benzo[de]isoquinoline-1,32H-dione

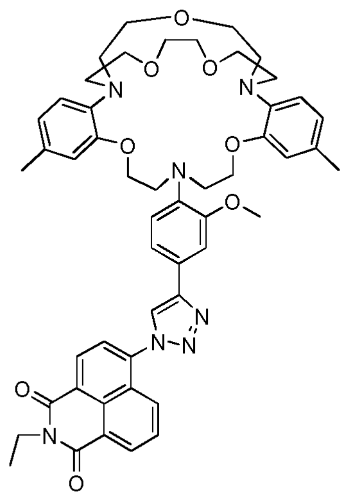

2-ethyl-6-{1-[3-methoxy-4{bis(4-methylbenzo[5, 6, 17, 18](O,N, N', O')-]-1,7, 16,-triaza-cryptand-[3,2,1]-phen-4-yl]-1H-1.2.3-triazol1-yl}-1H-benzo[de]isoquinoline-1,32H-dione

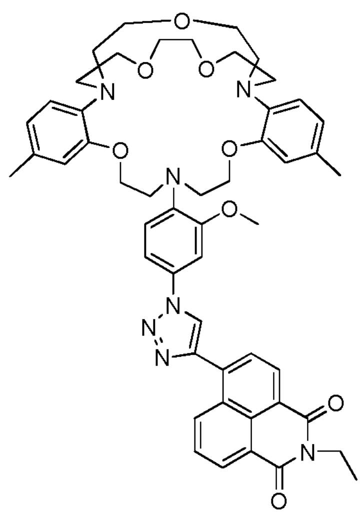

**7-BODIPY fluoroionophores**

Potassium selective

7-{4-[4-(1,4,7,10,13-pentaoxa-16-azacyclooctadecan-16-yl)phenyl]-1H-1,2,3-triazol-1-yl}-5,5-difluoro-1,3,9,10-tetramethyl-5H-dipyrrolo[1,2-c:1',2'-f][1,3,2]diazaborinin-4-ium-5-uide

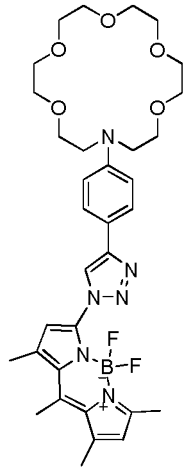

7-{1-[4-(1,4,7,10,13-pentaoxa-16-azacyclooctadecan-16-yl)phenyl]-1H-1,2,3-triazol-4-yl}-5,5-difluoro-1,3,9,10-tetramethyl-5H-dipyrrolo[1,2-c:1',2'-1,3,2diazaborinin-4-ium-5-uide

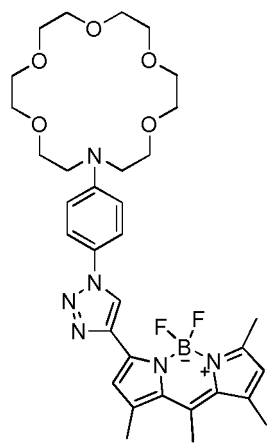

5,5-difluoro-7-{4-[3-(2-methoxyethoxy)-4-(1,4,7,10,13-pentaoxa-16-azacyclooctadecan-16-yl)phenyl]-1H-1,2,3-triazol-1-yl}-1,3,9,10-tetramethyl-5H-dipyrrolo[1,2-c:1',2'-f][1,3,2]diazaborinin-4-ium-5-uide

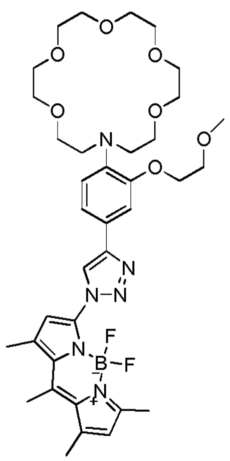

5,5-difluoro-7-{1-[3-(2-methoxyethoxy)-4-(1,4,7,10,13-pentaoxa-16-azacyclooctadecan-16-yl)phenyl]-1H-1,2,3-triazol-4-yl}-1,3,9,10-tetramethyl-5H-dipyrrolo[1,2-c:1',2'-f][1,3,2]diazaborinin-4-ium-5-uide

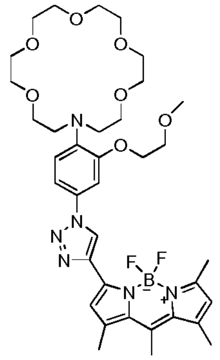

5,5-difluoro-7 {4-[3-(2-methoxyethoxy]-4(bis(4-methylbenzo[5, 6, 17, 18](O, N,N',O')-]-1,7,16,-triaza-cryptand-[3,2, 2]-phen-4-yl]-1H-1.2.3-triazol-1-yl}-4-1,3,9,10-tetramethyl-5H-dipyrrolo[1,2-c:1 ',2'-f][1,3,2]diazaborinin-4-ium-5-uide

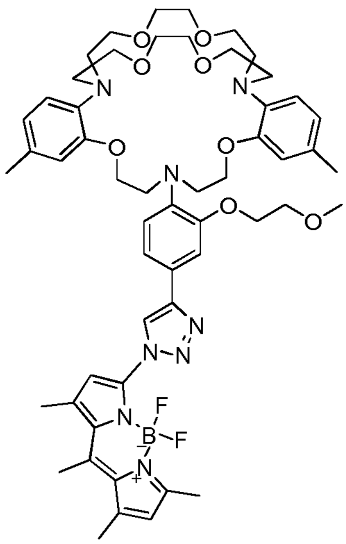

5,5-difluoro-7 {1-[3-(2-methoxyethoxy]-4(bis(4-methylbenzo[5, 6, 17, 18](O, N,N',O')-]-1,7,16,-triaza-cryptand-[3,2, 2]-phen-1-yl]-1H-1.2.3-triazol-1-yl}-4-1,3,9,10-tetramethyl-5H-dipyrrolo[1,2-c:1',2'-f][1,3,2]diazaborinin-4-ium-5-uide

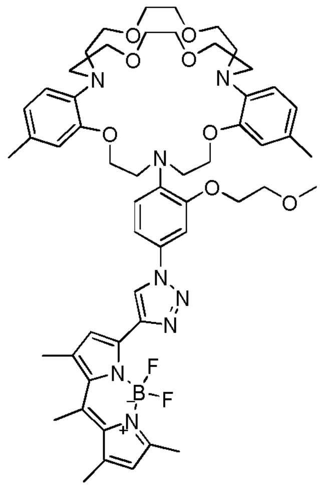

Sodium selective

7-{4-[4-(1,4,7,10-tetraoxa-13-azacyclopentadecan-13-yl)phenyl]-1H-1,2,3-triazol-1-yl}-5,5-difluoro-1,3,9,10-tetramethyl-5H-dipyrrolo[1,2-c:1',2'-f][1,3,2]diazaborinin-4-ium-5-uide

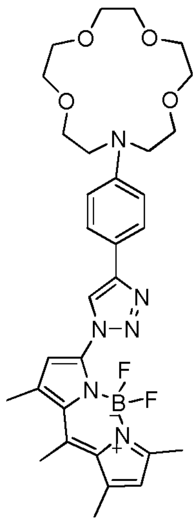

7-{1-[4-(1,4,7,10-tetraoxa-13-azacyclopentadecan-13-yl)phenyl]-1H-1,2,3-triazol-4-yl}-5,5-difluoro-1,3,9,10-tetramethyl-5H-dipyrrolo[1,2-c:1 ',2'-f][1,3,2]diazaborinin-4-ium-5-uide

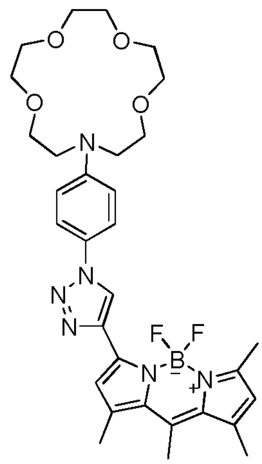

5,5-difluoro-7-{4-[3-methoxy-4-(1,4,7,10-tetraoxa-13-azacyclopentadecan-13-yl)phenyl]-1H-1,2,3-triazol-1-yl}-1,3,9,10-tetramethyl-5H-dipyrrolo[1,2-c:1',2'-f][1,3,2]diazaborinin-4-ium-5-uide

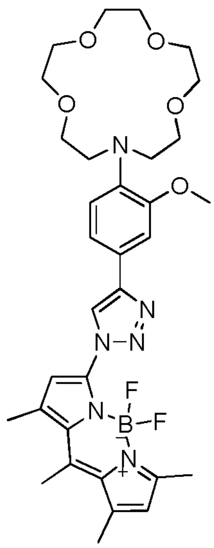

5,5-difluoro-7-{1-[3-methoxy-4-(1,4,7,10-tetraoxa-13-azacyclopentadecan-13-yl)phenyl]-1H-1,2,3-triazol-4-yl}-1,3,9,10-tetramethyl-5H-dipyrrolo[1,2-c:1',2'-f][1,3,2]diazaborinin-4-ium-5-uide

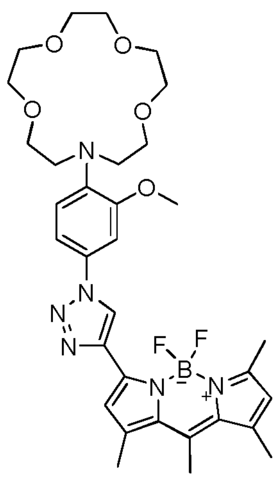

5,5-difluoro-7{4-[3-methoxy-4(bis(4-methylbenzo[5,6,17,18](O,N,N',O')-]-1,7,16,-triaza-cryptand-[3,2,1]-phen-4-yl]-1H-1.2.3-triazol-1-yl}-4-1,3,9,10-tetramethyl-5H-dipyrrolo[1,2-c:1',2'-f][1,3,2]diazaborinin-4-ium-5-uide

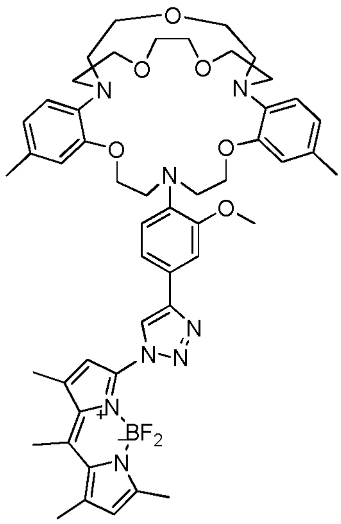

5,5-difluoro-7{1-[3-methoxy-4(bis(4-methylbenzo[5,6,17,18](O,N,N',O')-]-1,7,16, -triaza-cryptand-[3, 2, 2]-phen-1-yl]-1H-1.2.3-triazol-1-yl}-4-1,3,9,10-tetramethyl-5H-dipyrrolo[1,2-c:1',2'-f][1,3,2]diazaborinin-4-ium-5-uide

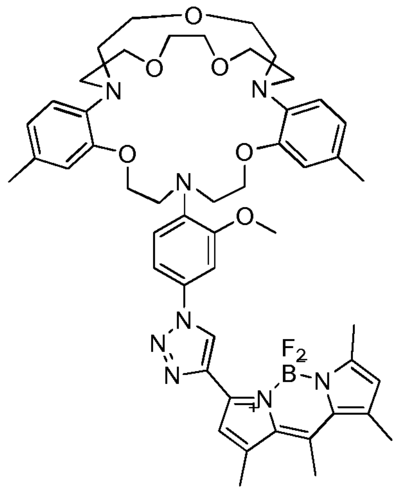

**10-phenyl-BODIPY-fluoroionophores**

Potassium selective

10-(4-{4-[4-(1,4,7,10,13-pentaoxa-16-azacyclooctadecan-16-yl)phenyl]-1H-1,2,3-triazol-1-yl}phenyl)-5,5-difluoro-1,3,7,9-tetramethyl-5H-dipyrrolo[1,2-c:1',2'-f][1,3,2]diazaborinin-4-ium-5-uide

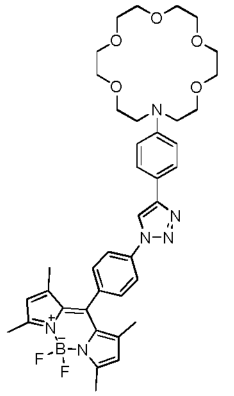

10-(4-{1-[4-(1,4,7,10,13-pentaoxa-16-azacyclooctadecan-16-yl)phenyl]-1H-1,2,3-triazol-4-yl}phenyl)-5,5-difluoro-1,3,7,9-tetramethyl-5H-dipyrrolo[1,2-c:1',2'-f][1,3,2]diazaborinin-4-ium-5-uide

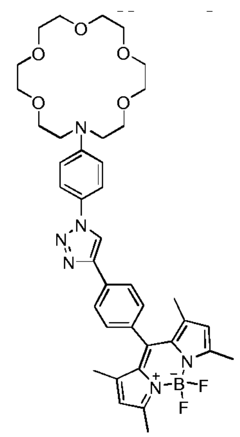

5,5-difluoro-10-(4-{4-[3-(2-methoxyethoxy)-4-(1,4,7,10,13-pentaoxa-16-azacyclooctadecan-16-ylphenyl]-1H-1,2,3-triazol-1-yl}phenyl)-1,3,7,9-tetramethyl-5H-dipyrrolo[1,2-c:1',2'-f][1,3,2]diazaborinin-4-ium-5-uide

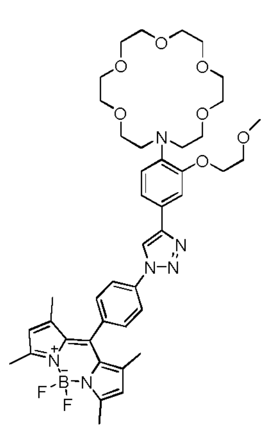

5,5-difluoro-10-(4-{1-[3-(2-methoxyethoxy)-4-(1,4,7,10,13-pentaoxa-16-azacyclooctadecan-16-yl)phenyl]-1H-1,2,3-triazol-4-yl}phenyl)-1,3,7,9-tetramethyl-5H-dipyrrolo[1,2-c:1',2'-f][1,3,2]diazaborinin-4-ium-5-uide

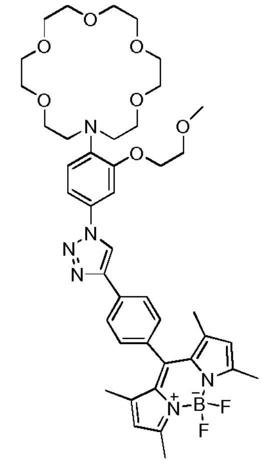

5,5-difluoro-10-(4-{4-[3-(2-methoxyethoxy)-4(bis(4-methylbenzo[5, 6, 17, 18](O,N,N',O')-]-1,7,16,-triaza-cryptand-[3,2, 2]-phen-4-yl]-1H-1.2.3-triazol1-yl}1,3,7,9-tetramethyl-5H-dipyrrolo)(1,2-c:1',2'-f)(1,3,2)diazaborinin-4-ium-5-uide

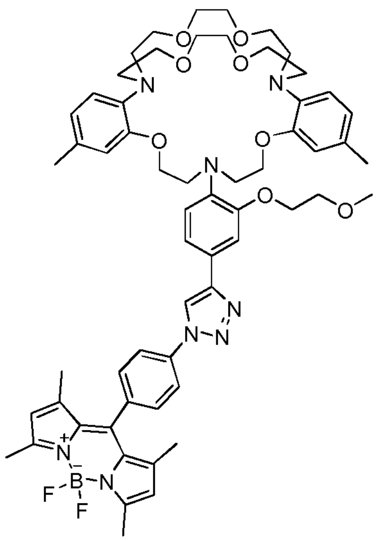

5,5-difluoro-10-(4-{1-[3-(2-methoxyethoxy)-4(bis(4-methylbenzo[5,6,17, 18](O,N,N',O')-]-1,7,16,-triaza-cryptand-[3,2, 2]-phen-4-yl]-1H-1.2.3-triazol 4-yl}1,3,7,9-tetramethyl-5H-dipyrrolo)(1,2-c:1',2'-f)(1,3,2)diazaborinin-4-ium-5-uide

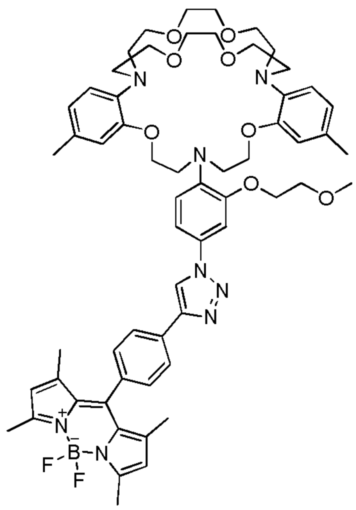

Sodium selective

10-(4-{4-[4-(1,4,7,10-tetraoxa-13-azacyclopentadecan-13-yl)phenyl]-1H-1,2,3-triazol-1-yl}phenyl)-5,5-difluoro-1,3,7,9-tetramethyl-5H-dipyrrolo[1,2-c:1',2'-f][1,3,2]diazabohnin-4-ium-5-uide

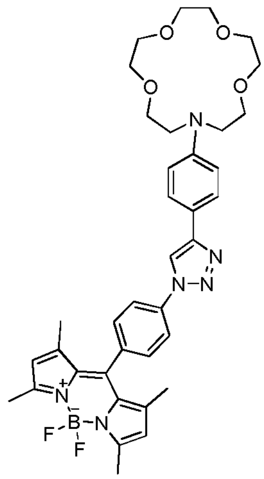

10-(4-{1-[4-(1,4,7,10-tetraoxa-13-azacyclopentadecan-13-yl)phenyl]-1H-1,2,3-triazol-4-yl}phenyl)-5,5-d ifluoro-1,3,7,9-tetramethyl-5H-dipyrrolo[1,2-c:1',2'-f][1,3,2]diazaborinin-4-ium-5-uide

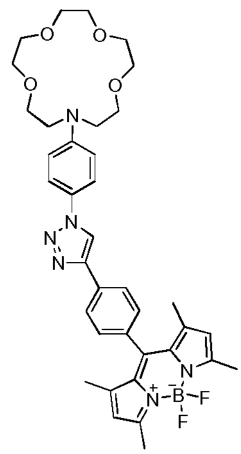

5,5-difluoro-10-(4-{4-[3-methoxy-4-(1,4,7,10-tetraoxa-13-azacyclopentadecan-13-yl)phenyl]-1H-1,2,3-triazol-1-yl}phenyl)-1,3,7,9-tetramethyl-5H-dirrolo[1,2-c:1',2'-1,3,2diazaborinin-4-ium-5-uide

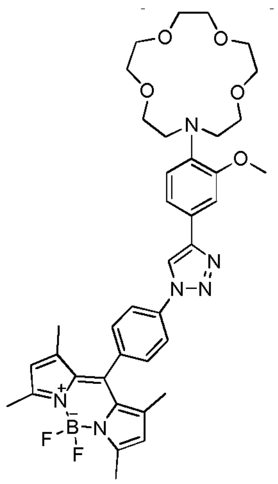

5,5-difluoro-10-(4-{1-[3-methoxy-4-(1,4,7,10-tetraoxa-13-azacyclopentadecan-13-yl)phenyl]-1H-1,2,3-triazol-4-yl}phenyl)-1,3,7,9-tetramethyl-5H-dipyrrolo[1,2-c:1',2'-f][1,3,2]diazaborinin-4-ium-5-uide

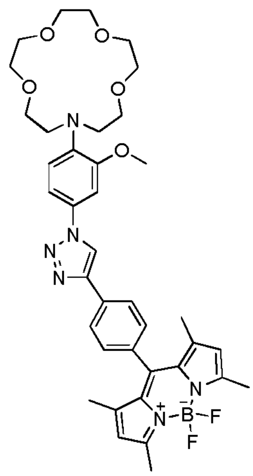

5,5-difluoro-10-(4-[3-methoxy-4{bis(4-methylbenzo[5,6,17,18](O,N,N',O')-]-1,7,16,-triaza-cryptand-[3,2,1]-phen-4-yl]-1H-1.2.3-triazol-1-yl}-(1,3,7,9-tetramethl-5H-dirrolo1,2-c:1',2'-f)(3,2diazaborinin-4-ium-5-uide

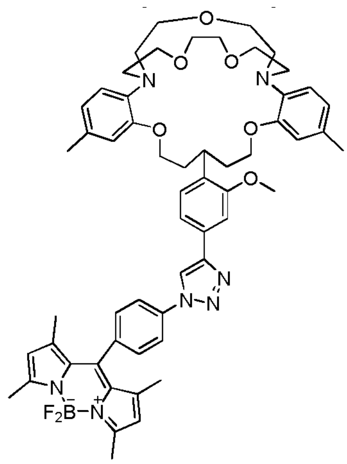

5,5-difluoro-10-(1-[3-methoxy-4{bis(4-methylbenzo[5,6,17,18](O,N,N',O')-]-1,7,16,-triaza-cryptand-[3,2,1]-phen-1-yl]-1H-1.2.3-triazol-1-yl}-(1,3,7,9-tetramethyl-5H-dipyrrolo)(1,2-c:1',2'-f)(1,3,2)diazaborinin-4-ium-5-uide

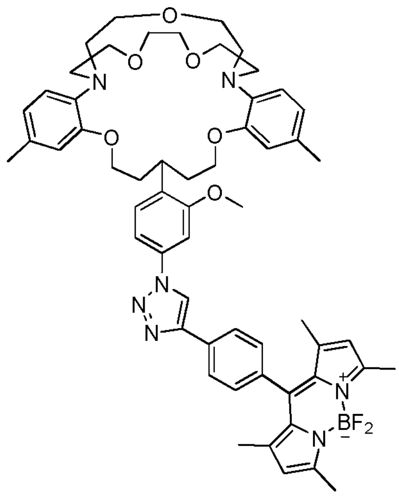

A further object of the present invention is a complex, consisting of a compound according to present invention as given in general Formula (I) and an alkali metal cation. The compounds of the present invention comprise an ionophore that has the capability to bind metal ions. This capability is given by the crown ether or the cryptand moiety of the compounds according to the invention.
According to the invention a complex is preferred, wherein the alkali metal cation is selected from the group consisting of $Na^+$ and $K^+$.
An object of the present invention is also a method for the determination of metal cations in a sample, comprising the steps of

a) contacting the metal cations with at least one compound according to the present invention,
b) forming a complex according to another object of the invention, whereupon fluorescence and/or luminescence appears or changes,
c) measuring the resulting fluorescence and/or luminescence.

According to the invention a method is preferred, wherein the metal cations are $Na^+$ or $K^+$, or a combination thereof.
According to the invention a method is especially preferred, wherein the at least one compound according to the invention selectively complexes the metal cations to be determined.
Lastly, another object of the invention is the use of a compound according to the invention in a method according to the invention for the quantitative determination of metal cations in a sample.

## Brief description of the figures

The figures show fluorescence spectra of preferred embodiments of the present invention.

**Fig. 1** Fluorescence spectra of **1** in MeCN (bottom line) upon addition of 0-2.5 mM $NaPF_6$ (middle line) and 0-1.16 mM $KPF_6$ (top line), respectively.

**Fig. 2** Fluorescence measurements of **1** ($\lambda_{ex}$ = 424 nm) under simulated physiological conditions [180 -0 mM choline chloride, 2mM $Ca^{2+}$, 2mM $Mg^{2+}$, pH=7.2 (10mM Tris)]. a) fluorescence spectra in the presence of 0-160 mM $Na^+$ (bottom lines) and $K^+$(top lines), respectively and b) corresponding FEF with error bars in the presence of 0-160 mM $K^+$ or $Na^+$ after repeating the experiment with n = 4.

**Fig. 3** $K^+$ Sensitivity of **1** ($\lambda_{ex}$ = 424 nm) under simulated physiological conditions, 0-2000mM KCl, saturation at 1000 mM.

**Fig. 4** $K^+$ Sensitivity of **5** ($\lambda_{ex}$ = 424 nm) under simulated physiological conditions [180 -0 mM choline chloride, 2mM $Ca^{2+}$, 2mM $Mg^{2+}$, pH=7.2 (10mM Tris)] in the presence of 0-160 mM $K^+$.

**Fig. 5** Fluorescence measurements of **5** ($\lambda_{ex}$ = 421 nm) under simulated physiological conditions [180 -0 mM choline chloride, 2mM $Ca^{2+}$, 2mM $Mg^{2+}$, pH=7.2 (10mM Tris)]. a) fluorescence spectra in the presence of 0-160 mM KCl and 180-0 mM NaCl, respectively and b) corresponding FEF in the presence of 0-160 mM $K^+$ or $Na^+$.

**Fig. 6** $K^+$ Sensitivity of **6** ($\lambda_{ex}$ = 367 nm) under simulated physiological conditions [180 -0 mM choline chloride, 2mM $Ca^{2+}$, 2mM $Mg^{2+}$, pH=7.2 (10mM Tris)] in the presence of 0-160 mM KCl.

**Fig. 7** Fluorescence measurements of **6** ($\lambda_{ex}$ = 367 nm) under simulated physiological conditions [180 -0 mM choline chloride, 2mM $Ca^{2+}$, 2mM $Mg^{2+}$, pH=7.2 (10mM Tris)]. a) fluorescence spectra in the presence of 0-160 mM KCl and 180-0 mM NaCl, respectively and b) corresponding FEF in the presence of 0-160 mM $K^+$ or $Na^+$.

**Fig. 8** $K^+$ Sensitivity of **14** ($\lambda_{ex}$ = 493 nm) under simulated physiological conditions [180 -0 mM choline chloride, 2mM $Ca^{2+}$, 2mM $Mg^{2+}$, pH=7.2 (10mM Tris)] in the presence of 0-180 mM $Na^+$.

**Fig.9** Fluorescence measurements of **14** ($\lambda_{ex}$ = 430 nm) under simulated physiological conditions [180 -0 mM choline chloride, 2mM $Ca^{2+}$, 2mM $Mg^{2+}$, pH=7.2 (10mM Tris)], fluorescence spectra in the presence of 0-180 mM $K^+$ plus $Na^+$.

**Fig. 10** $K^+$ Sensitivity of **15** ($\lambda_{ex}$ = 493 nm) under simulated physiological conditions [180 -0 mM choline chloride, 2mM $Ca^{2+}$, 2mM $Mg^{2+}$, pH=7.2 (10mM Tris)] in the presence of 0-180 mM $Na^+$.

**Fig. 11** Fluorescence measurements of **15** ($\lambda_{ex}$ = 422 nm) under simulated physiological conditions [180 -0 mM choline chloride, 2mM $Ca^{2+}$, 2mM $Mg^{2+}$, pH=7.2 (10mM Tris)], fluorescence spectra in the presence of 0-180 mM $K^+$ plus $Na^+$.

## Detailed description of the invention

As used herein, the terms have the following meaning:
The term "alkyl" or "lower alkyl" as used herein refers to a straight or branched chain, saturated hydrocarbon having the indicated number of carbon atoms. For example, (C1-C6) alkyl is meant to include, but is not limited to methyl, ethyl, propyl, isopropyl, butyl, sec-butyl, tert-butyl, pentyl, isopentyl, neopentyl, hexyl, isohexyl, and neohexyl. An alkyl or lower alkyl group can be unsubstituted or optionally substituted with one or more substituents.

The term "alkoxy" or "lower alkoxy" as used herein refers to an -O-alkyl group having the indicated number of carbon atoms. For example, a (C1-C6) alkoxy group includes -O-methyl, -O-ethyl, -O-propyl, -O-iospropyl, -O-butyl, -O-sec-butyl, -O-tert-butyl, -O-pentyl, -O-isopentyl, -O-neopentyl, -O-hexyl, -O-isohexyl, and -O-neohexyl.

The term "aromatic" as used herein refers to an aromatic or heteroaromatic moiety. An "aromatic" moiety refers to a 6- to 14-membered monocyclic, bicyclic or tricyclic aromatic hydrocarbon ring system. Examples of an aromatic group include phenyl and naphthyl. An aromatic group can be unsubstituted or optionally substituted with one or more substituents. The term "heteroaromatic" as used herein refers to an aromatic heterocyclic ring of 5 to 14 members and having at least one heteroatom selected from nitrogen, oxygen and sulfur, and containing at least 1 carbon atom, including monocyclic, bicyclic, and tricyclic ring systems. Representative heteroaromatics are triazolyl, tetrazolyl, oxadiazolyl,

pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, quinazolinyl, pyrimidyl, azepinyl, oxepinyl, naphthothiazolyl, quinoxalinyl. A heteroaromatic group can be unsubstituted or optionally substituted with one or more substituents.

The term "halogen" as used herein refers to -F, -Cl, -Br or -I.

As used herein, the term "heteroatom" is meant to include oxygen (O), nitrogen (N), and sulfur (S).

As used herein, the term "BODIPY" is an acronym for the compound class of boron-dipyrromethanes.

The new $\pi$-conjugated 1,2,3-triazol-1,4-diyl fluoroionophores according to the present invention generated via Cu(I) catalyzed [3+2] cycloaddition show high fluorescence enhancement factors in the presence of $Na^+$ and $K^+$ in MeCN and high selectivity towards $Na^+$ and $K^+$ under simulated physiological conditions.

Preferred embodiments of the present invention are compounds **1** and **2** as given in **Scheme 1**. The inventors found that in the simple CuAAC-generated fluoroionophore **1**, the electronic conjugation of the *N*-phenylaza-18-crown-6 ether and the 7-diethylaminocoumarin fluorophore through a 1,2,3-triazol-1,4-diyl $\pi$-linker results in a perfect signal transduction chain for the sensing of $Na^+$ and $K^+$. In MeCN high cation-induced FEFs were obtained for **1** ($FEF_{Na+}$: 58; $FEF_{K+}$: 27). The signal transduction in **1** also works nicely under simulated physiological conditions. In the presence of 160 mM $K^+$ a FEF of 2.5 was observed, whereas the same concentration range of $Na^+$ resulted in an almost negligible fluorescence enhancement. The inventors assume, that compound **1** is a PET-fluoroionophore with a virtual spacer between the anilinotriazole electron donor unit and the coumarin electron acceptor moiety.

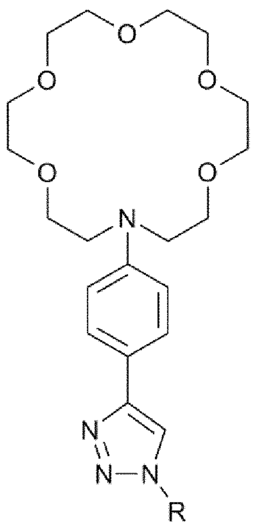

**1**

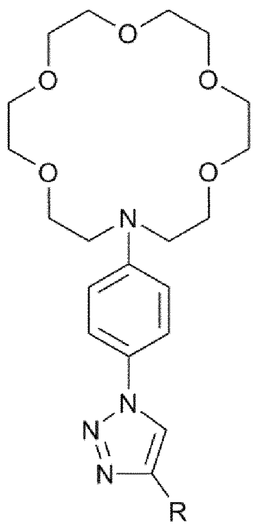

**2**

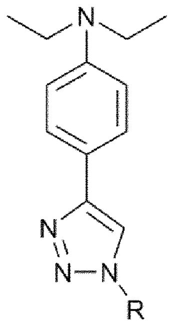

**3**

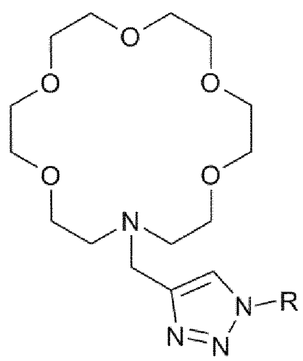

**4**

R = 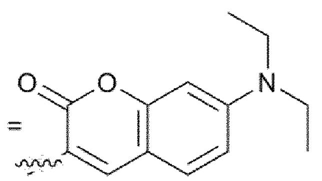

**Scheme 1** Conjugated fluoroionophores **1** and **2** with 1,2,3-triazol-1,4-diyl as the π-linker, and reference compounds **3** and **4**. ( indicates the binding position).

The CuAAC of the ethinyl-functionalized *N*-phenylaza-18-crown-6 ether[7] with 3-azido-7-diethylaminocoumarin[8] afforded 1,2,3-triazole-fluoroionophore **1**. The triazole-isomer **2** was obtained by the reaction of the azido-functionalized *N*-phenylaza-18-crown-6 ether[9] with 3-ethinyl-7-diethylaminocoumarin[10]. Dye **3**, in which the crown has been replaced by a diethylamino group was synthesised in order to represent a reference compound for **1**. A further reference compound for **1** is compound **4,** in which the p-phenylen linker between aza-18-crown-6 and triazole ring is replaced by a deconjugated methylene group. Compound **4** was obtained by the reaction of *N*-propargylaza-18-crown-6 ether[11] with 3-azido-7-diethyl-aminocoumarin. The new 1,4-disubstituted 1,2,3-triazoles **1-4** are stable in solutions of MeCN and DMSO at room temperature for many weeks. UV-irradiation over a period of several hours did not show any decomposition of the fluoroionophores **1** and **2**.

The fluorescence quantum yield of **1** in MeCN is extremely low ($\Phi_f$(**1**)**=** 0.008, Table 1). The fluorescence spectra of **1** in the presence of $Na^+$ and $K^+$, respectively (Fig. 1) show the impressive fluorescence enhancement upon coordination of the alkali metal ions. High FEFs could be determined for $Na^+$ (FEF=58) and for $K^+$ (FEF=27).[12]

**Table 1:**

Photophysical properties of **1-4** in MeCN.

| ligand | **1** | **2** | **3** | **4** |
|---|---|---|---|---|
| $\lambda_{em}$/nm[a] | 484 | 470 | 493 | 480 |
| $\lambda_{ex}$/nm[b] | 410 | 412 | 410 | 408 |
| $\Phi_f$(ligand)[c] | 0.008 | 0.03 | 0.017 | 0.56 |
| $\Phi_f$(ligand + $Na^+$)[d] complex)[d] | 0.62 | 0.79 | 0.018 | 0.55 |
| $\Phi_f$(ligand + $K^+$)[d] | 0.23 | 0.40 | 0.016 | 0.58 |

[a]Emission maxima, [b]excitation maxima, [c]fluorescence quantum yields ($\Phi_f$), [d]$\Phi_f$ were determined in the presence of 40 mM of $NaPF_6$ or 40 mM $KPF_6$, respectively.

The fluorescence quantum yield of the constitutional isomer **2** in MeCN is higher than that of **1**($\Phi_f$(**2**)= 0.03, Table 1). The fluorescence of **2** is also enhanced in the presence of increasing concentrations of $Na^+$ and $K^+$ (Fig. 7 and 8, respectively). However the observed FEFs for $Na^+$ (FEF=17) and $K^+$ (FEF=13) are significantly lower than for the isomer **1**.

The reference compound **4** consists of a poorer PET donor due to the aliphatic amine which is electronically separated from the triazole. Hence **4** has a high quantum yield ($\Phi_f$(4) = 0.56, Table 1) and is therefore less affected by the presence of $Na^+$ and $K^+$.

The inventors further investigated the influence of $Na^+$ and $K^+$ on the fluorescence of **1** under simulated physiological conditions. The ligand was exposed to aqueous solutions in the physiological interesting concentration range of 0-160 mM $Na^+$ or $K^+$, respectively. Additionally, the solutions contained 2 mM $Ca^{2+}$ and 2 mM $Mg^{2+}$. The pH was adjusted to 7.2 with 10 mM Tris and a constant ionic strength of 180 mM was maintained with choline chloride. Under these conditions, the fluorescence of 1 ($\lambda$ex = 424 nm, $\lambda$em = 500 nm, $\Phi_f$ = 0.07) is hardly increased in the presence of $Na^+$ whereas increasing concentrations of $K^+$ resulted in a modest fluorescence enhancement (Fig. 2a). In the presence of 160 mM $K^+$ a FEF of 2.5 is observed. The fluorescence measurements were repeated four times showing only very little variations in the FEF values (Fig. 2b). It is noteworthy, that the presence of physiological important extracellular cations, such as $Mg^{2+}$ and $Ca^{2+}$ does not affect the signal response.

In comparison, the FEF of **1** for $K^+$ in MeCN is clearly smaller than the FEF in water under simulated physiological conditions. This can be explained by the significantly smaller stability constants of the $K^+$ complex with the N-phenylaza-18-crown-6 in water [IgK ($H_2O$) > 0.5; IgK (MeCN) = 3.95$\pm$0.08]. The fact that the fluorescence of **1** in water is exclusively enhanced in the presence of $K^+$ and not by $Na^+$ can be rationalized by the stronger hydration enthalpy of $Na^+$. However, the fluorescence enhancement of **1** in the presence of $K^+$ under simulated physiological conditions shows that the signaling transduction chain in this fluoroionophore works well in water.

The dissociation constant (Kd) of **1** + $K^+$ amounts to ~ 260 mM in solutions which approximates physiological ionic strength. To measure intracellular or extracellular concentrations of $K^+$ (Kd around 140 or 4 mM) tuning of probe **1** towards a higher complex stability while maintaining the selectivity will be necessary.

To investigate the pH-sensitivity of **1**, the fluorescence intensity was measured in water at different pH values. The resulting pKa of **1** is near 4.5 meaning that the triazole-substituted N-phenylaza-18-crown-6 is less pH-sensitive than the N(o-methoxyphenyl)aza-15-crown-5-naphthalimide $Na^+$-fluoroionophore (pKa~5.5) of the authors He et al.[13]

In summary, the inventors have shown for the first time that an electronically conjugated 1,2,3-triazole-fluoroionophore consisting of the signaling transduction chain: anilino-ionophore - 1,2,3-triazol-1,4-diyl - fluorophore, works as an effective sensor for $Na^+$ and $K^+$ in acetonitrile with high cation-induced FEFs. Under simulated physiological conditions **1** selectively detects $K^+$ with the modest FEF being limited by the rather simple receptor unit. The inventors found that the substitution of ionophore and fluorophore on the 1,2,3-triazole ring has a basic influence on the quality of the fluoroionophore.

Recently He et al.[13a] and the Verkman group[13b] designed fluorescence switch-on PET sensors with high $K^+/Na^+$ selectivity and sensitivity for physiological $K^+$ in the concentration range of 0-40 mM. In these $K^+$ fluoroionophores a [3.2.2]-cryptand represents the ionophore. A drawback of the [3.2.2]-cryptand fluoroionophores though, is the very expensive synthetic procedure.[13c]

The inventors developed simple lariat aza-18-crown-6 ionophores, which show a high $K^+/Na^+$ selectivity and sensitivity under simulated physiological conditions. These novel lariat aza-18-crown-6 ionophores are also an object of the present invention.

The further preferred embodiments of the compounds of the present invention containing a 3(2-methoxyethoxy)phenyl-aza-18-crown-6 as $K^+/Na^+$ selective and sensitive ionophore are given in Scheme 3.

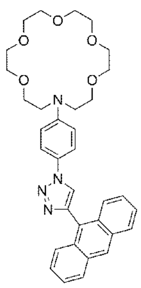
5

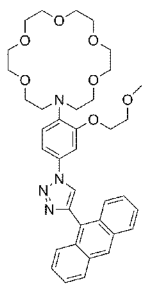
6

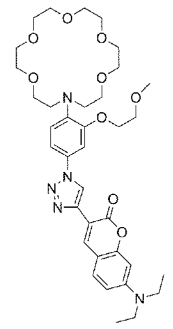
7

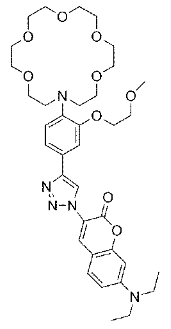
8

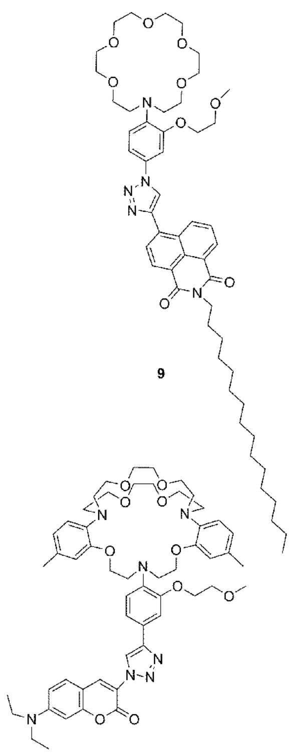
9

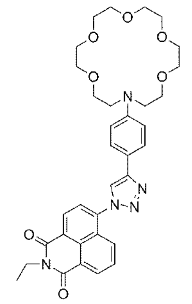
10

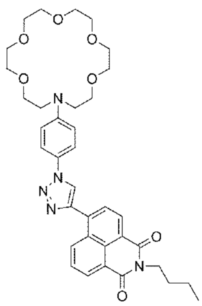
11

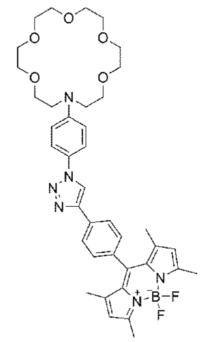
12

13

**Scheme 2** Conjugated $K^+$-fluoroionophores **5** to **13** with 1,2,3-triazol-1,4-diyl as the π-linker and either a phenyl-aza-18-crown-6 or a 3(2 methoxyeth-oxy)phenyl-aza-18-crown-6 as ionophoric unit

Compounds **6** to **9** have a 2-methoxyethoxy - lariate chain in close proximity to the aza-18-crown-6. This lariate group provides a higher binding selectivity for $K^+$. The fluorescence spectra are shown in **Figures 4 to 7**.

According to the invention $Na^+$-fluoroionophores **14** and **15** with a 1, 2,3-triazol-1,4-diyl as the π-linker (**Scheme 4**) are provided. These fluoroionophores show high $Na^+$/$K^+$-selectivity under simuated physiological conditions. The fluorescence spectra are shown in **Figures 8 to 11**.

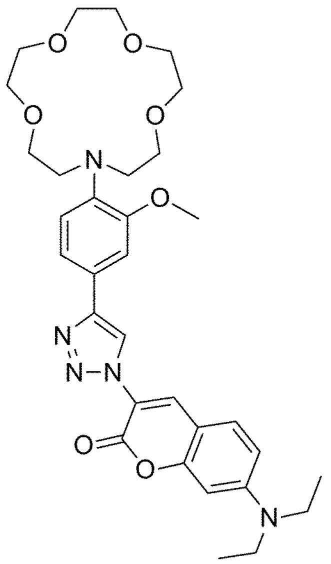

14

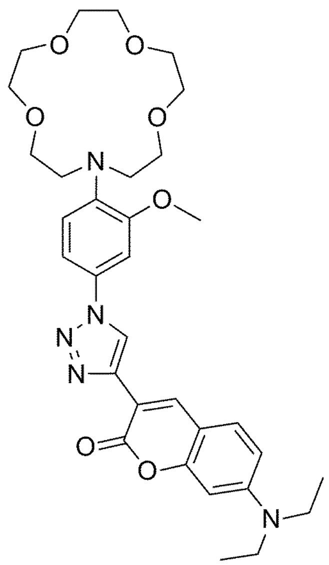

15

**Scheme 3** π-conjugated $Na^+$-fluoroionophores **14** and **15** with 1, 2,3-triazol-1,4-diyl as the π-linker.

The following examples explain the present invention in more detail. The embodiments described in the examples are not limiting the scope of the invention. The examples should only serve as preferred embodiments and a skilled artesian will derive other embodiments from those examples without any undue burden.

**Examples**

**Fluorescence measurements**

Fluorescence titration spectra of the compounds (c= $5 \cdot 10^{-6}$ $mol \cdot l^{-1}$) in acetonitrile were recorded 5 min after the addition of 0.02 ml of volumetric standard solution of $NaPF_6$ or $KPF_6$ (c= $5 \cdot 10^{-5}$ -$5 \cdot 10^{-2}$ $mol \cdot l^{-1}$), respectively. Titration was continued until no change in fluorescence enhancement was observed. Fluorescence Quantum yields were determined using a PL Quantum Yield measurement System C9920-2 of Hamamatsu, Japan.

Fluorescence spectra in aqueous solutions were carried out in buffered saline solutions (10 mM Tris-buffer, pH = 7.2) using a 1 mM ligand solution in DMSO. The water used was purified by a Milli-Q-Deioniser from Millipore®. To obtain physiological conditions, the salt solutions contained constant concentrations of $CaCl_2$ (2 mM) and $MgCl_2$ (2 mM). The physiological solutions were varied with regard to the concentrations of respectively KCl or NaCl and constant ionic strength was adjusted to a typical ionic strength in physiological systems (180 mM) by choline chloride.

The dye was exposed to different saline solutions containing 0, 5, 10, 20, 40, 80, 160 mM KCl or NaCl, respectively (Table 2 and 3). Each salt solution was mixed with 1 mM DMSO solution of the compound (990/10 v/v) to give a final ligand concentration of 10 μM. For each measurement, a freshly prepared dye mixture was used and every experiment was repeated with n = 4. Fluorescence response of the compounds was recorded exciting at the given wavelengths.

**Table 2.** Composition of the different aqueous solutions under simulated physiological conditions with regard to $K^+$-selectivity.

| KCl/ mM | Choline Cl/ mM | $MgCl_2$/ mM | $CaCl_2$/ mM |
|---|---|---|---|
| 0 | 180 | 2 | 2 |
| 5 | 175 | 2 | 2 |
| 10 | 170 | 2 | 2 |
| 20 | 160 | 2 | 2 |

(continued)

| KCl/ mM | Choline Cl/ mM | $MgCl_2$/ mM | $CaCl_2$/ mM |
|---|---|---|---|
| 40 | 140 | 2 | 2 |
| 80 | 100 | 2 | 2 |
| 160 | 20 | 2 | 2 |

**Table 3.** Composition of the different aqueous solutions under simulated physiological conditions with regard to $Na^+$-selectivity.

| NaCl/ mM | Choline Cl/ mM | $MgCl_2$/ mM | $CaCl_2$/ mM |
|---|---|---|---|
| 0 | 180 | 2 | 2 |
| 5 | 175 | 2 | 2 |
| 10 | 170 | 2 | 2 |
| 20 | 160 | 2 | 2 |
| 40 | 140 | 2 | 2 |
| 80 | 100 | 2 | 2 |
| 160 | 20 | 2 | 2 |

**General Methods and Procedures of Preparation of Compounds**

All commercially available chemicals were used without further purification. Solvents were distilled prior use. $^1H$ and $^{13}C$ NMR spectra were recorded on 300 MHz, 500MHz or 600MHz instruments. Data are reported as follows: chemical shifts in ppm (δ), multiplicity (s = singlet, d = doublet, t = triplet, m = multiplet, dd = doublet of doublets), integration, coupling constant (Hz). ESI spectra were recorded using a Micromass Q-TOF micro mass spectrometer in a positive electrospray mode. IR spectra were recorded using a Thermo Nicolet NEXUS FTIR instrument.

Air/water-sensitive reactions were performed in oven-dried glassware under an argon atmosphere. Column chromatography was performed with $SiO_2$ (Merck Silica Gel 60 (0.04-0.063 mesh)).

**Preparation of precursors**

The preparation of 3-azido-7-diethylaminocoumarine (**60**),[14] 3-ethinyl-7-diethylaminocoumarine (**61**),[15] *N*-phenylaza-18-crown-6 ether[16] and *N*-(4-formyl)phenylaza-18-crown-6 ether[17] has been described previously.

*16-L4-ethynylphenyl)-1,4,7,10,13-pentaoxa-16-azacyclooctadecane (**27**)*

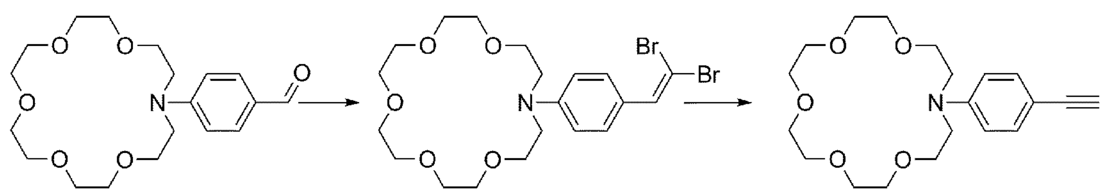

**25** **26** **27**

*N*-4-(2,2-Dibromovinyl)phenylaza-18-crown-6 ether (**26**)

The preparation of **27** followed the literature and references therein according to a modified procedure.[18]

A suspension of zinc (1.69 g, 25.9 mmol) and carbon tetrabromide (8.59 g, 25.9 mmol) in dry $CH_2Cl_2$ (40 ml) was cooled to -15°C with an ice-salt bath before a solution of triphenylphosphine (6.79 g, 25.9 mmol) in dry $CH_2Cl_2$ was added dropwise. The resulting yellow green mixture was kept at -15°C for 30 min and was then stirred at RT for 3 h whereupon a solution of **25** (4.31 g, 11.7 mmol) in dry $CH_2Cl_2$was added dropwise. The red brown mixture was stirred at RT for 2 h and then water (100 ml) was added. The organic phase was separated and washed with water (3 × 75 ml).The combined organic layer were dried with $MgSO_4$ and concentrated to give **26** as a light brown oil that was directly

used for the next step.

Note that the product decomposes when concentrated to dryness resulting in a green solid. Storage at -20°C is recommended.

HRMS ($^+$ESI): m/z calcd for $(M+H)^+$, 522.04, 524.05, 526.04; found, 522.11, 524.11, 526.11.

**26** (2.91 g, 5.56 mmol) was dissolved in 25 ml dry THF and set under argon atmosphere immediately. It was cooled to -78°C before n-butyllithium (7.5 ml, 1.6 M, solution in hexane) was added via a syringe. After stirring for 1h at - 78°C the solution was allowed to warm up to room temperature and stirring was continued at this temperature for 1h before $H_2O$ (15 ml) was added cautiously. The reaction mixture was extracted with $3\times CH_2Cl_2$ and the combined organic layers were dried with $MgSO_4$ and concentrated to give **27** as a red brown oil, which was purified via chromatography (silica gel, $CHCl_3$/MeOH, 95/5 v/v). Yield: 20%, overall.

$^1H$ NMR ($CDCl_3$, 300 MHz): $\delta$ = 2.93 (s, 1H), 3.6 (m, 24H), 6.56 (d, 2H, *J* = 9.23 Hz), 7.28 (d, 2H, *J* = 8.85 Hz); $^{13}C$ NMR ($CDCl_3$, 75 MHz): $\delta$ = 51.66, 68.94, 71.15, 75.12, 85.17, 108.82, 111.62, 133.73, 148.45; HRMS ($^+$ESI): m/z calcd for $(M+H)^+$, 364.21; found, 364.26; IR(ATR, $cm^{-1}$): 718, 692, 1115, 2097, 2868, 3054.

*16-(4-azidophenyl)-1,4,7,10,13-pentaoxa-16-azacyclooctadecane (**28**)*

The preparation of the *N*-(4-nitro)phenylaza-18-crown ether and subsequent hydrogenation to the corresponding amine followed the literature.[19]

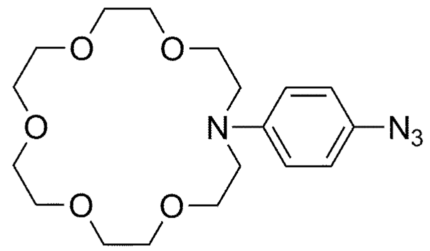

**28**

The synthesis of **28** followed the literature according to a modified procedure.[20] *N*-Anilino-4-aza-18-crown-6 ether (4.03 g, 11.4 mmol) was dissolved in 90 ml HCl (4M) and cooled to 0°C. A solution of $NaNO_2$ (0.784 g, 11.4 mmol) in 45 ml $H_20$ was added dropwise. The mixture was stirred for 10 min before a solution of $NaN_3$ (1.1 g, 17 mmol) in 45 ml $H_20$ was added drop wise. Stirring was continued for another 10 min at 0°C before the orange solution was allowed to warm up to room temperature and it was stirred for 14 h at ambient temperature. The reaction mixture was brought to pH = 7 with $Na_2CO_3$, was extracted with 3x180ml $CHCl_3$ and the combined organic layers were dried with $MgSO_4$. The solvent was removed under reduced pressure to yield **28** as brown oil (1.31 g, 30.3 %). The product was used for the next reaction step without further purification.

$^1H$ NMR ($CDCl_3$, 300 MHz): $\delta$ = 3.57-3.69 (m, 24H), 6.68 (d, 2H, *J* = 8.854 Hz), 6.88 (d, 2H, *J* = 9.042 Hz); $^{13}C$ NMR ($CDCl_3$, 75 MHz): $\delta$ = 51.48, 68.74, 70.70, 112.97, 119.94, 127.55, 145.98;

HRMS ($^+$ESI): m/z calcd for $(M+H)^+$, 381. 21; found, 381.34, calcd for $(M-N_2)^+$ 353.21; found, 353.30; IR (ATR, $cm^{-1}$): 1100 , 1508, 2120, 2097, 2867.

*16-(proip-2-ynyl)-1,4,7,10,13-pentaoxa-16-azacyclooctadecane (**29**)*

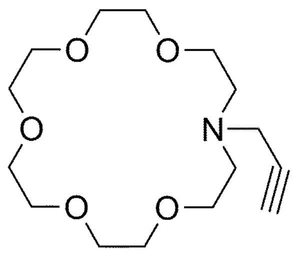

**29**

The synthesis followed the literature according to a modified procedure.[21] Monoaza-18-crown-6 ether[22] (0.6 g, 2.28 mmol) and propargylbromide (0.206 ml, 2.74 mmol) were dissolved in dry acetonitrile (70 ml) before $Cs_2CO_3$ (1.48 g, 4.56 mmol) was added. The suspension was stirred overnight at 85°C. After cooling to room temperature, the suspension was filtered and the filtrate was concentrated. The residue was purified by column chromatography on silica gel eluting with $CHCl_3$/MeOH (9/1 v/v) yielding **29** as a red oil (64 %)
$^1$H NMR ($CDCl_3$, 300 MHz): $\delta$ = 2.18 (t, 1H, *J* = 2,45Hz), 2.82 (t, 4H, *J* = 5.28Hz), 3.61-3.74 (m, 22H); $^{13}$C ($CDCl_3$, 75 MHz): $\delta$ = 43.42, 53.29, 68.94, 70.52, 73.61, 78.85; IR (ATR, $cm^{-1}$): 1115, 2867, 3189; HRMS ($^+$ESI): m/z calcd. for $(M+H)^+$,302.20; found, 302.21.

*N,N-diethyl-4-ethynylaniline (**32**)*

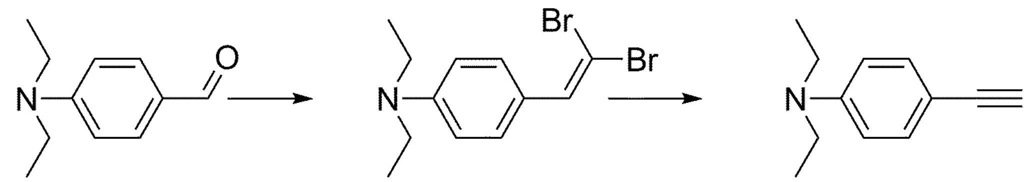

30 31 32

The synthesis followed the literature according to a modified procedure.[23]

1,1-Dibromo-2-(4-*N,N*-diethylaminophenyl)ethane (**31**):

A mixture of zinc (4.9 g, 75 mmol), triphenylphosphine (19.67 g, 75 mmol), and carbon tetrabromide (24.87 g, 75 mmol) in dry dichloromethane (250 ml) was sonicated for 2 h while cooling with an ice bath. *N,N*-diethylaminobenzaldehyde (**30**) (5.30 g, 29.9 mmol) was added to the grayish suspension and it was stirred overnight resulting in a brownish suspension.

The mixture was concentrated and petroleum ether (500 ml) was added, whereupon a tarry precipitate formed. It was washed with 1/1 $CH_2Cl_2$/light petroleum ether (2 $\times$ 100 ml). The combined organic phases were concentrated, and the residue was chromatographed on silica gel (150 g), eluting with 1/1 $CH_2Cl_2$/hexane to give **31** as a yellow oil (7.3 g, 21.0 mmol, 70 %).

The intermediate product was kept at -20°C until the next step.
$^1$H NMR ($CDCl_3$, 300 MHz): $\delta$ = 1.17 (t, 6H, *J* = 6.94 Hz, $CH_3$), 3.37 (q, 4H, *J* = 6.94 Hz, $CH_2$), 6.62 (d, 2H, *J* = 7.88 Hz, Ar-H), 7.32 (s, 1H, CH), 7.49 (d, 2H, *J* = 8.21 Hz); HRMS ($^+$ESI): m/z calcd. for $(M+H)^+$, 331.96, 333.96, 335.96; found, 331.93, 333.93, 335.94.

*N,N-diethyl-4-ethynylaniline (**32**):*

A solution of **31** (4.3 g, 12.4 mmol) in dry THF (93 ml) was cooled to -78°C before n-butyllithium (19 ml, 15% in hexane) was added dropwise. It was stirred for 45 min at this temperature before the reaction mixture was allowed to warm up to RT. After stirring for 1 h at RT, water (7ml) was added slowly. The solvent was removed and the residue was taken up in diethyl ether. The organic layer was washed with water (30 ml) and brine (30 ml) and dried over $MgSO_4$. Concentration of the ether phase gave **32** as orange oil that was used in the next step without further purification (1.29 g, 6.96 mmol, 56.2 %).
$^1$H NMR ($CDCl_3$, 300 MHz): $\delta$ = 1.16 (t, 6H, *J* = 7.25Hz), 2.97 (s, 1H), 3.37 (q, 4H, *J* = 7.25Hz,), 6.57 (d, 2H, *J* = 9.14Hz), 7.34 (d, 2H, *J* = 9.14Hz), $^{13}$C ($CDCl_3$, 75 MHz): $\delta$ = 12.46, 44.26, 74.41, 85.01, 107.47, 110.94, 133.39, 147.73, HRMS ($^+$ESI): m/z calcd. for $(M+H)^+$, 174.13; found, 174.16.

13-(4-Ethynyl-2-methoxyphenyl)-1,4,7,10-tetraoxa-13-azacyclopentadecane (**40**)

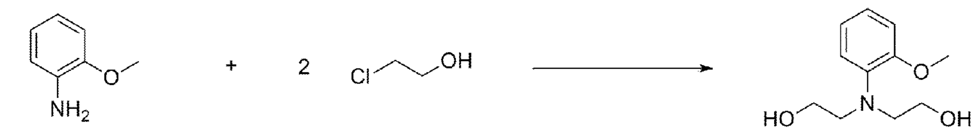

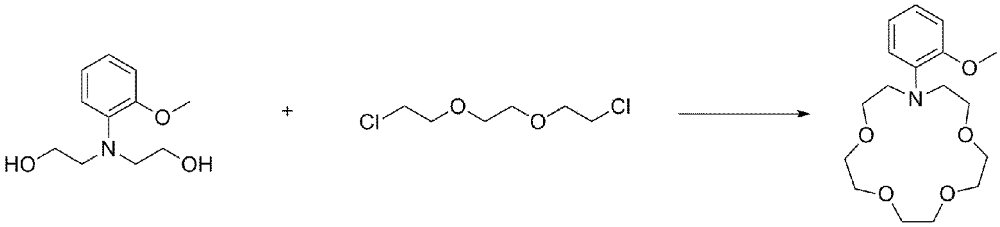

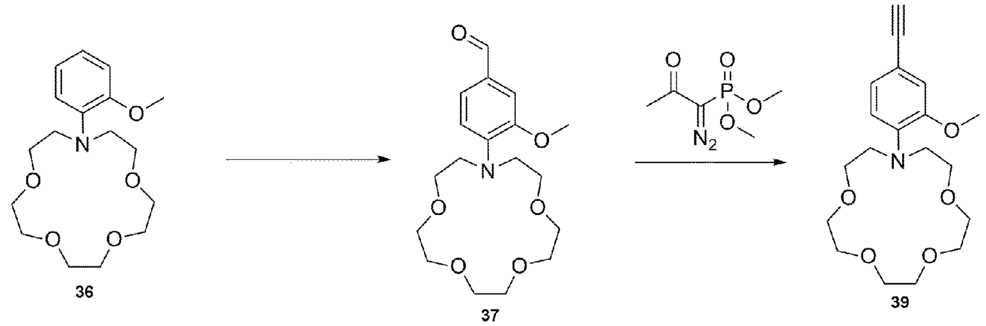

36 37 39

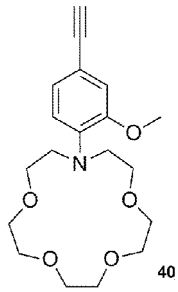

40

The synthesis followed the literature according to a modified procedure.[24] Into a stirred mixture of 860 mg (2, 43 mmol) Aldehyd **37** and 673 mg (4,86 mmol) $K_2CO_3$ in 35 ml dry Methanol was added 564 mg (2,93 mmol) dimethyl-1-diazo-2-oxopropylphosphonate (Bestmann-Ohira Reagent). This mixture was stirred for 20 hours at ambient temperature. After addition of 60 ml $CHCl_3$ and extraction 3 times with water, the organic layer was separated, dried with $MgSO_4$ and concentrated in vacuum. The residue was purified by column chromatography on silica with $CHCl_3/CH_3OH$ (95/5) as eluent. The product was obtained as a yellow oil (435 mg, 51%).

$^1$H NMR (300 MHz, $CDCl_3$): $\delta$ = 2.99 (s, 1H), 3.44-3.48 (m, 4H), 3.58-3.66 (m, 16H), 3.77 (s, 3H),

$^{13}$C NMR (75 MHz, $CDCl_3$): $\delta$ = 52.85, 55.37, 69.94, 70.25, 70.34, 70.85, 75.57, 84.15, 113.94, 115.13, 119.06, 125.21, 140.93, 151.32

MS (EI) m/z (%): 349(100) $[M]^+$.

IR (ATR, $cm^{-1}$): 2923 (s), 2855 (s), 2102 (m), 1507 (s), 1253 (s), 1110 (s). Elemental analysis (%) calcd. for $C_{19}H_{27}NO_5$ (349.42): C 65.31, H 7.79, N 4.01; found C 65.12, H 7.38, N 3.98

13-(4-Azido-2-methoxvphenvl)-1,4,7,10-tetraoxa-13-azacyclopentadecane (43)

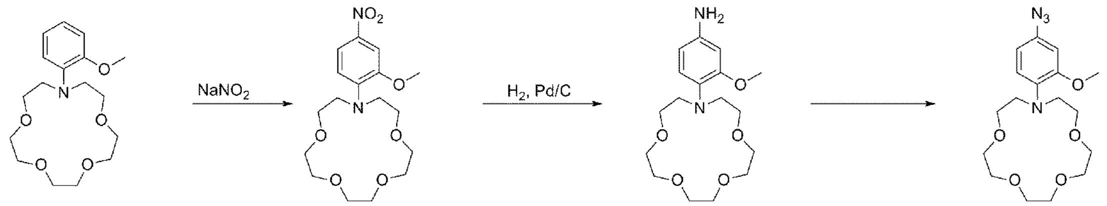


**36** **41** **42** **43**

The synthesis of **43** followed the literature according to a modified procedure.[25]

The appropriate aminocrownether (**42**) (807 mg, 2.37 mmol) was dissolved in 18 ml 4 M HCl and cooled. At 0°C was slowly added a solution of 163,6 mg (2.37 mmol) sodium nitrite in 10 ml water. The solution was stirred for 10 minutes at 0°C. Then was added a solution of 230 mg (3,53 mmol) sodium azide in 10 ml water. The resulting solution was stirred for further 10 min at 0°C and led warm up to room temperature. It was stirred overnight. The mixture was brought to pH 7 with potassium carbonate and extracted 3 times with $CHCl_3$. The combined organic layers were dried with $MgSO_4$ and concentrated in vacuum. The residue was purified by column chromatography on silica with $CHCl_3/CH_3OH$ (95/5) as eluent. The product was obtained as a darkbrown oil (265 mg, 31%).
$^1H$ NMR (300 MHz, $CDCl_3$): δ = 3.39-3.4 (m, 4H), 3.60-3.68 (m, 16H), 3.79 (s, 3H)
$^{13}C$ NMR (75 MHz, $CDCl_3$): δ = 53.13, 55.47, 70.08, 70.37, 70.48, 70.93, 103.25, 110.61, 122.16, 133.87, 137.25, 153.89
MS (EI) m/z (%): 366 (12) $[M]^+$, 338 (100)$[M-N_2]^+$.
IR (ATR, $cm^{-1}$): 2858 (s), 2102 (s), 1504 (s), 1234 (s), 1105 (s).

N-n-butyl-4-ethinyl-1,8-naphthalimide (**50**) *N-n-butyl-4-bromo-1,8-naphthalimide*

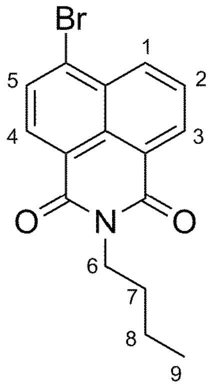

The reaction followed the literature according to a modified procedure.[26]

In a dry 250-ml round bottom flask, under a stream of argon gas, were placed the 4-bromo-1,8-naphthalic anhydride (95 w%, 1.35 g, 4.63 mmol, 1 equiv.) and 1-butylamine (99 w%, 410.35 mg, 5.5 mmol, 1.2 equiv.) in dry ethanol (96 ml) and the reaction mixture was heated at 60 °C for 16 h. After cooling to room temperature, the solid was filtered and washed with 200 ml $H_2O$ to give the product as a light yellow solid.
Yield: 0.800 mg, 52 %, mp: 100 - 102 °C (Lit.: 103-105 °C, $R_f$ = 0.8 (n-hexane/ethyl acetate = 4:1 v/v); **$^1$H NMR,** ($CDCl_3$, 300 MHz) δ (ppm), *J* (Hz): 8.63 (d, 1H, *J* = 7.29, $H^1$), 8.53 (d, 1H, *J* = 8.52, $H^3$), 8.39 (d, 1H, *J* = 7.86, $H^4$), 8.01 (d, 1H, *J* = 7.9, $H^5$), 7.82 (tr, 1H, *J* = 8.46, $H^2$), 4.16 (t, 2H, *J* = 7.55, $H^6$), 1.71 (qu, 2H, *J* = 7.5, $H^7$), 1.45 (sx, 2H, *J* = 7.5, $H^8$), 0.97 (t, 3H, *J* = 7.32, $H^9$); **$^{13}$C NMR**, ($CDCl_3$, 75 MHz) δ (ppm): 163.74, 163.71, 133.30, 132.11, 131.30, 131.21, 130.74, 130.28, 129.12, 128.19, 123.30, 122.45, 40.53, 30.31, 20.52, 13.98;

*N-n-Butyl-4-(trimethylsilylethinyl)-1,8-naphthalimide*

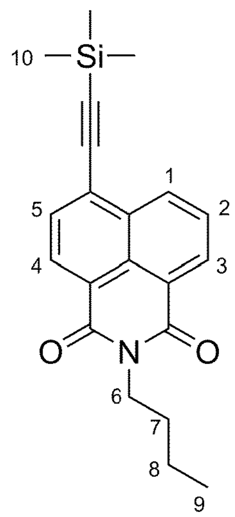

A dry 100-ml two-necked round-bottom flask was set under argon atmosphere before *N*-n-Butyl-4-Bromo-1,8-naphthalimide(0.8g, 2.4 mmol, 1equiv.), THF (8 mL), $Pd(PPh_3)_2Cl_2$ (33.8 mg, 0.048 mmol, 0.02 equiv.), $PPh_3$ (25.3 mg, 0.096 mmol, 0.04 equiv.), CuI (18.3 mg, 0.096 mmol, 0.04 equi..) and dry TEA (8 ml) were added followed by the trimethylsilylacetylene (331.1 mg, 3.37 mmol, 1.4 equiv.). It was refluxed for 6 h. Then the reaction mixture was allowed to cool down to room temperature and the solvent was evaporated under reduced pressure. The crude product was purified by column chromatorgraphy (50 x 3 cm, silica gel, $CH_2Cl_2$ = 10:1 v/v) to give the productas a yellow solid.
Yield: 0.780 g, 92 %, mp: 136.2-138.8 °C, $R_f$ = 0.87; **$^1$H NMR**, ($CDCl_3$, 300 MHz) $\delta$ (ppm), *J* (Hz): 8.61 (dd, 2H, $H^4$, $H^3$), 8.50 (d, 1H, *J* = 7.62, $H^1$), 7.88 (d, 1H, J = 7.62, $H^5$), 7.8 (t, 1H, *J* = 7.62, $H^2$), 4.17 (t, 2H, *J* = 7.5, $H^6$), 1.71 (qu, *J* = 7.5, 2H, $H^7$), 1.37-1.51 (m, 2H, *J* = 7.53$H^8$), 0.97 (t, 3H, J = 7.3, $H^9$), 0.36 (s, 9H, $H^{10}$); **$^{13}$C NMR**, ($CDCl_3$, 75 MHz) $\delta$ (ppm): 164.14, 163.86, 132.49, 131.93, 131.68, 131.32, 130.35, 128.07, 127.63, 127.37, 123.11, 122.53, 105.37, 101.44, 40.48, 30.36, 20.54, 13.99, 0.002;

*N-n-Butyl-4-ethinyl-1,8-naphthalimide (**50**)*

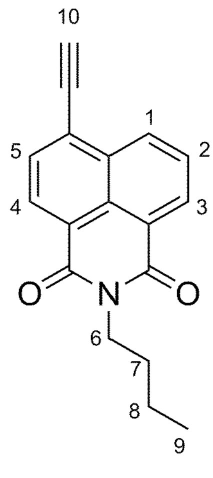

*N*-n-Butyl-4-(trimethylsilylethinyl)-1,8-naphthalimide (710 mg, 2.03 mmol) was placed into a 100-ml round-bottom flask and in dry methanol (50 ml) and dry $K_2CO_3$ (1.09 g, 7.92 mmol, 3.9 equiv.) were added. It was heated for 16 h at room temperature. $CH_2Cl_2$ (40 ml) was added to the reaction mixture and the organic phase was washed with water (2× 20 ml). The combined organic phases were dried over $Na_2SO_4$ and concentrated in vacuo. The crude product was purified by column chromatography (50 x 3 cm, silica gel, $CH_2Cl_2$/ethyl acetate = 10:1 V/V) to give the product as a yellow solid.
Yield: 0.200 g, 35 %, mp.: 127.5-130.8 °C, $R_f$= 0.81; **$^1$H NMR**, ($CDCl_3$, 600 MHz) $\delta$ (ppm), *J* (Hz): 8.65 (d, 1H, *J* = 8.43, $H^3$), 8.63 (d, 1H, *J* = 7.32, $H^4$,), 8.53 (d, 1H, *J* = 7.56, $H^1$), 7.93 (d, 1H, *J* = 7.56, $H^5$), 7.82 (t, 1H, *J* = 8.25, $H^2$), 4.17 (t,

2H, $J$ = 7.62, H[6]), 3.73 (s, 1H, H[10]), 1.71 (qu, 2H, $J$ = 7.56, H[7]), 1.44 (sx, 2H, $J$ = 7.56, H[8]), 0.97 (t, 3H, $J$ = 7.38, H[9]); **$^{13}$C NMR**, ($CDCl_3$, 75 MHz) δ (ppm): 164.05, 163.78, 132.24, 132.12, 131.77, 130.245, 128.09, 127.81, 126.30, 123.24, 123.07, 122.01, 86.52, 80.51, 40.50, 30.36, 20.52, 13.95;

6-azido-N-ethyl-1,8-nalphthalimide (**51**)[27] *N-n-ethyl-4-bromo-1,8-naphthalimide*

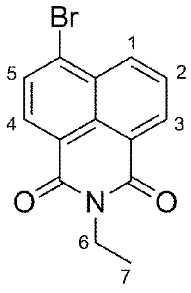

The synthesis followed the literature according to a modified procedure.'[1b] A suspension of 4-bromo-1,8-naphthalic anhydride (2g, 7.2 mmol, 1equiv.) and ethylamine (70 w% in water ,0.69 ml, 8.66 mmol, 1.2 equiv.) in 100 ml 1.4-dioxane was refluxed for 6 h giving a brownish solution. The solution was cooled to room temperature before it was poured onto 300 ml ice water resulting in an off white precipitate. After complete melting of the ice, the precipitate was collected by filtration and was washed with water. The resulting solid was dried in vacuo in a desiccator equipped with $CaCl_2$ to give the product as an off white solid which was directly used in the next step.
Yield: 1.72g, 78 %, mp: 145-146 °C, **$^1$H NMR**, ($CDCl_3$, 500 MHz) δ (ppm), $J$ (Hz): 8.62 (dd, 1H, H[1]), 8.34 (dd, 1H, H[3]), 8.37 (d, 1H, $J$ = 7.85, H[4]), 8.0 (d, 1H, $J$ = 7.80, H[5]), 7.81 (tr, 1H, J = 7.35, H[2]), 4.22 (q, 2H, $J$ = 7.15, H[6]), 1.32 (tr, 3H, $J$ = 7.10, H[7]); MS (EI): m/z calcd.: 303; found, 303+305: $[m/z\text{-}\cdot CH_2CH_3]^+$ calcd.:275, found 275+277;

*6-azido-N-ethyl-1,8-naphthalimide (**51**)*

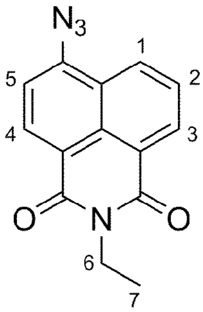

The synthesis followed the literature and references therein. In a 25-ml round-bottom flask was placed the *N*-n-ethyl-4-bromo-1,8-naphthalimide (0.5 g, 1.64 mmol, 1 equiv.) and sodium azide (0.53 g, 8.2 mmol, 5 equiv.). Then *N*-methylpyrrolidinone (7ml) was added and the mixture was heated at 110 °C for 1.5 h. The solution was allowed to cool to room temperature and diluted with 25 ml water. It was extracted with ethyl acetate (3×20 ml) and the combined organic phases were washed with brine. The organic phase was dried over $MgSO_4$ and concentrated under reduced pressure. The crude product was purified by column chromatography (silica gel, hexane/ethyl acetate = 4:1 v/v) to give the product as a yellow solid.
Yield: 0.254 g, 58 %, **$^1$H NMR**, ($CDCl_3$, 300 MHz) δ (ppm), $J$ (Hz): 8.62 (dd, 1H, H[1]), 8.56 (dd, 1H, H[4]), 8.41 (dd, 1H, H[3]), 7.72 (m, 1H, H[2]), 7.45 (dd, 1H, H[5]), 4.23 (q, 2H, $J$ = 7.14, H[6]), 1.32 (tr, 3H, $J$ = 7.08, H[7]); **$^{13}$C NMR**, ($CDCl_3$, 75 MHz) δ (ppm): 163.92, 163.51, 143.50, 132.26, 131,74, 129.27, 128.82, 126.96, 124.49, 122.85, 119.15, 114.78, 35.65, 13.48; MS (EI): m/z calcd.:266; found, 266; $[m/z\text{-}\cdot N_2]^+$ calcd.:238, found 238; IR (KBr, $cm^{-1}$): 2131 (s, $N_3$).

10-ethynylanthracene (**52**)

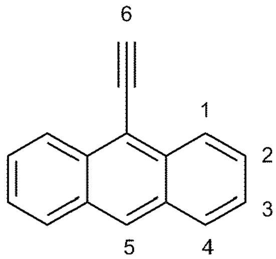

The synthesis followed the literature. [28]
Yield: 78 %, $^{1}$**H NMR**, ($CDCl_3$, 300 MHz) δ (ppm)): 8.58 (dd, 2H, $H^1$), 8.46 (s, 1H, $H^5$), 8.02 (dd, 2H, $H^4$), 8.62-8.45 (m, 4H, $H^{2,3}$), 3.99 (s, 1H, $H^6$)

**General procedure for the synthesis of azidoanthracene derivatives**

9-(azidomethyl)-anthracene (**53**)

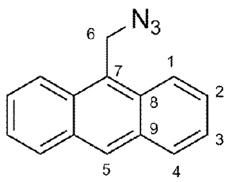

The synthesis followed the literature according to a modified procedure. [29]

A suspension of chloromethylanthracene (1g, 4.41 mmol, 1equiv.) and sodium azide (0.43g, 6.62 mmol, 1.5 equiv.) in 30 ml acetonitrile was refluxed for 5 h. After the reaction was complete (monitoring via DC) the reaction mixture was cooled to room temperature and the resulting solid was filtered off. The solution was concentrated in vacuo and the resulting yellow solid was purified by column chromatography on silica gel (n-hexane/ethyl acetate = 3:1 v/v).
Yield: 0.92 g (90%), mp.: 78-80°C, $^{1}$H-NMR, ($CDCl_3$, 500 MHz) δ (ppm), *J* (Hz): 8.5 (s, 1H, $H^5$), 8.29 (d, 2H, *J* = 8.85, $H^1$), 8.05 (d, 2H, *J* = 8.40, $H^4$), 7.60 (m, 2H, $H^2$), 7.52 (m, 2H, $H^3$), 5.31 (s, 2H, $H^6$); $^{13}$C-NMR, ($CDCl_3$, 125 MHz) δ (ppm): 131.45, 130.79, 129.39, 129.09, 126.94, 125.87, 125.30, 123.31, 46.42; MS (EI): m/z calcd.:233; found, 233; FT-IR (KBr), $cm^{-1}$: 2095 (s, $-N_3$), 1444 (m, $-CH_2-$).

10-(azidomethyl)-9-carbonitrilanthracene (**54**)

The Synthesis of the 10-Bromomethyl-9-cyanoanthracene via 10-Methyl-9-cyanoanthracene followed the literature. [30]

*10-methyl-9-cyanoanthracene*

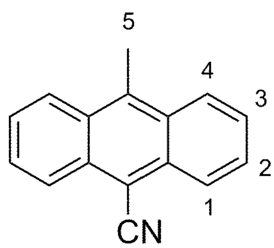

The product was collected after crystallization from acetic anhydride in form of yellow needles. Yield: 81 %, mp: 207-208 °C; **$^1$H-NMR**, ($CDCl_3$, 300 MHz) δ (ppm), *J* (Hz): 8.38 (d, 2H, *J* = 8.61, $H^1$), 8.29 (d, 2H, *J* = 8.79, $H^4$), 7.69-7.5 (m, 4H, $H^{2,3}$), 3.09 (s, 3H, $H^5$); **$^{13}$C-NMR**, ($CDCl_3$, 75 MHz) δ (ppm): 138.24, 132.98, 129.51, 128.45, 126.29, 126.17, 125.48, 117.83, 104.41, 14.91; HRMS ($^+$ESI): m/z calcd for $(M+H)^+$, 218.10; found, 218.15.

*10-bromomethyl-9-cyanoanthracene*

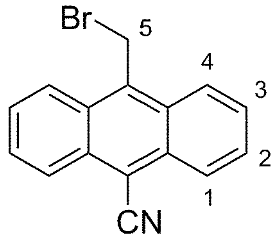

Recrystallization from *n*-hexanes/$CHCl_3$ at -20°C gave the product as a yellow solid (needles). Yield: 90,1% mp.: 248-254 °C, R*f* = 0.33 (n-hexanes/ethyl acetate = 8:2 v/v); **$^1$H-NMR,** ($CDCl_3$, 300 MHz) δ (ppm), *J* (Hz):8.49-8.43 (m, 2H, $H^1$), 8.39-8.31 (m, 2H, $H^4$), 7.77-7.69 (m, 4$H^{2,3}$), 5.46 (s, 2H, $H^5$); **$^{13}$C-NMR**, ($CDCl_3$, 75 MHz) δ (ppm): 135.07, 133.18, 128.95, 128.85, 127.68, 126.45, 124.42, 117.20, 107.84, 24.97; **FT-IR** (KBr,$cm^{-1}$): 3051 (w, Br-$CH_2$-), 2212 (s, -C≡N), 1444 (m, -$CH_2$-).

*10-(azidomethyl)-9-carbonitrilanthracene (**54**)*

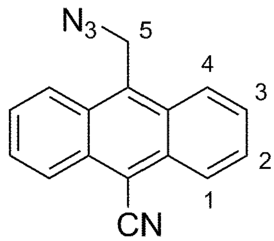

The synthesis followed the procedure of the preparation of 9-(azidomethyl)-anthracene as described above. The crude product was recrystallized from $CH_2Cl_2$/MeOH to give the product as yellow fluffy needles. Yield: 39 %, $R_f$ = 0.41; $^1$H-NMR, ($CDCl_3$, 300 MHz) δ (ppm), *J* (Hz): 8.48 (d, 2H, *J* = 8.84, $H^1$), 8.35 (d, 2H, *J* = 8.13, $H^4$), 7.79-7.65 (m, 4H, $H^{2,3}$), 5.33 (s, 2H, $H^5$); $^{13}$C-NMR, ($CDCl_3$, 75 MHz) δ (ppm): 133.04, 132.90, 129.94, 128.85, 127.84, 126.52, 124.48, 117.07, 108.08, 46.29; HRMS ($^+$ESI): m/z calcd for $(M+H)^+$, 259.10; found, 259.15; FT-IR (KBr) u ($cm^{-1}$): 2213 (s,-C≡N), 2100 (s, -$N_3$), 1444 (m, -$CH_2$-).

9-(azidomethyl)-10-methylanthracene (**55**)

*9-(10-methyl)-bromomethylanthracene* [31]

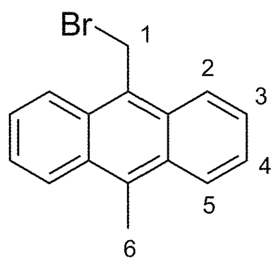

A 500-ml round-bottom flask, equipped with condenser, thermometer and stirbar was set under argon atmosphere and the anthraquinone (10.5 g, 0.050 mol) and dry THF (375 ml) were added. The mixture was cooled to -78 °C before a solution of methyl lithium (110 ml, 1.4 M in diethylether, 0.151 mol, 3.05 equiv.) was added cautiously via a syringe. The reaction mixture was slowly allowed to warm up to room temperature and was stirred at this temperature for 2h before water (100 ml) was added. The mixture was extracted with $CH_2Cl_2$ (2 x 100 ml) and the combined organic phases were concentrated to give the 9,10-dihydroxy-9,10-dimethylanthracene as a white solid which was used in the next step without further purification. It was dissolved in THF (250 ml) and a solution of THF (130 ml) and HBr (150 ml, 48 w%) was added drop wise. The reaction mixture was stirred at ambient temperature for 30 min where upon yellow crystals formed. The crystals were collected by filtration, washed with water and dried under vacuum to give the final product.
Yield: 10.16 g, 72 %, mp: 177.5-179.0 °C, $R_f$ = 0.25 ($CHCl_3$/MeOH = 97:3 v/v); **$^1$H-NMR,** ($CDCl_3$, 300 MHz) δ (ppm), *J* (Hz): 8.35 (t, 4H, *J* = 7.56, $H^{1,5}$), 7.65 (tr, 2H, *J* = 6.9, *J* = 8.2, $H^3$), 7.54 (tr, 2H, *J* = 6.9, *J* = 8.2, $H^4$), 5.55 (s, 2H, $H^1$), 3.09 (s, 3H, $H^6$); **$^{13}$C-NMR**, ($CDCl_3$, 75 MHz) δ (ppm): 133.30, 130.28, 129.58, 126.39, 126.32, 125.67, 125.39, 124.26, 27.97, 14.74; HRMS ($^+$ESI): m/z calcd for $(M+H)^+$, 286.19; found, 286.32;

*9-(azidomethyl)-10-methylanthracene (**55**)*

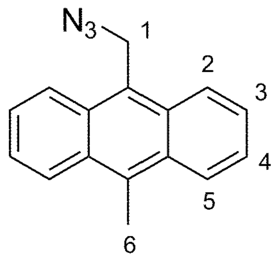

The synthesis followed the procedure of the preparation of 9-(azidomethyl)-anthracene as described above. Yield: 90 %, $R_f$ = 0.32; **$^1$H-NMR**, ($CDCl_3$, 300 MHz) δ (ppm), *J* (Hz): 8.39-8.31 (m, 4H, $H^{2,5}$), 7.63-7.53 (m, 4H, $H^{3,4}$), 5.34 (s, 2H, $H^1$), 3.14 (s, 3H, $H^6$); **$^{13}$C-NMR**, ($CDCl_3$, 75 MHz) δ (ppm): 132.91, 130.62, 130.03, 126.44, 125.74, 125.22, 124.29, 46.69, 14.64; MS (EI): m/z:247; [m/z-·$N_2$]= 218; [m/z-·$CH_2N_3$]= 205; **FT-IR** (KBr), $cm^{-1}$: 2214 (s, C≡N), 2114 (s, $N_3$), 1444 (m, $CH_2$).

N.N-bis(2-hydroxyethyl)-2-methoxyethyloxyaniline

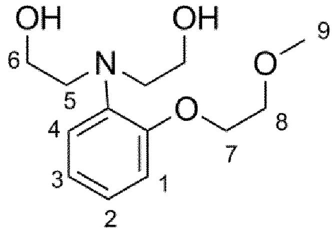

The Synthesis followed the literature according to a modified procedure. [32] A mixture of *N*-(2-methoxyethoxy)-2-nitrobenzole[33] (21.9 g; 0.131 mol; 1 equiv.). 2-chlorethanol (52.77g; 0.656 mol; 5 equiv.) and $CaCO_3$ (18.37; 0.184 mol; 1.4 equiv.) in dist. water (300 ml) was stirred for 6 days at 60 °C. After cooling to room temperature, $Na_2CO_3$ (75.0g; 0.7 mol; 5.34 equiv.) was added and it was stirred for 40 Min at 60 °C after which the solid is filtered off. The aqueous phase is extracted tertbutyl-methyl-ether (3×500ml). The combined organic phases are dried over $MgSO_4$ and concentration in vacuo gave the crude product as a brownish oil, which was purified by column chromatography (silica gel. ethyl acetate). Yield: (30.1 g. 90 %).
**$^1$H NMR ($CDCl_3$. 300 MHz):** $\delta$ = 3.15 (tr. 4H. $H^6$). 3.43 (s. 3H. $H^9$). 3.47 (tr. 4H. $H^5$). 3.74 (tr. 2H. $H^8$). 4.11 (tr. 2H. $H^7$). 6.91 (dd. 1H. $H^4$). 6.98 (tr. 1H. $H^2$). 7.11 (tr. 1H. $H^3$). 7.22 (d. 1H. $H^1$); $^{13}$C NMR **($CDCl_3$. 75 MHz):** $\delta$ = 57.93; 58.93; 59.66; 67.80; 70.70; 113.33; 122.23; 125.51; 126.01; 139.28; 155.25; HRMS ($^+$ESI): m/z calc. for $(M+H)^+$. 256.15; found. 256.13.

2-methoxythoxyphenylaza-18-crown-6-ether

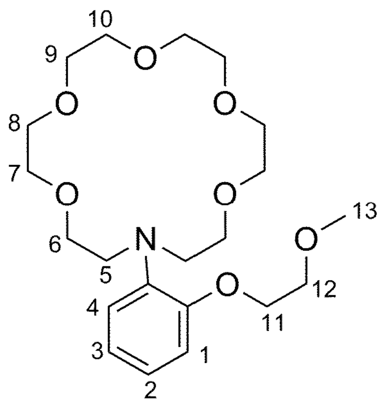

The synthesis followed the literature according to a modified procedure. [34] The *N.N*-bis(2-hydroxyethyl)-2-methoxyethyloxyaniline (15.46 g; 60.55 mmol; 1equiv.) was dissolved in 440 ml dry acetonitrile and set under argon atmosphere. Under a stream of argon sodiumhydride (80% ig; 4.5 g; 2.86 equiv.) was added to the reaction mixture over a period of 1h. The 1,17-ditosyl-3,6,9,12,15-pentaoxaheptadecane (30.4 g; 60.55 mmol; 1equiv.) was dissolved in 216 ml dry acetonitrile and added dropwise to the refluxing reaction mixture over 4 h. It was heated to reflux for 11 h before the yellow suspension was allowed to cool to room temperature. It was filtered and the solvent was removed to give a brown oil. which was purified by column chromatography (silica gel. $CHCl_3$. MeOH, 95/5, v/v) to give the product as a light brown oil.
$^1$H NMR ($CDCl_3$. 300 MHz): $\delta$ = 3.41 (s. 3H. $H^{13}$). 3.45-3.66 (m. 24H. $H^{5-10}$). 3.73 (tr. 2H. *J* = 4.9 Hz. $H^{12}$). 4.10 (tr. 2H.. *J* = 4.9 Hz. $H^{13}$). 6.82-7.12 (m. 4H. $H^{1-4}$); $^{13}$C NMR ($CDCl_3$. 75 MHz): $\delta$ = 56.70; 59.91; 59.83; 67.83; 69.57-71.18; 114.39;121.24; 122.21; 124.21; 151.71; 153.9; HRMS ($^+$ESI): m/z ber. für $(M+H)^+$. 255.16; gefunden. 255.13.

N-2-methoxyethoxy-4-nitrophenylaza-18-crown-6-ether

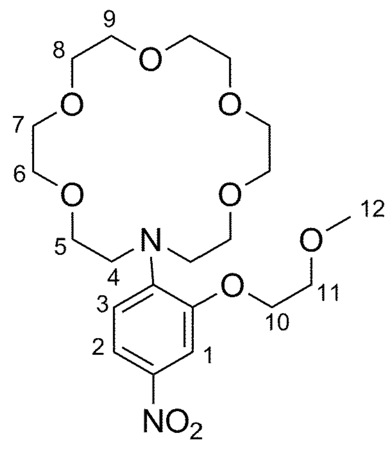

The synthesis followed the literature according to a modified procedure. [35] The 2-methoxyethoxyphenylaza-18-crown-6-ether (4.4g; 10.65 mmol; 1equiv.) was dissolved in a mixture of dist. water (340 ml) und glacial acetic acid (34 ml) before a solution of $NaNO_2$ (0.81; 11.7 mmol; 1.1 equiv.) in deionized water was added drop wise over a period of 10 min. It was stirred for 16 h at room temperature. The deep orange reaction mixture was neutralized with LiOH followed by extraction with $CH_2Cl_2$ (3×100ml) The combined organic phases were dried over $MgSO_4$ and the solvent was removed in vacuo to give the crude product as an orange oil. Purification by column chromatography yielded the product as an orange oil (silica gel. $CH_3Cl$/MeOH, 95/5, v/v) (1.1 g. 22.4 %).
**$^1$H NMR ($CDCl_3$. 300 MHz):** $\delta$ = 3.40 (s. 3H. $H^{12}$). 3.60-3.70 (m. 24 H. $H^{4-9}$). 3.73 (m, 2H, $H^{11}$). 4.14 (m, 2H, $H^{10}$). 6.88 (d. 1H. $J$ = 9.04 Hz. $H^3$). 7.65 (d, 1H. $J$ = 2.5 Hz, $H^1$). 7.80 (dd, 1H, $H^2$); HRMS ($^+$ESI): m/z calc. $(M+H)^+$. 459.23; found, 459.26. **$^{13}$C NMR ($CDCl_3$. 75 MHz):** $\delta$ = 52.83, 58.82, 67.91, 69.93, 70.52, 70.55, 72.57, 70.64, 70.69, 70.77, 108.20. 115.65, 118.57, 139.07 ,146.32, 148.38; HRMS ($^+$ESI): m/z calc. for $(M+H)^+$. 459.23; found. 459.25.

*N*-3-(-2-methoxyethoxyphenylaza-18-crown-6-)aniline

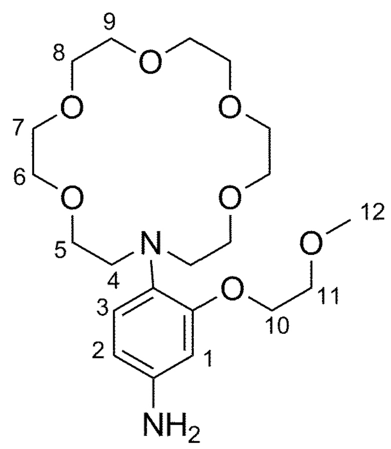

The *N*-2-methoxyethoxy-4-nitrophenylaza-18-crown-6-ether (0.22g. 0.48 mmol) was dissolved in dry methanol (30 ml) After addition of Pd/C (30 mg) it was hydrated in an autoclave for 16 h at 75 bar. The catalyst was filtered of through a bed of Celite® and the solvent was removed in vacuo to yield a colorless oil (0.2g; 97%). Note that the compound decomposes quickly when exposed to air, resulting in a purple colour. That's why it was used in the next step without further purification.
HRMS ($^+$ESI): m/z calc. for $(M+H)^+$. 429.26; found. 429.20.

*N*-4-(azido)-2-methoxyethoxyphenylaza-18-crown-6-ether (**56**)

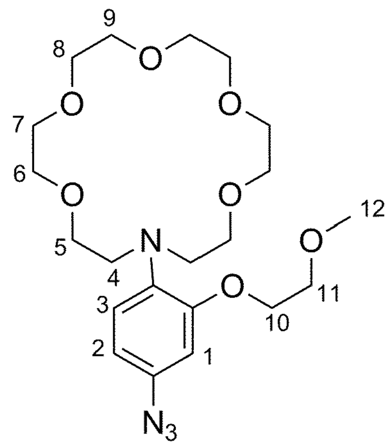

Due to the instability of the *N*-3-(-2-methoxyethoxyphenylaza-18-crown-6-)aniline. the reaction was carried out under argon atmosphere and in oven dried glassware. The aqueous solutions were degassed and flushed with argon prior use.

The *N*-3-(-2-methoxyethoxyphenylaza-18-crown-6-)aniline (0.2 g;0.467 mmol; 1equiv.) was dissolved in HCl (3.6 ml; 4M) and cooled to 0°C before a solution of $NaNO_2$ (32 mg; 0.467 mmol; 1equiv.) in 1.8 ml $H_2O$ was slowly added via a syringe. The reaction mixture was stirred at this temperature for 10 min. before a solution of $NaN_3$ (0.45 mg. 0.7 mmol; 1.5 equiv.) in 1.8 ml $H_20$ was added dropwise followed by 10 in of stirring at 0°C. The solution was allowed to warm up to room temperatur and stirred at ambient temperatuer for 14 h. The reaction mixture was neutralized with $Li_2CO_3$ before it was extracted with 3×50ml $CHCl_3$. The organic layers were combined, dried with $MgSO_4$, filtered and the solvent was removed under reduced pressure. The crude product was purified by column chromatography (silica gel. $CH_3Cl$/MeOH (95/5) yielding the desired product as light yellow oil (0.13 g. 61.3 %).
**$^1$H NMR ($CDCl_3$. 300 MHz):** $\delta$ = 3.4 (s. 3H. $H^{12}$). 3.52-3.68 (m. 24H. $H^{4-9}$). 3.75 (tr. 2H. *J* = 4.9 Hz. $H^{11}$). 4.10 (tr. 2H. *J* = 4.9 Hz. $H^{10}$). 6.51 (s. 1H. $H^3$). 6.60 (d. 1H. *J* = 8.48. $H^2$). 7.09 (d. 1H. *J* = 8.48 Hz. $H^2$); **$^{13}$C NMR ($CDCl_3$. 75 MHz):** $\delta$ = 53.18. 59.06. 67.82. 69.76. 70.31. 70.55. 70.72. 70.61. 71.00. 105.02. 111.48. 123.52. 134.51. 136.95. 153.87; HRMS ($^+$ESI): m/z calc. for $(M+H)^+$ found. 455.17.
IR (ATR. $cm^{-1}$): 2106 (strong. $-N_3$)

6-ethynyl-2-hexadecyl-1.8-naphthalimid (**57**)

The synthesis of the 6-ethynyl-2-hexyl-1.8-naphthalimid_followed the literature according to a modified procedure. [36]

*6-bromo-2-hexadecyl-1.8-naphthalimid*

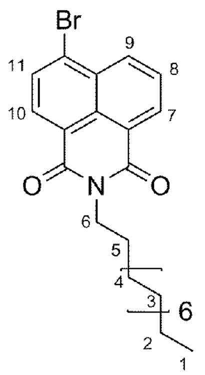

The 4-bromonaphthalene anhydride (1.0 g. 3.61 mmol. 1 equiv.) and n-butylamine (1.04 g. 4.32 mmol. 1.2 equiv.) were stirred in dry ethanol (96 ml) at 60 C for 16 h. The reaction mixture was allowed to warm up to room temperature and before the solid was filtered off. It was washed with 200 ml $H_2O$ and dried in high vacuo to give the

product as a light yellow solid (1.74 g; 93 %)
**$^1$H NMR ($CDCl_3$. 300 MHz):** $\delta$ = 0.89 (tr. 3H. $H^1$). 1.25-1.48 (m. 24H. $H^{2-4}$). 1.73 (qu. 2H. $H^5$). 4.17 (tr. 2H. $H^6$). 7.85 (tr. 1H. $J$ = 7.35 Hz. $H^8$). 8.06 (d. 1H. $J$ = 7.72 Hz. $H^{11}$). 8.57 (d. 1H. J = 7.91 Hz. $H^{10}$). 8.57 (dd. 1H. $H^7$). 8.66 (d. 1H. J = 7.35 Hz. $H^9$); **$^{13}$C NMR ($CDCl_3$. 75 MHz):** $\delta$ = 14.50. 23.08. 27.52. 28.47. 29.75. 29.95. 30.00. 30.03. 30.05. 30.06. 30.09. 32.32. 41.02. 122.69. 123.55. 128.42. 129.34. 130.50. 130.96. 131.43. 131.52. 132.33. 133.50. 163.90. 163.92; MS (EI): m/z calcd.: 505; found. 499+501;

*2-hexadecyl-6-((trimethylsilyl)ethynyl)-1. 8-naphthalimid*

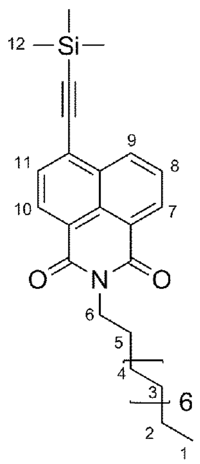

Under argon atmosphere, to a dry flask provided with the 6-bromo-2-hexadecyl-1.8-naphthalimid (0.916 g; 1.83 mmol; 1 equiv.). $Pd(PPh_3)_2Cl_2$ (26.0 mg. 0.037 mmol. 0.02 equiv.). $PPh_3$ (19.0 mg. 0.037 mmol. 0.04 equiv.). CuI (14.0 mg. 0.037 mmol. 0.04 equiv.) and dry triethylamine were added. Dry THF (10 mL) was transfered into the flask via a canula followed by the dropwise addition of trimethylsilylacetylene (0.252 mg. 2.56 mmol. 1.4 equiv.). The reaction mixture was heated to reflux for 6 h and then allowed to cool down to room temperature. The solvent was removed and the residue was taken up $CHCl_3$. Water was added and it was extracted with 3×30ml $CHCl_3$. The organic layers were combined, dried over $MgSO_4$ and filtered. The solvent was removed in vacuo to yield the desired product quantitatively as a grey solid (0.94 g. 100 %).
**$^1$H NMR ($CDCl_3$. 300 MHz):** $\delta$ = 0.35 (s. 9H. J = 6.97 Hz. $H^{12}$). 0.86 (tr. 3H. $H^1$). 1.23-1.34 (m. 24H. $H^{2-4}$). 1.71 (qu. 2H. J = 7.54 Hz. $H^5$). 4.14 (tr. 2H. J = 7.72 Hz. $H^6$). 7.80 (tr. 1H. J = 7.35. $H^8$). 7.89 (d. 1H. J = 7.35 Hz. $H^7$). 8.51 (d. 1H. J = 7.72Hz $H^{11}$). 8.62 (dd. 2H. $H^{9.\ 10}$); **$^{13}$C NMR ($CDCl_3$. 75 MHz):** $\delta$ = 9.08. 14.03. 22.64. 27.13. 28.12. 29.31. 29.34. 29.51. 29.56. 29.60. 29.62. 29.63. 29.65. 31.89. 40.57. 46.15. 101.32. 105.20. 122.45. 123.36. 127.46. 130.16. 131.15. 131.48. 132.29. 163.163.94; MS (EI): m/z calcd.: 517; found. 517;

*6-ethynyl-2-hexadecyl-1.8-naphthalimid (57)*

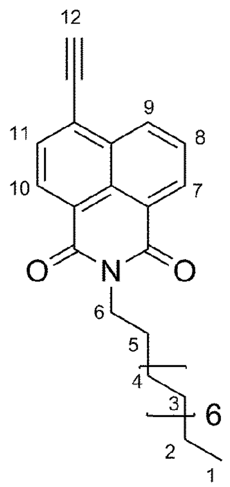

The 2-hexadecyl-6-((trimethylsilyl)ethynyl)-1.8-naphthalimid (198 mg. 0.383 mmol; 1 equiv.) was dissolved in 10 ml dry methanol. dry $K_2CO_3$(212 mg. 1.53 mmol. 4 equiv.) was added and it was stirred for 50 h at roomtemperature The red brown suspension was filtered and the collected solid was purified by column chromatography (80 x 3 cm. Silicagel. methylene chloride/ ethyl acetate = 95:5 v/v) to give the pure product as a light yellow solid (0.91 g. 53 %). **$^1$H NMR ($CDCl_3$. 300 MHz):** $\delta$ = 0.87 (tr. 3H. $J$ = 6.78Hz. $H^1$). 1.24-1.44 (m. 26H. $H^{2-4}$). 1.72 (qu. 2H. $J$ = 7.53Hz. $H^5$). 3.73 (s. 1H. $H^{12}$). 4.19 (tr. 2H. $J$ = 7.53Hz. $H^6$). 7.82 (tr. 1H. $J$ = 7.35Hz. $H^8$). 7.96 (d. 1H. $J$ = 7.72Hz. $H^{11}$). 8.26 (d. 1H. $J$ = 7.53Hz. $H^{10}$). 8.64 (dd. 2H. $H^{7.9}$); **$^{13}$C NMR ($CDCl_3$. 75 MHz):** $\delta$ = 14.22. 22.83. 27.32. 28.30. 29.50. 29.52. 29.70. 29.56. 28.80. 29.82. 29.85. 32.08. 40.79. 80.56. 86.50. 123.16. 123.30. 126.31. 127.80. 128.14. 130.24. 131.76. 131.79. 132.16. 132.23. 163.76. 164.03; MS (EI): m/z calcd.: 445; found. 445;

**General Procedure of the CuAAC reaction**

The Cu(I) catalyzed reaction between an azide and an alkyne (CuAAC) has been performed as follows: All reactions were performed on a mmol-scale. To a solution of azide (1 eq) and alkyne (1 eq) in THF/$H_2O$ (3/1) was added CuI (5 mol%) and sodium ascorbate (2.5 mol%). It was stirred overnight at 50°C.

The THF was removed under reduced pressure and the residue was taken up in $CHCl_3$. It was washed with water and the organic phase was dried over $MgSO_4$. The organic phase was concentrated and the residue was chromatographed on silica gel eluting with $CH_2Cl_2$/MeOH (95/5).

**Example 1**

Synthesis of 3-{4-[4-(1,4,7,10,13-pentaoxa-16-azacyclooctadecan-16-yl)phenyl]-1H-1,2,3-triazol-1-yl}-7-(diethylamino)-2H-chromen-2-one (**1**):

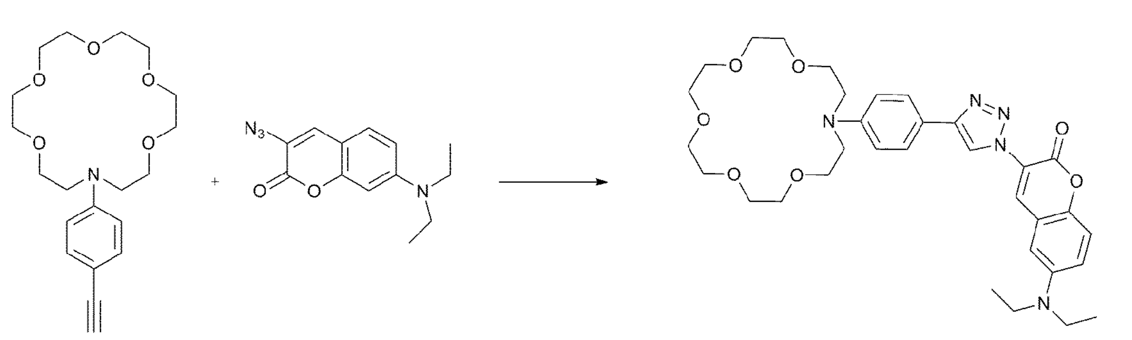

1

**27** **33**

The synthesis followed the general procedure of the CuAAC reaction.
Yield: 49 %, $^{1}H$ NMR ($CDCl_3$, 500 MHz): $\delta$ = 1.23 (t, 6H, *J* = 7.25Hz), 3.44 (q, 4H, *J* = 7.25Hz,), 3.64-3.67 (m, 20H), 3.72 (t, 4H, *J* = 5.68Hz), 6.55 (s, 1H), 6.66 (dd, 1H), 6.75 (d, 2H, *J* = 8.52Hz), 7.41 (d, 1H, *J* = 8.83Hz), 7.74 (d, 2H, *J* = 8.83Hz), 8.15 (s, 1H), 8.38 (s, 1H); $^{13}C$ ($CDCl_3$, 75 MHz): $\delta$ = 13.53, 46.05, 52.38, 69.78, 71.89, 98.08, 108.27, 110.91, 112.67, 118.12, 119.22, 119.70, 127.96, 130.95, 135.34, 148.82, 149.06, 152.40, 156.79, 158.02; HRMS ($^{+}$ESI): m/z calcd. for $(M+H)^+$, 622.32; found, 623.33; UV/Vis (Acetonitrile), $\lambda_{max}$ ($\varepsilon$) = 410 nm (21228 $M^{-1}$ $cm^{-1}$), $\lambda_{max}$ ($\varepsilon$) = 288 nm (20211 $M^{-1}$ $cm^{-1}$).

## Example 2

Synthesis of 3-{1-[4-(1,4,7,10,13-pentaoxa-16-azacyclooctadecan-16-yl)phenyl]-1H-1,2,3-triazol-4-yl}-7-(diethylamino)-2H-chromen-2-one (**2**):

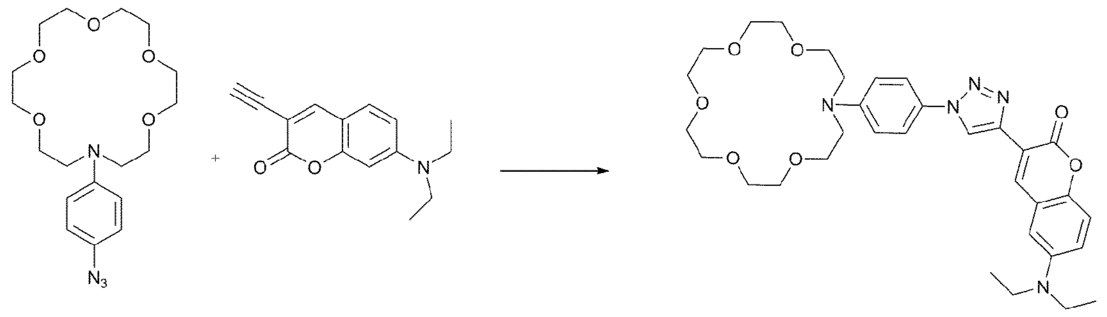

2

**28** **34**

The synthesis followed the general procedure of the CuAAC reaction.
Yield: 55 %, $^{1}H$ NMR ($CDCl_3$, 600 MHz): $\delta$ = 1.23 (t, 6H, *J* = 7.25Hz), 3.44 (q, 4H, *J* = 7.25Hz,), 3.63-3.67 (m, 20H), 3.72 (t, 4H, *J* = 5.68Hz), 6.53 (s, 1H), 6.62 (dd, 1H), 6.76 (d, 2H, *J* = 8.48Hz), 7.41 (d, 1H, *J* = 8.85Hz), 7.76 (d, 2H, *J* = 8.48Hz), 8.60 (s, 1H), 8.64 (s, 1H); $^{13}C$ ($CDCl_3$, 150 MHz): $\delta$ = 12.54, 44.94, 51.51, 68.58, 70.84, 97.13, 108.83, 109.44, 110.87, 111.93, 120.54, 122.12, 126.56, 129.60, 138.51, 142.06, 148.26, 150.82, 156.11, 160.79; HRMS ($^{+}$ESI): m/z calcd. for $(M+H)^+$, 622.32; found, 623.35; UV/Vis (Acetonitrile), $\lambda_{max}$ ($\varepsilon$) = 413 nm (40057 $M^{-1}$ $cm^{-1}$), $\lambda_{max}$ ($\varepsilon$) = 293 nm (18136 $M^{-1}$ $cm^{-1}$).

## Example 3 (reference compound)

Synthesis of *N,N*-Diethyl-4-[1-(7-diethylaminocoumarin-3-yl)-1*H*-1,2,3-triazol-4-yl)]aniline (**3**):

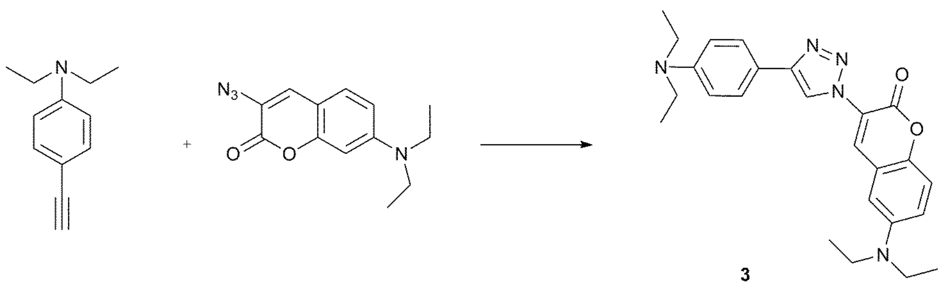

3

**32** **33**

The synthesis followed the general procedure of the CuAAC reaction.
Yield: 60 %, $^{1}H$ NMR ($CDCl_3$, 300 MHz): 5 = 1.17-1.27 (m, 12H), 3.37-3.50 (m, 8H), 6.57 (s, 1H), 6.68 (dd, 1H), 6.75

(d, 2H, *J* = 8.48Hz), 7.42 (d, 1H, *J* = 8.85Hz), 7.76 (d, 2H, *J* = 8.1Hz), 8.44 (s, 1H), 8.65 (s, 1H), $^{13}C$ ($CDCl_3$, 75 MHz): $\delta$ = 12.43, 12.62, 29.69, 44.39, 44.98, 97.08, 107.28, 109.99, 111.80, 117.31, 117.57, 118.61, 127.06, 129.91, 134.15, 147.75, 148.25, 151.41, 155.72, 157.01; HRMS ($^+$ESI): m/z calcd. for $(M+H)^+$, 431.23; found, 432.32; UV/Vis (acetonitrile), $\lambda_{max}$ ($\varepsilon$) = 410 nm (29501 $M^{-1}$ $cm^{-1}$), 248 nm (22823 $M^{-1}$ $cm^{-1}$).

**Example 4 (reference compound)**

Synthesis of 3-(4-((1,4,7,10,13-pentaoxa-16-azacyclooctadecan-16-yl)methyl)-1H-1,2,3-triazol-1-yl)-7-(diethylamino)-2H-chromen-2-one (**4**):

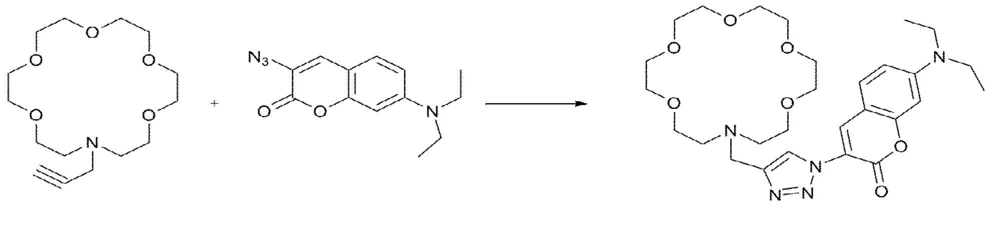

4

**29** **33**

The synthesis followed the general procedure of the CuAAC reaction.

Yield: 23 %, $^1H$ NMR ($CDCl_3$, 300 MHz): $\delta$ =1.14 (t, 6H, *J* = 7.16Hz), 2.70 (m, 4H), 3.36 (q, 4H, *J* = 7.16Hz,), 3.54-3.68 (m, 20 H), 3.79 (s, 2H), 6.42 (s, 1H), 6.60 (dd, 1H), 7.40 (d, 1H, *J* = 9.04Hz), 8.24 (s, 1H), 8.33 (s, 1H); $^{13}C$ ($CDCl_3$, 75 MHz): $\delta$ = 12.76, 45.34, 49.51, 53.78, 67.91, 69.72, 97.16, 107.21, 110.60, 124.15, 129.08, 130.67, 131.26, 136.14, 152.12, 156.27, 127.46 ; HRMS ($^+$ESI): m/z calcd. for $(M+H)^+$, 560.31; found,560.43; UV/Vis (acetonitrile), $\lambda_{max}$ ($\varepsilon$) = 408 nm (10534 $M^{-1}$ $cm^{-1}$), 246 nm (11149 $M^{-1}$ $cm^{-1}$).

**Example 5**

Synthesis of 7-(diethylamino)-3-(4-(3-methoxy-4-(1,4,7,10-tetraoxa-13-azacyclopentadecan-13-yl)phenyl)-1H-1,2,3-triazol-1-yl)-2H-chromen-2-one (**14**):

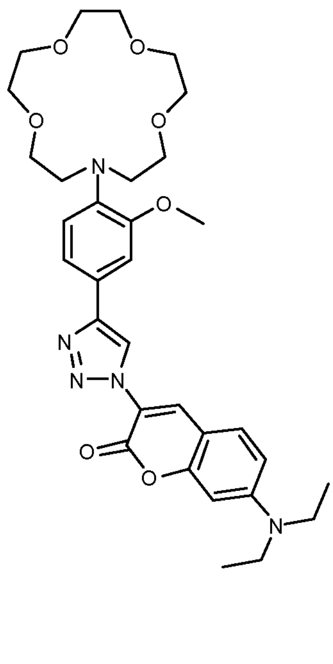

A mixture of compound **40** (130 mg, 0.37 mmol), 3-azido-7-diethylaminocoumarin **60** (96.1 mg, 0.37 mmol), copper sulfate pentahydrate (4.6 mg, 5 mol%) and sodium ascorbate (7.3 mg, 10 mol%) in 6 ml THF/water (2/1) was stirred at 60 °C for 48 hours. To the reaction mixture was added water (5 mL) and it was extracted 3 times with $CHCl_3$ (3×10 mL). The combined organic layers were dried with $MgSO_4$ and concentrated in vacuum. The residue was purified by column chromatography on silica with $CHCl_3/CH_3OH$ (95/5) as eluent. The product was obtained as a dark yellow oil, which crystallised upon standing in a freezer. (90 mg, 40%).
$^1H$ NMR (500 MHz, $CDCl_3$): $\delta$ = 1.24 (t, 6H, $^3J$ = 7.09 Hz, 1-H), 3.45 (q, 4H, $^3J$ = 7.09 Hz, 2-H), 3.51-3.55 (m, 4H, 21-H), 3.64-3.70 (m, 16H, 22-H, 23-H, 24-H, 25-H), 3.93 (s, 3H, 20-H), 6.56 (d, 1H, $^4J$ = 2.21 Hz, 3-H), 6.68 (dd, 1H, $^3J$ = 8.83 Hz, $^4J$ = 2.36 Hz, 5-H), 7.17 (d, 1H, $^3J$ = 7.09 Hz, 16-H), 7.38 (d, 1H, $^3J$ = 7.88 Hz, 15-H), 7.43 (d, 1H, $^3J$ = 8.99 Hz, 6-H), 7.46 (s, 1H, 19-H), 8.44 (s, 1H, 9-H), 8.74 ppm (s, 1H, 12-H); $^{13}C$ NMR (125 MHz, $CDCl_3$): $\delta$ = 12.39 (C1), 44.96 (C2), 53.05 (C21), 55.63 (C20), 70.08, 70.37, 70.49, 70.90 (C22, C23, C24, C25), 97.00 (C3), 107.13 (C7), 109.26 (C19), 110.05 (C5), 116.98 (C10), 118.38 (C15), 119.63 (C12), 120.52 (C16), 124.07 (C18), 129.96 (C6), 134.34 (C9), 139.92 (C13), 147.68 (C14), 151.50 (C4), 152.66 (C17), 155.74 (C8), 156.95 ppm (C11)
ESI-MS: m/z calcd. for $[M+H]^+$ 608.31; found 608.38;
IR (KBr, $cm^{-1}$): 1130 (s), 1239 (s), 1602 (s),1728 (s), 2855 (s), 2923 (s) UV/Vis ($CH_3CN$): $\lambda_{max}$ ($\varepsilon$) = 267 (4352), 413 nm (8649).

**Example 6**

Synthesis of 7-(diethylamino)-3-(1-(3-methoxy-4-(1,4,7,10-tetraoxa-13-azacyclopentadecan-13-yl)phenyl)-1H-1,2,3-triazol-4-yl)-2H-chromen-2-one (**15**):

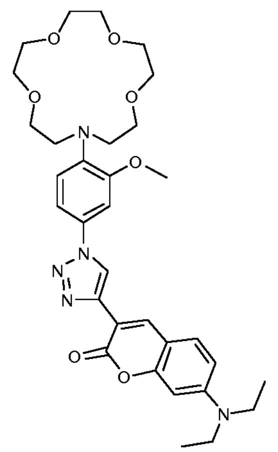

**15**

A mixture of compound **43** (117.5 mg, 0.32 mmol), 3-acetylen-7-diethylaminocoumarin **61** (77.4 mg, 0.32 mmol), Cu/C (16 mg, 20 mol%) and Triethylamin (32.5 mg, 0.32 mmol) was stirred in THF (4 mL) for 48 hours. Cu/C was filtered off through Celite, washed several times with $CHCl_3$ and the solvent was removed under reduced pressure. The residue was purified by column chromatography on silica with $CHCl_3/CH_3OH$ (95/5) as eluent. The product was obtained as a dark yellow oil, which crystallised upon standing in a freezer. (91 mg, 47%).
$^1H$ NMR (300 MHz, $CDCl_3$): $\delta$ = 1.22 (t, 6H, $^3J$ = 7.06 Hz, 1-H), 3.43 (q, 4H, $^3J$ = 7.06 Hz, 2-H), 3.51-3.55 (m, 4H, 21-H), 3.65-3.69 (m, 16H, 22-H, 23-H, 24-H,25-H), 3.91 (s, 3H, 20-H), 6.54 (d, 1H, $^4J$ = 1.98 Hz, 3-H), 6.63 (dd, 1H, $^3J$ = 8.76 Hz, $^4J$ = 2.31 Hz, 5-H), 7.13-7.23 (m, 2H, 15-H, 16-H), 7.32 (s, 1H, 19-H), 7.41 (d, 1H, $^3J$ = 8.85 Hz, 6-H), 8.65 (s, 1H, 9-H), 8.66 ppm (s, 1H, 13-H); $^{13}C$ NMR (75 MHz, $CDCl_3$): $\delta$ = 12.42 (C1), 44.82 (C2), 53.03 (C21), 55.78 (C20), 70.03, 70.42, 70.94, (C22, C23, C24, C25), 97.03 (C3), 104.73 (C19), 108.67 (C7), 109.32 (C5), 110.59 (C14), 112.45 (C16), 120.40 (C15), 122.05 (C13), 129.49 (C6), 131.15 (C10), 138.49 (C9), 140.25 (C12), 142.14 (C18), 150.78 (C4), 152.83 (C17), 156.00 (C8), 160.62 ppm (C11) ESI-MS: m/z calcd. for $[M+H]^+$ 608.31; found 608.35;
IR (KBr, $cm^{-1}$): 1130 (s), 1230 (s), 1600 (s), 1694 (s), 1720 (s), 2861 (s), 2969 (s)
UV/Vis ($CH_3CN$): $\lambda_{max}$ ($\varepsilon$) = 259 (14131), 410 nm (34604)

Elemental analysis (%) calcd. for $C_{32}H_{41}N_5O_7$ (607.70): C 63.25, H 6.80, N 11.52; found C 62.91, H 6.69, N 11.12.

**Example 7**

Synthesis of 6-(1-(4-(1,4,7,10,13-pentaoxa-16-azacyclooctadecan-16-yl)lphenyl)-1H-1,2,3-triazol-4-yl)-2-butyl-1H-benzo[de]isoquinoline-1,3(2H)-dione (**11**):

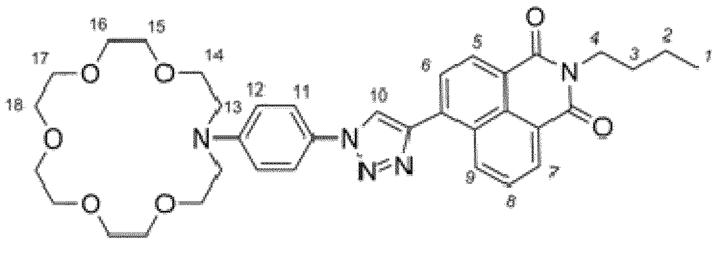

The synthesis followed the general procedure of the CuAAC reaction using precursors **28** and **50**.

The crude product was purified by column chromatography (65 x 2 cm, silica gel, $CHCl_3$/MeOH = 98:2 v/v) to yield as a yellow solid.

Yield: 0.086 g, 49 %, mp: 136.1-139.0 °C, $R_f$ = 0.52; **$^1$H NMR**, ($CDCl_3$, 600 MHz) δ (ppm), *J* (Hz): 9.1 (dd, 1H, $H^9$), 8.63 (dd, 1H, $H^7$), 8.61 (d, 1H, *J* = 8.55, $H^5$), 8.26 (s, 1H, $H^{10}$), 7.99 (d, 1H, *J* = 7.56, $H^6$), 7.79 (dd, 1H, $H^8$), 7.61, (d, 2H, *J* = 9.12, $H^{11}$), 6.81 (d, 2H, *J* = 9.12, $H^{12}$), 4.18 (t, 2H, *J* = 7.62, $H^4$), 3.74-3.64 (m, 24H, $H^{13-18}$), 1.76-1.69 (m, 2H, $H^3$), 1.45 (sx, 2H, *J* = 7,62, $H^2$), 0.98 (t, 3H, *J* = 7.38, $H^1$); **$^{13}$C NMR**, ($CDCl_3$, 150 MHz) δ (ppm): 164.34, 164.06, 148.68, 146.15, 145.66, 134.32, 132.92, 131.52, 130.81, 129.40, 128.95, 127.48, 127.35, 125.96, 122.94, 122.56, 121.52, 120.06, 112.96, 111.99, 70.98-70.82,68.8, 68.60, 51.59, 40.41, 30.32, 20.52, 13.99; HRMS ($^+$ESI): m/z calcd for $(M+H)^+$, 658.32; found, 658.35;

**Example 8**

Synthesis of 6-(4-(4-(1,4,7,10,13-pentaoxa-16-azacyclooctadecan-16-yl)lphenyl)-1H-1,2,3-triazol-1-yl)-2-ethyl-1H-benzo[de]isoquinoline-1,3(2H)-dione (**10**):

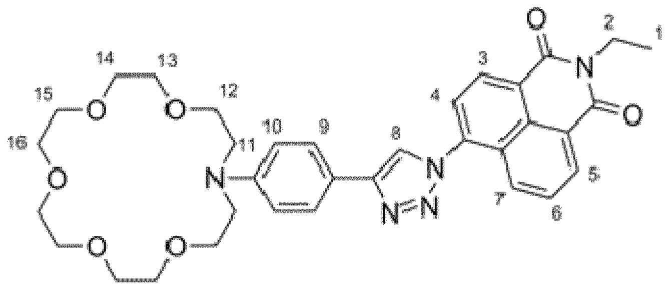

The synthesis followed the general procedure of the CuAAC reaction using precursors **27** and **51**.

The crude product was purified by column chromatography (65 x 2 cm, silica gel, $CHCl_3$/MeOH = 95:5 v/v) to yield as an orange solid.

Yield: 0.024 g, 19 %, mp: 213.6-216.4 °C, $R_f$ = 0.14; **$^1$H NMR**, ($CDCl_3$, 600 MHz) δ (ppm), *J* (Hz): 8.71 (t, 2H, *J* = 7.47 $H^3$, $H^5$), 8.36 (d, 1H, *J* = 8.1 , $H^7$), 8.09 (s, 1H, $H^8$), 7.89 (d, 1H, *J* = 7.47, $H^4$), 7.83 (tr, 1H, *J* = 5.26, $H^6$), 7.79, (d, 2H, *J* = 8.7, $H^9$), 6.79 (d, 2H, *J* = 8.82, $H^{10}$), 4.28 (q, 2H, *J* = 7.12, $H^2$), 3.74-3.68 (m, 24H, $H^{11-16}$), 1.36 (t, 3H, *J* = 7.12, $H^1$); **$^{13}$C NMR**, ($CDCl_3$, 150 MHz) δ (ppm): 163.68, 163.16, 149.13, 148.48, 138.59, 132.29, 130.82, 129.87, 129.29, 128.63, 127.31, 126.71, 123.93, 123.46, 123.19, 120.20, 117.23, 112.01, 71.04-70.93, 68.82, 51.50, 35.93, 13.47; HRMS ($^+$ESI): m/z calcd for $(M+H)^+$, 630.29; found,630.14

**Example 9**

Synthesis of 16-(4-(4-(anthracen-9-yl)-1H-1,2,3-triazol-1-yl)phenyl)-1,4,7,10,13-pentaoxa-16-azacyclooctadecane (**5**):

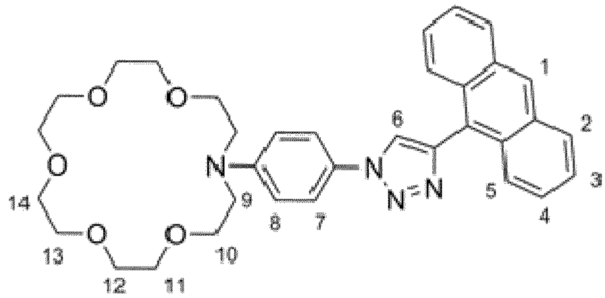

The synthesis followed the general procedure of the CuAAC reaction using precursors **28** and **52**.

The crude product was purified by column chromatography (65 x 2 cm, silica gel, $CHCl_3$/MeOH = 95:5 v/v) to yield as a light brown oil.

Yield: 23%, $^1$H NMR ($CDCl_3$, 300 MHz): $\delta$ = 3.53-3.758 (m, 24H, $H^{9-14}$), 6.31 (d, 2H, J = 9.231, $H^8$), 7.40-7.55 (m, 4H, $H^{4,2}$), 7.7 (d, 2H, J = 9.231, $H^7$), 8.03 (m, 4H, $H^{5,2}$), 8.1 (s, 1H,$H^6$), 8,57 (s, 1H, $H^1$); $^{13}$C ($CDCl_3$, 75 MHz):$\delta$ = 51.16, 68.37, 70.85, 111.24, 119.95, 122.07, 124.54, 125.21, 125.48, 126.07, 126.89, 128.64, 131.02, 131.34, 145.62, 148, 02; HRMS ($^+$ESI): m/z calcd. $(M+H)^+$, 583.34; found, 583.29; UV/Vis (MeCN,) $\lambda_{max}$ ($\varepsilon$) = 388 nm (5596 $M^{-1}$ $cm^{-1}$), 420 nm (5782 $M^{-1}$ $cm^{-1}$).

**Example 10**

Synthesis of 7-(diethylamino)-3-(1-(3-(2-methoxyethoxy)-4-(1,4,7,10,13-pentaoxa-16-azacyclooctadecan-16-yl)phenyl)-1H-1,2,3-triazol-4-yl)-2H-chromen-2-one (**7**)

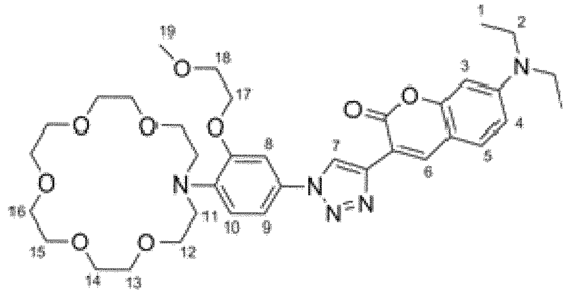

The synthesis followed the general procedure of the CuAAC reaction using precursors **56** and **61**.

The crude product was purified by column chromatography (65 x 2 cm, silica gel. $CHCl_3$/MeOH = 95:5 v/v) to yield as a yellow oil.

Yield: 13%, HRMS ($^+$ESI): m/z calc. $(M+H)^+$. 696.36; found. 696.47.

**Example 11**

Synthesis of 16-(4-(4-(anthracen-9-yl)-1H-1,2,3-triazol-1-yl)-2-(2-methoxyethoxy)phenyl)-1,4,7,10,13-pentaoxa-16-azacyclooctadecane (**6**)

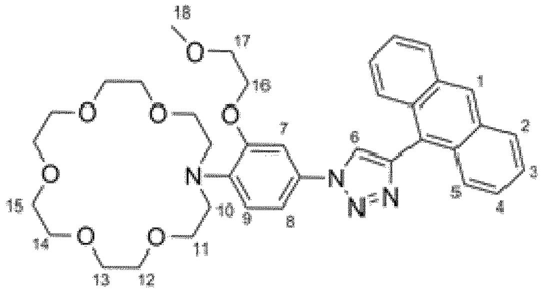

The synthesis followed the general procedure of the CuAAC reaction using precursors **56** and **52**.

The crude product was purified by column chromatography (65 x 2 cm, silica gel. $CHCl_3$/MeOH = 98:2 v/v) to yield 5 as a light brown oil.

Yield: 19.3 %; **$^1$H NMR ($CDCl_3$. 600 MHz):** $\delta$ = 3.35-3.75 (m. 27H). 3.81 (tr. 2H). 4.28 (tr. 2H). 7.31 (d. 1H). 7.45 (m. 5H). 7.58 (s. 1H). 7.91 (d. 2H). 8.05 (d. 2H). 8.21 (s. 1H). 8.55 (s. 1H);

**Example 12**

Synthesis of 2-hexadecyl-6-(1-(3-(2-methoxyethoxy)-4-(1,4,7,10,13-pentaoxa-16-azacyclooctadecan-16-yl)phenyl)-1H-1,2,3-triazol-4-yl)-1H-benzo[de]isoquinoline-1,3(2H)-dione (**9**)

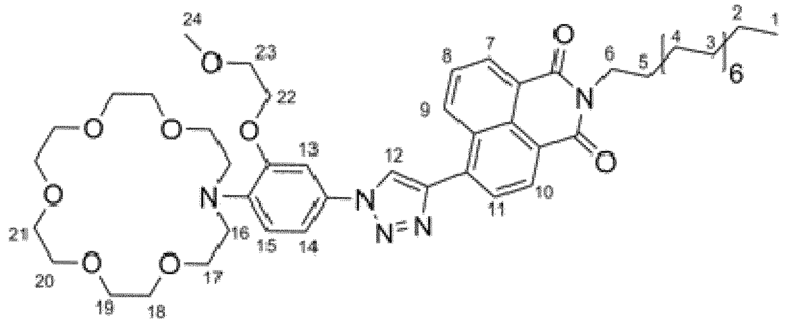

The synthesis followed the general procedure of the CuAAC reaction using precursors **56** and **57**.

The crude product was purified by column chromatography (65 x 2 cm, silica gel. $CHCl_3$/MeOH = 95:5 v/v) to yield **9** as a light brown oil.

Yield: 24 %; $^1$H NMR ($CDCl_3$. 600 MHz): $\delta$ = 0.9 (tr. 3H). 1.27 (m. 24 H). 1.39 (qu. 2H). 1.47 (qu. 2H). 1.78 (qu. 2H). 3.40-3.78 (m. 27 H). 3.83 (tr. 2H). 4.28 (tr. 2H). 7.21 (d. 1H). 7.35 (d. 1H). 7.50 (s. 1H). 7.86 (tr. 1H). 8.09 (d. 1H). 8.49 (s. 1H). 8.7 (tr. 2H). 9.17 (d. 1H).

**Example 13**

Synthesis of 7-(diethylamino)-3-{4-3-[2-methoxyethoxy)-4{bis(4-methylbenzo[5, 6, 17, 18](O, N, N', O')-]-1, 7, 16, -triaza-cryptand-[3, 2, 1]-phen-4-yl]-1H-1.2.3-triazol-1yl}-2H-chromen-2-one (**13**)

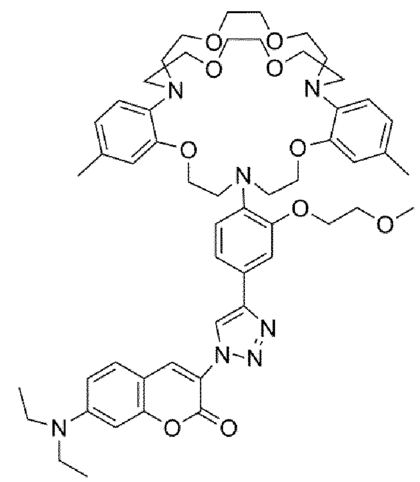

The synthesis followed the general procedure of the CuAAC. Yield: 62%, HRMS ($^+$ESI): m/z calc. (M+H)$^+$. 976.52; found. 976.59.

References

1

a) E. Tamanini, A. Katewa, L. M. Sedger, M. H. Todd and M. Watkinson, Inorg. Chem,. 2009, 48, 319; b) E. Tamanini, K. Flavin, M. Motevalli, M. H. Todd and M. Watkinson, Inorg. Chem., 2010, 49, 3798; c) S.Huang, R. J. Clark, L. Zhu, Org. Lett. 2007, 9, 4999;

2 S. Maisonneuve, Q. Fang, J. Xie, Tetrahedron, 2008, 64, 8716.
3 K.Varazo, F. Xie, D. Gulledge, Q. Wang. Tetrahedron Lett., 2008, 49, 5293.
4 H.-C. Hung, C.-W. Cheng, Y.-Y. Wang, Y.-J. Chen, W.-S. Chung, Eur. J. Org. Chem., 2009, 15, 6360.
5 D. Maity, T. Govindaraju, Inorg. Chem., 2010, 49, 7229.
6 P. D. Jarowski, Y.-L. Wu, B. Schweizer, F. Diederich, Org. Lett., 2008, 10, 3347.
7 J. D. Lewis and J. M. Moore, Dalton Trans., 2004, 1376.
8 K. Sivakumar, F. Xie and Q. Wang, Org. Lett., 2004, 6, 4603.
9 J. W. Sibert and V. Lynch, Inorg. Chem., 2007, 46, 10913; J. W. Sibert, P. B. Forshee, Inorg. Chem., 2002, 41(23), 5928.
10 D.-N. Lee, G.-J. Kim, H.-J. Kim, Tetrahedron Lett., 2009, 50, 4766.
11 E. Tamanini, S. E. J. Rigby, M. Motevalli, M. H. Todd, M. Watkinson, Chem. Eur., J. 2009, 15, 3720.
12 The FEFs were determined by division of the relative fluorescence intensities at the $\lambda_{max}$ of the complexed and free fluoroionophores.
13

a) H. He, M. A. Mortellaro, M. J. P. Leiner, R. J. Fraatz, J. K. Tusa, J. Am. Chem. Soc., 2003, 125, 1468; b) P. Padmawar, X. Yao, O. Bloch, G. T. Manley, A. S. Verkman, Nat. Methods., 2005, 2, 825; R. D. Carpenter, A. S. Verkman, Org. Lett., 2010, 12, 1160; c) H. He, M. A. Mortellaro, M. J. P. Leiner, S. T. Young, R. J. Fraatz, J. K. Tusa, Anal. Chem., 2003, 75, 549.

14 K. Sivakumar, F. Xie, B. M. Cash, S. Long, H. N. Barnhill and Q. Wang, Org. Lett., 2004, 6, 4603-4606.
15

a) J.-S. Wu, W.-M. Liu, X.-Q. Zhuang, F. Wang and S.-T. Lee, Org. Lett., 2007, 9, 33-36.
b) D.-N. Lee, G.-J. Kim, H.-J. Kim, Tetrahedron Lett., 2000, 50, 4766-4768.

16 D. Jimenez, R. Martínez-Máñez, F. Sancenón, and E.García-Breijo, Eur. J. Inorg. Chem. 2005, 2393-2403
17 J. P. Dix and F. Vögtle, Chem. Ber. 1980, 113, 457.

18 J. D.Lewis, J. M. Moore, Dalton Trans., 2004, 1376-1385.
19 J. W. Sibert, V. Lynch, Inorg. Chem., 2007, 46, 10913-10925.
20 P. A. S. Smith and J. H. Boyer, Org. Synth., 1963, 4, 74.
21 E. Tamanini, S. E. J. Rigby, M. Motevalli, M. H. Todd, M. Watkinson, Chem. Eur. J. 2009, 15, 3720-3728.
22 M. Hirokazu; S. Furuyoshi, Y. Nakatsuji, M. Okahara. Bull. Chem. Soc. Jpn. 1983, 56, 212-218.
23 R. Wortmann, C. Glania, P. Krämer, R. Matschiner, J. J. Wolff, S. Kraft, B. Treptow, E. Barbu, D. Längle and G. Görlitz, Chem. Eur. J., 1997, 3, 1765-1773.
24 Synlett 1996, 521-522
25 Chem. Commun. 2011, 47, 4685-4687
26 Huimin Guo, Maria L. Muro-Small, Shaomin Ji, Jianzhang Zhao, Felix N. Castellano, Inorg. Chem. 2010, 49, 6802-6804
27 T. Gunnlaugsson, Paul. E. Kruger and G. M. Hussey, J. of. Org. Chem., 2005, 70(25), 10875-10878.
28 Q. Xiao, T. Brown, Tetrahedron, 2007, 63, 3483.
29 K.-C. Chang, I.-H. Su, A. Senthilvelan, W.-S. Chung, Org. Lett. 2007, 9(17), 3363-3366
30 A. Torrado, B, Imperiali, J. Org. Chem. 1996, 61, 8940-8948
31 Liu et al., Journal of Polymer Science: Part A: Polymer Chemistry 2001, 39, 1495-1504
32 Kreicberga. E. Jecs. V. Kampars. Rigas Tehniskas Universitates Zinatniskie Raksti. Serija 1: Materialzinatne un Lietiska Kimija. 2005. 10. 46-53.
33 H. He und J. K. Tusa. J. Am. Chem. Soc. 2003. 125. 1468-1469
34 D. Jiménez und E.García-Breijo. Eur. J. Inorg. Chem. 2005. 2393-2403
35 T. Gunnlaugsson und M. Nieuwenhuyzen. J. Chem. Soc. Trans. 1. 2002. 1954-1962
36 H. Guo und F. N. Castellano. Inorg. Chem. 2010. 49. 6802-6804.

## Claims

**1.** Fluoroionophoric compound of the general formula I

**Ionophore – π-Linker - Fluorophore (I)**

wherein

the Ionophore is an anilino containing crown ether or cryptand with one or more anilino donor moieties as electron donors, forming a stable complex with an alkali metal ion,
the π-Linker is a heteroaromatic conjugative linking moiety and is selected from the group consisting of isomeric disubstituted 1,2,3-triazoles, preferably:

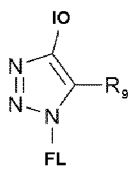 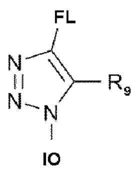 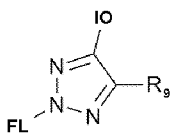 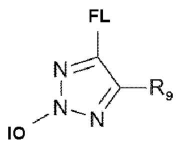 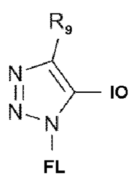 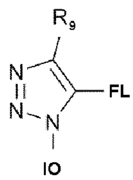

wherein
IO is the ionophore;
Fl is the fluorophore;
$R_9$ is selected from hydrogen, halogen, nitro, amino, hydroxyl, lower alkyl, selected from methyl, ethyl, propyl, isopropyl, butyl, sec-butyl, tert-butyl, pentyl, isopentyl, neopentyl, hexyl, isohexyl or neohexyl and lower alkoxy, selected from -O-methyl, -O-ethyl, -O-propyl, -O-isopropyl, -O-butyl, -O-sec-butyl, -O-tert-butyl, -O-pentyl, -O-isopentyl, -O-neopentyl, -O-hexyl, -O-isohexyl or -O-neohexyl, optionally substituted with halogen, nitro, amino, hydroxyl or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from - O-methyl, -O-ethyl, -O-propyl, -O-isopropyl, -O-butyl, -O-sec-butyl, -O-tert-butyl, -O-pentyl, -O-isopentyl, -O-neopentyl, -O-hexyl, -O-isohexyl or -O-neohexyl, and
the Fluorophore is an electron acceptor moiety.

**2.** Compound, according to claim 1, wherein the ionophore is selected from the group consisting of

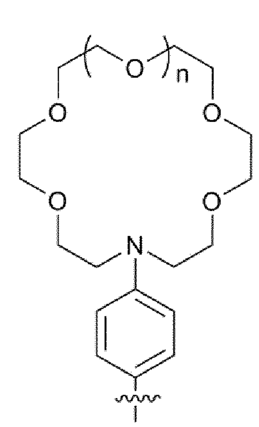

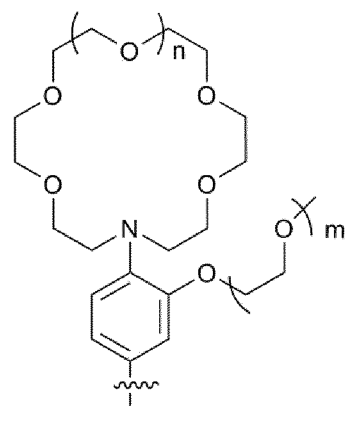

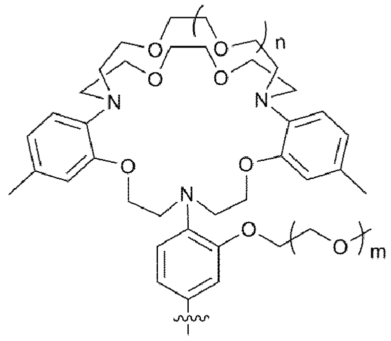

wherein

n is a number selected from 0 and 1,
m is a number selected from 0, 1, and 2, and
wherein the phenyl ring is optionally substituted with halogen, nitro, amino, hydroxyl, lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl, -O-butyl, -O-sec-butyl, -O-tert-butyl, -O-pentyl,-O-isopentyl, -O-neopentyl, -O-hexyl, -O-isohexyl or -O-neohexyl,, wherein the lower alkyl or lower alkoxy are optionally substituted with halogen, nitro, amino, hydroxyl or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from-O-methyl, -O-ethyl, -O-propyl, -O-isopropyl, -O-butyl, -O-sec-butyl, -O-tert-butyl, -O-pentyl, -O-isopentyl, -O-neopentyl, -O-hexyl, -O-isohexyl or -O-neohexyl, and
wherein the phenyl ring may optionally be a part of a condensed aromatic system, that is optionally substituted with halogen, nitro, amino, hydroxyl, or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl, -O-butyl, -O-sec-butyl, -O-tert-butyl, -O-pentyl, - O-isopentyl, -O-neopentyl, -O-hexyl, -O-isohexyl or -O-neohexyl, or phenyl, optionally substituted with halogen, nitro, amino, hydroxyl or lower alkyl, selected from methyl, ethyl, propyl, isopropyl, butyl, sec-butyl, tert-butyl, pentyl, isopentyl, neopentyl, hexyl, isohexyl or neohexyl or lower alkoxy, selected from -O-methyl, -O-ethyl, -O-propyl, -O-isopropyl, -O-butyl, -O-sec-butyl, -O-tert-butyl, -O-pentyl, -O-isopentyl, - O-neopentyl, -O-hexyl, -O-isohexyl or -O-neohexyl.

**3.** Compound, according to claim 1 or 2, wherein the π-Linker is selected from the group consisting of 1,4-disubstitued triazoles, namely

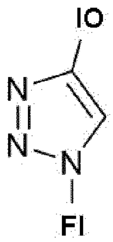

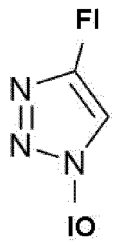

wherein IO is the ionophore and FI is the fluorophore.

**4.** Compound, according to claim 1, wherein the fluorophore moiety is represented by the formula

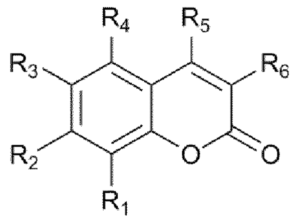

wherein

$R_1$, $R_4$, $R_5$ = H, lower alkyl, selected from methyl, ethyl, propyl, isopropyl, butyl, sec-butyl, tert-butyl, pentyl, isopentyl, neopentyl, hexyl, isohexyl or neohexyl, $CF_3$, MeO, halogen, $NO_2$, CN, $R_2$, $R_3$ = H, $NH_2$, N(lower alkyl)$_2$, lower alkyl, selected from methyl, ethyl, propyl, isopropyl, butyl, sec-butyl, tert-butyl, pentyl, isopentyl, neopentyl, hexyl, isohexyl or neohexyl, optionally substituted with carboxyl or carbonyl, diethyl amino
$R_6$ = alkinyl, azide.

**5.** Compound, according to claim 1, wherein the fluorophore moiety is selected from the group consisting of

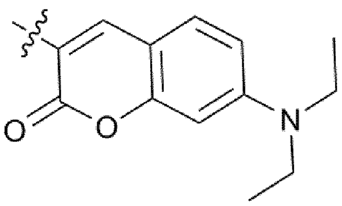

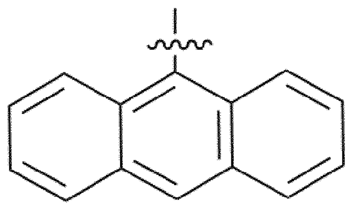

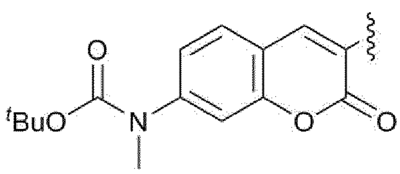

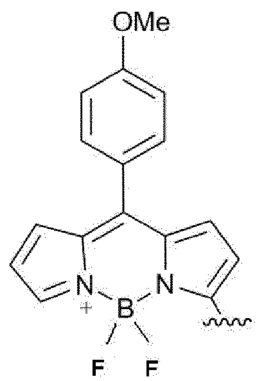

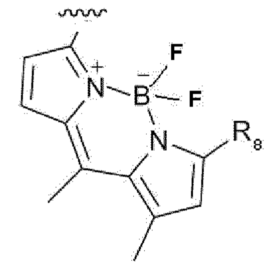

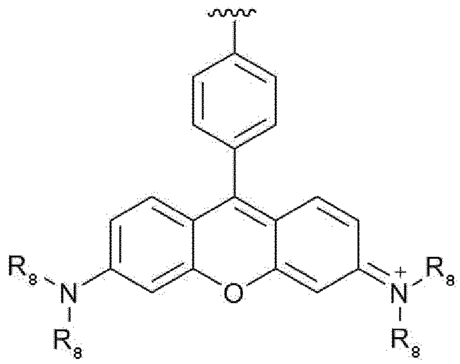

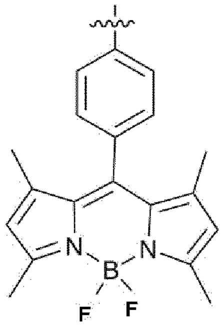

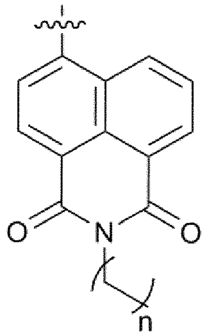

wherein

n is integer ranging from 0 to 15;
$R_8$ is selected from hydrogen or lower alkyl, selected from methyl, ethyl, propyl, isopropyl, butyl, sec-butyl, tert-butyl, pentyl, isopentyl, neopentyl, hexyl, isohexyl or neohexyl.

**6.** Compound, according to claim 1, namely

3-{4-[4-(1,4,7,10,13-pentaoxa-16-azacyclooctadecan-16-yl)phenyl]-1H-1,2,3-triazol-1-yl}-7-(diethylamino)-2H-chromen-2-one,
3-{1-[4-(1,4,7,10,13-pentaoxa-16-azacyclooctadecan-16-yl)phenyl]-1H-1,2,3-triazol-4-yl}-7-(diethylamino)-2H-chromen-2-one,
7-(diethylamino)-3-{1-[3-(2-methoxyethoxy)-4-(1,4,7,10,13-pentaoxa-16-azacyclooctadecan-16-yl)phenyl]-1H-1,2,3-triazol-4-yl}-2H-chromen-2-one,
7-(diethylamino)-3-{4-[3-(2-methoxyethoxy)-4-(1,4,7,10,13-pentaoxa-16-azacyclooctadecan-16-yl)phenyl]-1H-1,2,3-triazol-1-yl}-2H-chromen-2-one,
7-(diethylamino)-3-{4-3-[2-methoxyethoxy)-4{bis(4-methylbenzo[5, 6, 17, 18](O, N, N', O')-]-1, 7, 16, -triaza-cryptand-[3, 2, 1]-phen-4-yl]-1H-1.2.3-triazol-1yl}-2H-chromen-2-one,
7-(diethylamino)-3-{4-[3-(2-methoxyethoxy]-4{bis(4-methylbenzo[5, 6, 17, 18](O, N, N', O')-]-1, 7, 16, -triaza-cryptand-[3, 2, 1]-phen-4-yl]-1H-1.2.3-triazol-1yl}-2H-chromen-2-one,
3-{4-[4-(1,4,7,10-tetraoxa-13-azacyclopentadecan-13-yl)phenyl]-1H-1,2,3-triazol-1-yl}-7-(diethylamino)-2H-chromen-2-one,
3-{1-[4-(1,4,7,10-tetraoxa-13-azacyclopentadecan-13-yl)phenyl]-1H-1,2,3-triazol-4-yl}-7-(diethylamino)-2H-chromen-2-one,
7-(diethylamino)-3-{4-[3-methoxy-4-(1,4,7,10-tetraoxa-13-azacyclopentadecan-13-yl)phenyl]-1H-1,2,3-triazol-1-yl}-2H-chromen-2-one,
7-(diethylamino)-3-{1-[3-methoxy-4-(1,4,7,10-tetraoxa-13-azacyclopentadecan-13-yl)phenyl]-1H-1,2,3-triazol-4-yl}-2H-chromen-2-one,
7-(diethylamino)-3-{4-[3-methoxy-4{bis(4-methylbenzo[5, 6, 17, 18](O, N, N', O')-]-1, 7, 16, -triaza-cryptand-[3, 2, 1]-phen-4-yl]-1H-1.2.3-triazol-1yl}-2H-chromen-2-one,
7-(diethylamino)-3-{1-[3methoxy-4{bis(4-methylbenzo[5, 6, 17, 18](O, N, N', O')-]-1, 7, 16, -triaza-cryptand-[3, 2, 1]-phen-1-yl]-1H-1.2.3-triazol-1yl}-2H-chromen-2-one,
6-{4-[4-(1,4,7,10,13-pentaoxa-16-azacyclooctadecan-16-yl)phenyl]-1H-1,2,3-triazol-1-yl}-2-ethyl-1H-benzo[de]isoquinoline-1,3(2H)-dione,
6-{1-[4-(1,4,7,10,13-pentaoxa-16-azacyclooctadecan-16-yl)phenyl]-1H-1,2,3-triazol-4-yl}-2-ethyl-1H-benzo[de]isoquinoline-1,3(2H)-dione,
2-ethyl-6-{4-[3-(2-methoxyethoxy)-4-(1,4,7,10,13-pentaoxa-16-azacyclooctadecan-16-yl)phenyl]-1H-1,2,3-triazol-1-yl}-1H-benzo[de]isoquinoline-1,3(2H)-dione,
2-ethyl-6-{1-[3-(2-methoxyethoxy)-4-(1,4,7,10,13-pentaoxa-16-azacyclooctadecan-16-yl)phenyl]-1H-1,2,3-triazol-4-yl}-1H-benzo[de]isoquinoline-1,3(2H)-dione,
2-ethyl-6-{4-[3-(2-methoxyethoxy)-4{bis(4-methylbenzo[5, 6, 17, 18](O, N, N', O')-]-1, 7, 16, -triaza-cryptand-[3, 2, 2]-phen-1-yl]-1H-1.2.3-triazol 1-yl}-1H-benzo[de]isoquinoline-1 ,3(2H)-dione,
2-ethyl-6-{1-[3-(2-methoxyethoxy)-4{bis(4-methylbenzo[5, 6, 17, 18](O, N, N', O')-]-1, 7, 16, -triaza-cryptand-[3, 2, 2]-phen-4-yl]-1H-1.2.3-triazol 1-yl}-1H-benzo[de]isoquinoline-1,3(2H)-dione,
6-{4-[4-(1,4,7,10-tetraoxa-13-azacyclopentadecan-13-yl)phenyl]-1H-1,2,3-triazol-1-yl}-2-ethyl-1H-benzo[de]isoquinoline-1,3(2H)-dione,
6-{1-[4-(1,4,7,10-tetraoxa-13-azacyclopentadecan-13-yl)phenyl]-1H-1,2,3-triazol-4-yl}-2-ethyl-1H-benzo[de]isoquinoline-1,3(2H)-dione,
2-ethyl-6-{4-[3-methoxy-4-(1,4,7,10-tetraoxa-13-azacyclopentadecan-13-yl)phenyl]-1H-1,2,3-triazol-1-yl}-1H-benzo[de]isoquinoline-1,3(2H)-dione,
2-ethyl-6-{1-[3-methoxy-4-(1,4,7,10-tetraoxa-13-azacyclopentadecan-13-yl)phenyl]-1H-1,2,3-triazol-4-yl}-1H-benzo[de]isoquinoline-1,3(2H)-dione,
2-ethyl-6-{4-[3-methoxy-4{bis(4-methylbenzo[5, 6, 17, 18](O, N, N', O')-]-1, 7, 16, -triaza-cryptand-[3, 2, 1]-phen-1-yl]-1H-1.2.3-triazol 1-yl}-1H-benzo[de]isoquinoline-1,3(2H)-dione,
2-ethyl-6-{1-[3-methoxy-4{bis(4-methylbenzo[5, 6, 17, 18](O, N, N', O')-]-1, 7, 16, -triaza-cryptand-[3, 2, 1]-phen-4-yl]-1H-1.2.3-triazol 1-yl}-1H-benzo[de]isoquinoline-1,3(2H)-dione,
7-{4-[4-(1,4,7,10,13-pentaoxa-16-azacyclooctadecan-16-yl)phenyl]-1H-1,2,3-triazol-1-yl}-5,5-difluoro-1,3,9,10-tetramethyl-5H-dipyrrolo[1,2-c:1',2'-f][1,3,2]diazaborinin-4-ium-5-uide,
7-{1-[4-(1,4,7,10,13-pentaoxa-16-azacyclooctadecan-16-yl)phenyl]-1H-1,2,3-triazol-4-yl}-5,5-difluoro-1,3,9,10-tetramethyl-5H-dipyrrolo[1,2-c:1',2'-f][1,3,2]diazaborinin-4-ium-5-uide,
5,5-difluoro-7-{4-[3-(2-methoxyethoxy)-4-(1,4,7,10,13-pentaoxa-16-azacyclooctadecan-16-yl)phenyl]-1H-1,2,3-triazol-1-yl}-1,3,9,10-tetramethyl-5H-dipyrrolo[1,2-c:1',2'-f][1,3,2]diazaborinin-4-ium-5-uide,
5,5-difluoro-7-{1-[3-(2-methoxyethoxy)-4-(1,4,7,10,13-pentaoxa-16-azacyclooctadecan-16-yl)phenyl]-1H-1,2,3-triazol-4-yl}-1,3,9,10-tetramethyl-5H-dipyrrolo[1,2-c:10',2'-f][1,3,2]diazaborinin-4-ium-5-uide,
5,5-difluoro-7{4-[3-(2-methoxyethoxy]-4(bis(4-methylbenzo[5,6,17, 18](O,N,N',O')-]-1,7,16,-triaza-cryptand- [3, 2,2]-phen-4-yl]-1H-1.2.3-triazol-1-yl}-4-1,3,9,10-tetramethyl-5H-dipyrrolo[1,2-c:1',2'-f][1,3,2]diazaborinin-4-ium-5-uide,
5,5-difluoro-7{1-[3-(2-methoxyethoxy]-4(bis(4-methylbenzo[5,6,17, 18](O,N,N',O')-]-1,7,16,-triaza-cryptand- [3, 2,2]-phen-1-yl]-1H-1.2.3-triazol-1-yl}-4-1,3,9,10-tetramethyl-5H-dipyrrolo[1,2-c:1',2'-f][1,3,2]diazaborinin-4-ium-5-uide,
7-{4-[4-(1,4,7,10-tetraoxa-13-azacyclopentadecan-13-yl)phenyl]-1H-1,2,3-triazol-1-yl}-5,5-difluoro-1,3,9,10-tetramethyl-5H-dipyrrolo[1,2-c:1',2'-f][1,3,2]diazaborinin-4-ium-5-uide,
7-{1-[4-(1,4,7,10-tetraoxa-13-azacyclopentadecan-13-yl)phenyl]-1H-1,2,3-triazol-4-yl}-5,5-difluoro-1,3,9,10-tetramethyl-5H-dipyrrolo[1,2-c:1',2'-f][1,3,2]diazaborinin-4-ium-5-uide,
5,5-difluoro-7-{4-[3-methoxy-4-(1,4,7,10-tetraoxa-13-azacyclopentadecan-13-yl)phenyl]-1H-1,2,3-triazol-1-yl}-1,3,9,10-tetramethyl-5H-dipyrrolo[1,2-c:1 ',2'-f][1,3,2]diazaborinin-4-ium-5-uide,
5,5-difluoro-7-{1-[3-methoxy-4-(1,4,7,10-tetraoxa-13-azacyclopentadecan-13-yl)phenyl]-1H-1,2,3-triazol-4-yl}-1,3,9,10-tetramethyl-5H-dipyrrolo[1,2-c:1',2'-f][1,3,2]diazaborinin-4-ium-5-uide,
5,5-difluoro-7{4-[3-methoxy-4(bis(4-methylbenzo[5,6,17,18](O,N,N', O')-]-1,7,16,-triaza-cryptand-[3,2,1]-phen-4-yl]-1H-1.2.3-triazol-1-yl}-4-1,3,9,10-tetramethyl-5H-dipyrrolo[1,2-c:1',2'-f][1,3,2]diazaborinin-4-ium-5-uide,
5,5-difluoro-7{1-[3-methoxy-4(bis(4-methylbenzo[5,6,17,18](O,N,N', O')-]-1,7,16,-triaza-cryptand-[3,2,2]-phen-1-yl]-1H-1.2.3-triazol-1-yl}-4-1,3,9,10-tetramethyl-5H-dipyrrolo[1,2-c:1',2'-f][1,3,2]diazaborinin-4-ium-5-uide,
10-(4-{4-[4-(1,4,7,10,13-pentaoxa-16-azacyclooctadecan-16-yl)phenyl]-1H-1,2,3-triazol-1-yl}phenyl)-5,5-difluoro-1,3,7,9-tetramethyl-5H-dipyrrolo[1,2-c:1',2'-f][1,3,2]diazaborinin-4-ium-5-uide,
10-(4-{1-[4-(1,4,7,10,13-pentaoxa-16-azacyclooctadecan-16-yl)phenyl]-1H-1,2,3-triazol-4-yl}phenyl)-5,5-difluoro-1,3,7,9-tetramethyl-5H-dipyrrolo[1,2-c:1',2'-f][1,3,2]diazaborinin-4-ium-5-uide,
5,5-difluoro-10-(4-{4-[3-(2-methoxyethoxy)-4-(1,4,7,10,13-pentaoxa-16-azacyclooctadecan-16-ylphenyl]-1H-1,2,3-triazol-1-yl}phenyl)-1,3,7,9-tetramethyl-5H-dipyrrolo[1,2-c:1',2'-f][1,3,2]diazaborinin-4-ium-5-uide,
5,5-difluoro-10-(4-{1-[3-(2-methoxyethoxy)-4-(1,4,7,10,13-pentaoxa-16-azacyclooctadecan-16-yl)phenyl]-1H-1,2,3-triazol-4-yl}phenyl)-1,3,7,9-tetramethyl-5H-dipyrrolo[1,2-c:1',2'-f][1,3,2]diazaborinin-4-ium-5-uide,
5,5-difluoro-10-(4-{4-[3-(2-methoxyethoxy)-4(bis(4-methylbenzo[5,6, 17,18](O,N,N',O')-]-1,7,16,-triaza-cryptand-[3,2,2]-phen-4-yl]-1H-1.2.3-triazol1-yl}1,3,7,9-tetramethyl-5H-dipyrrolo)(1,2-c:1',2'-f)(1,3,2)diazaborinin-4-ium-5-uide,
5,5-difluoro-10-(4-{1-[3-(2-methoxyethoxy)-4(bis(4-methylbenzo[5,6, 17,18](O,N,N',O')-]-1,7,16,-triaza-cryptand-[3,2,2]-phen-4-yl]-1H-1.2.3-triazol 4-yl}1,3,7,9-tetramethyl-5H-dipyrrolo)(1,2-c:1',2'-f)(1,3,2)diazaborinin-4-ium-5-uide,
10-(4-{4-[4-(1,4,7,10-tetraoxa-13-azacyclopentadecan-13-yl)phenyl]-1H-1,2,3-triazol-1-yl}phenyl)-5,5-difluoro-1,3,7,9-tetramethyl-5H-dipyrrolo[1,2-c:1',2'-f][1,3,2]diazaborinin-4-ium-5-uide,
10-(4-{1-[4-(1,4,7,10-tetraoxa-13-azacyclopentadecan-13-yl)phenyl]-1H-1,2,3-triazol-4-yl}phenyl)-5,5-d ifluoro-1,3,7,9-tetramethyl-5H-dipyrrolo[1,2-c:1',2'-f][1,3,2]diazaborinin-4-ium-5-uide,
5,5-difluoro-10-(4-{4-[3-methoxy-4-(1,4,7,10-tetraoxa-13-azacyclopentadecan-13-yl)phenyl]-1H-1,2,3-triazol-1-yl}phenyl)-1,3,7,9-tetramethyl-5H-dipyrrolo[1,2-c:1 ',2'-f][1,3,2]diazaborinin-4-ium-5-uide,
5,5-difluoro-10-(4-{1-[3-methoxy-4-(1,4,7,10-tetraoxa-13-azacyclopentadecan-13-yl)phenyl]-1H-1,2,3-triazol-4-yl}phenyl)-1,3,7,9-tetramethyl-5H-dipyrrolo[1,2-c:1 ',2'-f][1,3,2]diazaborinin-4-ium-5-uide,
5,5-difluoro-10-(4-[3-methoxy-4{bis(4-methylbenzo[5,6,17,18](O,N, N',O')-]-1,7,16,-triaza-cryptand-[3,2,1]-phen-4-yl]-1H-1.2.3-triazol-1-yl}-(1,3,7,9-tetramethyl-5H-dipyrrolo)(1,2-c:1',2'-f)(1,3,2)diazabohnin-4-ium-5-uide, and
5,5-difluoro-10-(1-[3-methoxy-4{bis(4-methylbenzo[5,6,17,18](O,N, N',O')-]-1,7,16,-triaza-cryptand-[3,2,1]-phen-1-yl]-1H-1.2.3-triazol-1-yl}-(1,3,7,9-tetramethyl-5H-dipyrrolo)(1,2-c:1',2'-f)(1,3,2)diazaborinin-4-ium-5-uide.

**7.** Complex, consisting of a compound according to claim 1 and an alkali metal cation.

**8.** Complex, according to claim 7, wherein the alkali metal cation is selected from the group consisting of $Na^+$ and $K^+$.

**9.** Method for the determination of metal cations in a sample, comprising the steps of

a) contacting the metal cations with at least one compound according to claim 1,
b) forming a complex according to claim 7, whereupon fluorescence and/or luminescence appears or changes,
c) measuring the resulting fluorescence and/or luminescence.

**10.** Method, according to claim 9, wherein the metal cations are $Na^+$ or $K^+$, or a combination thereof.

**11.** Method, according to claim 9, wherein the at least one compound according to claim 1 selectively complexes the metal cations to be determined.

**12.** Use of a compound according to claim 1 for the qualitative and/or quantitative determination of metal cations in a sample.

**Patentansprüche**

**1.** Fluoroionophore Verbindung der allgemeinen Formel I

**Ionophor – π-Linker - Fluorophor (I)**

worin

der Ionophor ein Anilino-enthaltender Kronenether oder Kryptand mit einem oder mehreren Anilino-Donorteilen als Elektronendonatoren ist, einen stabilen Komplex mit einen Alkalimetalion bildend,
der π-Linker ein heteroaromatischer durch Konjugation verbindender Teil ist und ausgewählt ist aus der Gruppe, bestehend aus isomeren disubstituierten 1,2,3-Triazolen, bevorzugt:

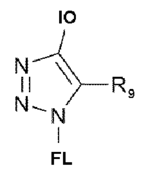 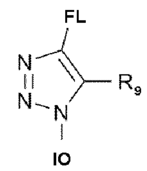 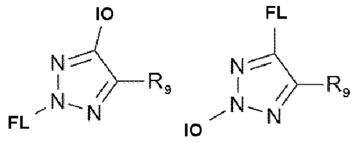 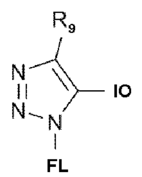 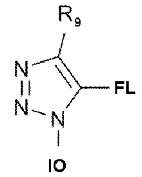

worin
IO der Ionophor ist;
Fl der Fluorophor ist;
$R_9$ ausgewählt ist aus Wasserstoff, Halogen, Nitro, Amino, Hydroxyl, niedrigem Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, und niedrigem Alkoxy, ausgewählt aus -O-Methyl, -O-Ethyl, -O-Propyl, -O-Isopropyl, -O-Butyl, -O-sec-Butyl, -O-tert-Butyl, -O-Pentyl, -O-Isopentyl, -O-Neopentyl, -O-Hexyl, -O-Isohexyl oder -O-Neohexyl, optional substituiert mit Halogen, Nitro, Amino, Hydroxyl oder niedrigem Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, oder niedrigem Alkoxy, ausgewählt aus -O-Methyl, -O-Ethyl, -O-Propyl, -O-Isopropyl, -O-Butyl, -O-sec-Butyl, -O-tert-Butyl, -O-Pentyl, -O-Isopentyl, -O-Neopentyl, -O-Hexyl, -O-Isohexyl oder -O-Neohexyl, und
der Fluorophor ein Elektronenakzeptorteil ist.

2. Verbindung, gemäß Anspruch 1, worin der Ionophor ausgewählt ist aus der Gruppe, bestehend aus

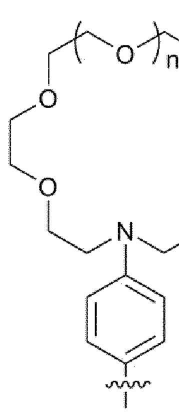 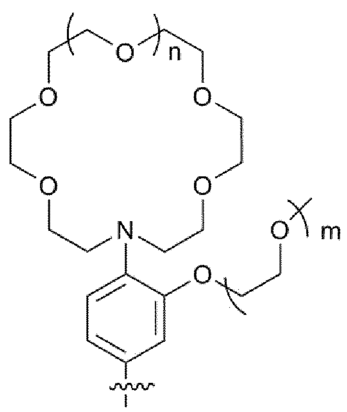

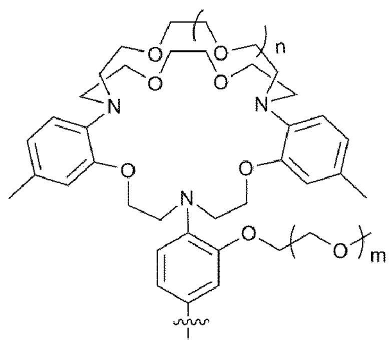

worin

n eine Zahl ist, ausgewählt aus 0 und 1,
m eine Zahl ist, ausgewählt aus 0, 1 und 2, und
worin der Phenylring optional substituiert ist mit Halogen, Nitro, Amino, Hydroxyl, niedrigem Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, oder niedrigem Alkoxy, ausgewählt aus -O-Methyl, -O-Ethyl, -O-Propyl, -O-Isopropyl, -O-Butyl, -O-sec-Butyl, -O-tert-Butyl, -O-Pentyl, -O-Isopentyl, -O-Neopentyl, -O-Hexyl, -O-Isohexyl oder -O-Neohexyl, worin das niedrige Alkyl oder niedrige Alkoxy optional substituiert sind mit Halogen, Nitro, Amino, Hydroxyl oder

niedrigem Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, oder niedrigem Alkoxy, ausgewählt aus -O-methyl, -O-Ethyl, -O-Propyl, -O-Isopropyl, -O-Butyl, -O-sec-Butyl, -O-tert-Butyl, -O-Pentyl, -O-Isopentyl, -O-Neopentyl, -O-Hexyl, -O-Isohexyl oder -O-Neohexyl, und

worin der Phenylring optional ein Teil eines kondensierten aromatischen Systems sein kann, das optional substituiert ist mit Halogen, Nitro, Amino, Hydroxyl, oder niedrigem Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, oder niedrigem Alkoxy, ausgewählt aus -O-Methyl, -O-Ethyl, -O-Propyl, -O-Isopropyl, -O-Butyl, -O-sec-Butyl, -O-tert-Butyl, -O-Pentyl, -O-Isopentyl, -O-Neopentyl, -O-Hexyl, -O-Isohexyl oder -O-Neohexyl, oder Phenyl, optional substituiert mit Halogen, Nitro, Amino, Hydroxyl oder niedrigem Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, oder niedrigem Alkoxy, ausgewählt aus -O-Methyl, -O-Ethyl, -O-Propyl, -O-Isopropyl, -O-Butyl, -O-sec-Butyl, -O-tert-Butyl, -O-Pentyl, -O-Isopentyl, -O-Neopentyl, -O-Hexyl, -O-Isohexyl oder -O-Neohexyl.

**3.** Verbindung, gemäß Anspruch 1 oder 2, worin der $\pi$-Linker ausgewählt ist aus der Gruppe, bestehend aus 1,4-disubstituierten Triazolen, nämlich

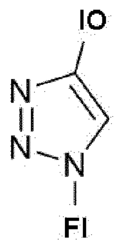

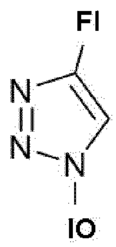

worin IO der Ionophor ist und FI der Fluorophor ist.

**4.** Verbindung, gemäß Anspruch 1, worin der Fluorophorteil repräsentiert wird durch die Formel

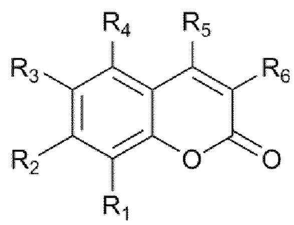

worin

$R_1$, $R_4$, $R_5$ = H, niedriges Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, $CF_3$, MeO, Halogen, $NO_2$, CN, $R_2$, $R_3$ = H, $NH_2$, N(niedriges Alkyl)$_2$, niedriges Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl, optional substituiert mit Carboxyl oder Carbonyl, Diethylamino,
$R_6$ = Alkinyl, Azid ist.

**5.** Verbindung, gemäß Anspruch 1, worin der Fluorophorteil ausgewählt ist aus der Gruppe, bestehend aus

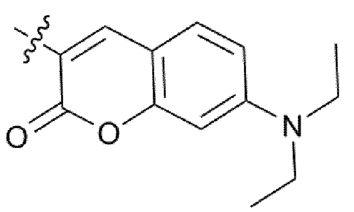

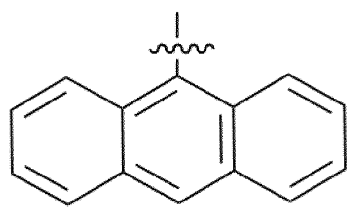

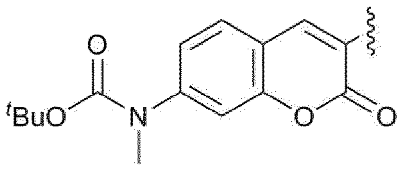

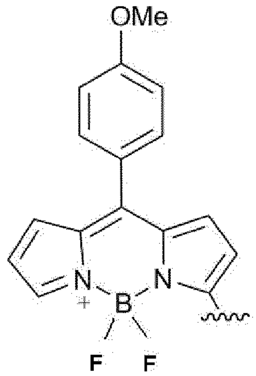

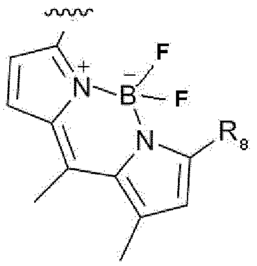

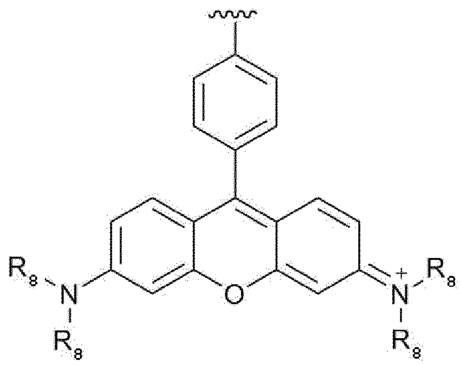

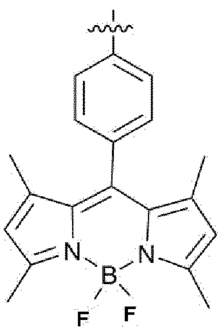

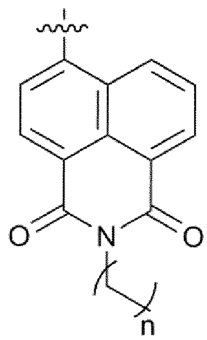

worin

n eine ganze Zahl ist, die von 0 bis 15 reicht;
$R_8$ ausgewählt ist aus Wasserstoff oder niedrigem Alkyl, ausgewählt aus Methyl, Ethyl, Propyl, Isopropyl, Butyl, sec-Butyl, tert-Butyl, Pentyl, Isopentyl, Neopentyl, Hexyl, Isohexyl oder Neohexyl.

**6.** Verbindung, gemäß Anspruch 1, nämlich

3-{4-[4-(1,4,7,10,13-Pentaoxa-16-azacyclooctadecan-16-yl)phenyl]-1H-1,2,3-triazol-1-yl}-7-(diethylamino)-2H-chromen-2-on,
3-{1-[4-(1,4,7,10,13-Pentaoxa-16-azacyclooctadecan-16-yl)phenyl]-1H-1,2,3-triazol-4-yl}-7-(diethylamino)-2H-chromen-2-on,
7-(Diethylamino)-3-{1-[3-(2-methoxyethoxy)-4-(1,4,7,10,13-pentaoxa-16-azacyclooctadecan-16-yl)phenyl]-1H-1,2,3-triazol-4-yl}-2H-chromen-2-on,

7-(Diethylamino)-3-{4-[3-(2-methoxyethoxy)-4-(1,4,7,10,13-pentaoxa-16-azacyclooctadecan-16-yl)phenyl]-1H-1,2,3-triazol-1-yl}-2H-chromen-2-on,
7-(Diethylamino)-3-{4-3-[2-methoxyethoxy)-4{bis(4-methylbenzo[5, 6, 17,18](O,N,N',O')-]-1,7,16,-triaza-cryptand-[3,2,1]-phen-4-yl]-1H-1.2.3-triazol-1yl}-2H-chromen-2-on,
7-(Diethylamino)-3-{4-[3-(2-methoxyethoxy]-4{bis(4-methylbenzo[5,6, 17,18](O,N,N',O')-]-1,7,16,-triaza-cryptand-[3,2,1]-phen-4-yl]-1H-1.2.3-triazol-1yl}-2H-chromen-2-on,
3-{4-[4-(1,4,7,10-Tetraoxa-13-azacyclopentadecan-13-yl)phenyl]-1H-1,2,3-triazol-1-yl}-7-(diethylamino)-2H-chromen-2-on,
3-{1-[4-(1,4,7,10-Tetraoxa-13-azacyclopentadecan-13-yl)phenyl]-1H-1,2,3-triazol-4-yl}-7-(diethylamino)-2H-chromen-2-on,
7-(Diethylamino)-3-{4-[3-methoxy-4-(1,4,7,10-tetraoxa-13-azacyclopentadecan-13-yl)phenyl]-1H-1,2,3-triazol-1-yl}-2H-chromen-2-on,
7-(Diethylamin-[3-methoxy-4-(1,4,7,10-tetraoxa-13-azacyclopentadecan-13-yl)phenyl]-1H-1,2,3-triazol-4-yl}-2H-chromen-2-on,
7-(Diethylamino)-3-{4-[3-methoxy-4{bis(4-methylbenzo[5,6,17,18](O, N,N',O')-]-1,7,16,-triaza-cryptand-[3,2,1]-phen-4-yl]-1H-1.2.3-triazol-1yl}-2H-chromen-2-on,
7-(Diethylamino)-3-{1-[3methoxy-4{bis(4-methylbenzo[5,6,17,18](O, N,N',O')-]-1,7,16,-triaza-cryptand-[3,2,1]-phen-1-yl]-1H-1.2.3-triazol-1yl}-2H-chromen-2-on,
6-{4-[4-(1,4,7,10,13-Pentaoxa-16-azacyclooctadecan-16-yl)phenyl]-1H-1,2,3-triazol-1-yl}-2-ethyl-1H-benzo[de]isochinolin-1,3(2H)-dion,
6-{1-[4-(1,4,7,10,13-Pentaoxa-16-azacyclooctadecan-16-yl)phenyl]-1H-1,2,3-triazol-4-yl}-2-ethyl-1H-benzo[de]isochinolin-1,3(2H)-dion,
2-Ethyl-6-{4-[3-(2-methoxyethoxy)-4-(1,4,7,10,13-pentaoxa-16-azacyclooctadecan-16-yl)phenyl]-1H-1,2,3-triazol-1-yl}-1H-benzo[de]isochinolin-1,3(2H)-dion,
2-Ethyl-6-{1-[3-(2-methoxyethoxy)-4-(1,4,7,10,13-pentaoxa-16-azacyclooctadecan-16-yl)phenyl]-1H-1,2,3-triazol-4-yl}-1H-benzo[de]isochinolin-1,3(2H)-dion,
2-Ethyl-6-{4-[3-(2-methoxyethoxy)-4{bis(4-methylbenzo[5,6,17,18](O, N,N',O')-]-1,7,16,-triaza-cryptand-[3,2,2]-phen-1-yl]-1H-1.2.3-triazol 1-yl}-1H-benzo[de]isochinolin-1,3(2H)-dion,
2-Ethyl-6-{1-[3-(2-methoxyethoxy)-4{bis(4-methylbenzo[5,6,17,18](O, N,N',O')-]-1,7,16,-triaza-cryptand-[3,2,2]-phen-4-yl]-1H-1.2.3-triazol 1-yl}-1H-benzo[de]isochinolin-1,3(2H)-dion,
6-{4-[4-(1,4,7,10-Tetraoxa-13-azacyclopentadecan-13-yl)phenyl]-1H-1,2,3-triazol-1-yl}-2-ethyl-1H-benzo[de]isochinolin-1,3(2H)-dion,
6-{1-[4-(1,4,7,10-Tetraoxa-13-azacyclopentadecan-13-yl)phenyl]-1H-1,2,3-triazol-4-yl}-2-ethyl-1H-benzo[de]isochinolin-1,3(2H)-dion,
2-Ethyl-6-{4-[3-methoxy-4-(1,4,7,10-tetraoxa-13-azacyclopentadecan-13-yl)phenyl]-1H-1,2,3-triazol-1-yl}-1H-benzo[de]isochinolin-1,3(2H)-dion,
2-Ethyl-6-{1-[3-methoxy-4-(1,4,7,10-tetraoxa-13-azacyclopentadecan-13-yl)phenyl]-1H-1,2,3-triazol-4-yl}-1H-benzo[de]isochinolin-1,3(2H)-dion,
2-Ethyl-6-{4-[3-methoxy-4{bis(4-methylbenzo[5,6,17,18](O,N,N',O')-]-1,7,16,-triaza-cryptand-[3,2,1]-phen-1-yl]-1H-1.2.3-triazol1-yl}-1H-benzo[de]isochinolin-1,3(2H)-dion,
2-Ethyl-6-{1-[3-methoxy-4{bis(4-methylbenzo[5,6,17,18](O,N,N',O')-] -1,7,16,-triaza-cryptand-[3,2,1]-phen-4-yl]-1H-1.2.3-triazol1-yl}-1H-benzo[de]isochinolin-1,3(2H)-dion,
7-{4-[4-(1,4,7,10,13-Pentaoxa-16-azacyclooctadecan-16-yl)phenyl]-1H-1,2,3-triazol-1-yl}-5,5-difluor-1,3,9,10-tetramethyl-5H-dipyrrolo[1,2-c:1',2'-f][1,3,2]diazaborinin-4-ium-5-uid,
7-{1-[4-(1,4,7,10,13-Pentaoxa-16-azacyclooctadecan-16-yl)phenyl]-1H-1,2,3-triazol-4-yl}-5,5-difluor-1,3,9,10-tetramethyl-5H-dipyrrolo[1,2-c:1',2'-f][1,3,2]diazaborinin-4-ium-5-uid,
5,5-Difluor-7-{4-[3-(2-methoxyethoxy)-4-(1,4,7,10,13-pentaoxa-16-azacyclooctadecan-16-yl)phenyl]-1H-1,2,3-triazol-1-yl}-1,3,9,10-tetramethyl-5H-dipyrrolo[1,2-c:1',2'-f][1,3,2]diazaborinin-4-ium-5-uid,
5,5-Difluor-7-{1-[3-(2-methoxyethoxy)-4-(1,4,7,10,13-pentaoxa-16-azacyclooctadecan-16-yl)phenyl]-1H-1,2,3-triazol-4-yl}-1,3,9,10-tetramethyl-5H-dipyrrolo[1,2-c:1',2'-f][1,3,2]diazaborinin-4-ium-5-uid,
5,5-Difluor-7{4-[3-(2-methoxyethoxy]-4(bis(4-methylbenzo[5,6,17, 18](O,N,N',O')-]-1,7,16,-triaza-cryptand-[3,2,2]-phen-4-yl]-1H-1.2.3-triazol-1-yl}-4-1,3,9,10-tetramethyl-5H-dipyrrolo[1,2-c:1',2'-f][1,3,2]diazaborinin-4-ium-5-uid,
5,5-Difluor-7{1-[3-(2-methoxyethoxy]-4(bis(4-methylbenzo[5,6,17, 18](O,N,N',O')-]-1,7,16,-triaza-cryptand-[3,2,2]-phen-1-yl]-1H-1.2.3-triazol -1-yl}-4-1,3,9,10-tetramethyl-5H-dipyrrolo[1,2-c:1',2'-f][1,3,2]diazaborinin-4-ium-5-uid,
7-{4-[4-(1,4,7,10-Tetraoxa-13-azacyclopentadecan-13-yl)phenyl]-1H-1,2,3-triazol-1-yl}-5,5-difluor-1,3,9,10-tetramethyl-5H-dipyrrolo[1,2-c:1',2'-f][1,3,2]diazaborinin-4-ium-5-uid,

7-{1-[4-(1,4,7,10-Tetraoxa-13-azacyclopentadecan-13-yl)phenyl]-1H-1,2,3-triazol-4-yl}-5,5-difluor-1,3,9,10-tetramethyl-5H-dipyrrolo[1,2-c:1',2'-f][1,3,2]diazaborinin-4-ium-5-uid,
5,5-Difluor-7-{4-[3-methoxy-4-(1,4,7,10-tetraoxa-13-azacyclopentadecan-13-yl)phenyl]-1H-1,2,3-triazol-1-yl}-1,3,9,10-tetramethyl-5H-dipyrrolo[1,2-c:1',2'-f][1,3,2]diazaborinin-4-ium-5-uid,
5,5-Difluor-7-{1-[3-methoxy-4-(1,4,7,10-tetraoxa-13-azacyclopentadecan-13-yl)phenyl]-1H-1,2,3-triazol-4-yl}-1,3,9,10-tetramethyl-5H-dipyrrolo[1,2-c:1',2'-f][1,3,2]diazaborinin-4-ium-5-uid,
5,5-Difluor-7{4-[3-methoxy-4(bis(4-methylbenzo[5,6,17,18](O,N,N', O')-]-1,7,16,-triaza-cryptand-[3,2,1]-phen-4-yl]-1H-1.2.3-triazol-1-yl}-4-1,3,9,10-tetramethyl-5H-dipyrrolo[1,2-c:1',2'-f][1,3,2]diazaborinin-4-ium-5-uid,
5,5-Difluor-7{1-[3-methoxy-4(bis(4-methylbenzo[5,6,17,18](O,N,N', O')-]-1,7,16,-triaza-cryptand-[3,2,2]-phen-1-yl]-1H-1.2.3-triazol-1-yl}-4-1,3,9,10-tetramethyl-5H-dipyrrolo[1,2-c:1',2'-f][1,3,2]diazaborinin-4-ium-5-uid,
10-(4-{4-[4-(1,4,7,10,13-Pentaoxa-16-azacyclooctadecan-16-yl)phenyl]-1H-1,2,3-triazol-1-yl}phenyl)-5,5-difluor-1,3,7,9-tetramethyl-5H-dipyrrolo[1,2-c:1',2'-f][1,3,2]diazaborinin-4-ium-5-uid,
10-(4-{1-[4-(1,4,7,10,13-Pentaoxa-16-azacyclooctadecan-16-yl)phenyl]-1H-1,2,3-triazol-4-yl}phenyl)-5,5-difluor-1,3,7,9-tetramethyl-5H-dipyrrolo[1,2-c:1',2'-f][1,3,2]diazaborinin-4-ium-5-uid,
5,5-Difluor-10-(4-{4-[3-(2-methoxyethoxy)-4-(1,4,7,10,13-pentaoxa-16-azacyclooctadecan-16-ylphenyl]-1H-1,2,3-triazol-1-yl}phenyl)-1,3,7,9-tetramethyl-5H-dipyrrolo[1,2-c:1',2'-f][1,3,2]diazaborinin-4-ium-5-uid,
5,5-Difluor-10-(4-{1-[3-(2-methoxyethoxy)-4-(1,4,7,10,13-pentaoxa-16-azacyclooctadecan-16-yl)phenyl]-1H-1,2,3-triazol-4-yl}phenyl)-1,3,7,9-tetramethyl-5H-dipyrrolo[1,2-c:1',2'-f][1,3,2]diazaborinin-4-ium-5-uid,
5,5-Difluor-10-(4-{4-[3-(2-methoxyethoxy)-4(bis(4-methylbenzo[5,6,17, 18](O,N,N',O')-]-1,7,16,-triaza-cryptand-[3,2,2]-phen-4-yl]-1H-1.2.3-triazol1-yl}1,3,7,9-tetramethyl-5H-dipyrrolo)(1,2-c:1',2'-f)(1,3,2)diazaborinin-4-ium-5-uid,
5,5-Difluor-10-(4-{1-[3-(2-methoxyethoxy)-4(bis(4-methylbenzo[5,6,17, 18](O,N,N',O')-]-1,7,16,-triaza-cryptand-[3,2,2]-phen-4-yl]-1H-1.2.3-triazol4-yl}1,3,7,9-tetramethyl-5H-dipyrrolo)(1,2-c:1',2'-f)(1,3,2)diazaborinin-4-ium-5-uid,
10-(4-{4-[4-(1,4,7,10-Tetraoxa-13-azacyclopentadecan-13-yl)phenyl]-1H-1,2,3-triazol-1-yl}phenyl)-5,5-difluor-1,3,7,9-tetramethyl-5H-dipyrrolo[1,2-c:1',2'-f][1,3,2]diazaborinin-4-ium-5-uid,
10-(4-{1-[4-(1,4,7,10-Tetraoxa-13-azacyclopentadecan-13-yl)phenyl]-1H-1,2,3-triazol-4-yl}phenyl)-5,5-difluor-1,3,7,9-tetramethyl-5H-dipyrrolo[1,2-c:1',2'-f][1,3,2]diazaborinin-4-ium-5-uid,
5,5-Difluor-10-(4-{4-[3-methoxy-4-(1,4,7,10-tetraoxa-13-azacyclopentadecan-13-yl)phenyl]-1H-1,2,3-triazol-1-yl}phenyl)-1,3,7,9-tetramethyl-5H-dipyrrolo[1,2-c:1',2'-f][1,3,2]diazaborinin-4-ium-5-uid,
5,5-Difluor-10-(4-{1-[3-methoxy-4-(1,4,7,10-tetraoxa-13-azacyclopentadecan-13-yl)phenyl]-1H-1,2,3-triazol-4-yl}phenyl)-1,3,7,9-tetramethyl-5H-dipyrrolo[1,2-c:1',2'-f][1,3,2]diazaborinin-4-ium-5-uid,
5,5-Difluor-10-(4-[3-methoxy-4{bis(4-methylbenzo[5,6,17,18](O,N,N', O')-]-1,7,16,-triaza-cryptand-[3,2,1]-phen-4-yl]-1H-1.2.3-triazol-1-yl}-(1,3,7,9-tetramethyl-5H-dipyrrolo)(1,2-c:1',2'-f)(1,3,2)diazaborinin-4-ium-5-uid, und
5,5-Difluor-10-(1-[3-methoxy-4{bis(4-methylbenzo[5,6,17,18](O,N,N', O')-]-1,7,16,-triaza-cryptand-[3,2,1]-phen-1-yl]-1H-1.2.3-triazol-1-yl}-(1,3,7,9-tetramethyl-5H-dipyrrolo)(1,2-c:1',2'-f)(1,3,2)diazaborinin-4-ium-5-uid.

**7.** Komplex, bestehend aus einer Verbindung gemäß Anspruch 1 und einem Alkalimetallkation.

**8.** Komplex, gemäß Anspruch 7, worin das Alkalimetallkation ausgewählt ist aus der Gruppe, bestehend aus $Na^+$ und $K^+$.

**9.** Verfahren zur Bestimmung von Metallkationen in einer Probe, umfassend die Schritte des

a) in Kontakt Bringens der Metallkationen mit wenigstens einer Verbindung gemäß Anspruch 1,
b) Bildens eines Komplexes gemäß Anspruch 7, woraufhin Fluoreszenz und/oder Lumineszenz auftritt oder sich ändert,
c) Messens der resultierenden Fluoreszenz und/oder Lumineszenz.

**10.** Verfahren, gemäß Anspruch 9, worin die Metallkationen $Na^+$ oder $K^+$ oder eine Kombination davon sind.

**11.** Verfahren, gemäß Anspruch 9, worin wenigstens eine Verbindung gemäß Anspruch 1 selektiv die zu bestimmenden Metallkationen komplexiert.

**12.** Verwendung einer Verbindung, gemäß Anspruch 1 für die qualitative und/oder quantitative Bestimmung von Metallkationen in einer Probe.

## Revendications

1. Composé fluoroionophore de formule générale I

**Ionophore - π-Lieur – Fluorophore (I)**

dans laquelle

l'ionophore est un éther-couronne ou un cryptand contenant un anilino avec une ou plusieurs fractions donneurs d'anilino en tant que donneurs d'électrons, formant un complexe stable avec un ion de métal alcalin,
le $\pi$-lieur est une fraction de liaison par conjugaison hétéroaromatique et est choisie dans le groupe constitué par les 1,2,3-triazoles disubstitués isomères, de préférence :

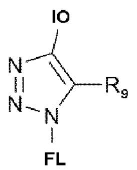
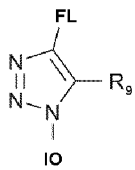
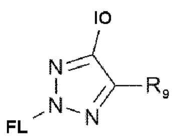
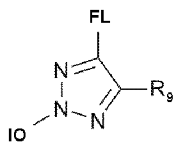
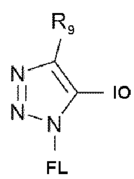
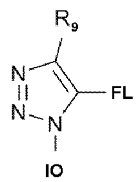

dans lesquelles
IO est l'ionophore ;
Fl est le fluorophore ;
$R_9$ est choisi parmi un hydrogène, un atome d'halogène, un nitro, un amino, un hydroxyle, un alkyle inférieur, choisi parmi le méthyle, l'éthyle, le propyle, l'isopropyle, le butyle, le sec-butyle, le tert-butyle, le pentyle, l' isopentyle, le néopentyle, l'hexyle, l'isohexyle ou le néohexyle et un alcoxy inférieur, choisi parmi un -O-méthyle, -O-éthyle, -O-propyle, -O-isopropyle, -O-butyle, -O-sec-butyle, -O-tert-butyle, -O-pentyle, -O-isopentyle, -O-néopentyle, O-hexyle, -O-isohexyle ou -O-néohexyle, éventuellement substitué par un atome d'halogène, un nitro, un amino, un hydroxyle ou un alkyle inférieur, choisi parmi le méthyle, l'éthyle, le propyle, l'isopropyle, le butyle, le sec-butyle, le tert-butyle, le pentyle, l'isopentyle, le néopentyle, l'hexyle, l'isohexyle ou le néohexyle ou un alcoxy inférieur, choisi parmi un -O-méthyle, -O-éthyle, -O-propyle, -O-isopropyle, -O-butyle, -O-sec-butyle, -O-tert-butyle, -O-pentyle, -O-isopentyle, -O-néopentyle, - O-hexyle, -O-isohexyle ou -O-néohexyle, et
le fluorophore est une fraction accepteur d'électrons.

2. Composé, selon la revendication 1, dans lequel l'ionophore est choisi dans le groupe constitué par

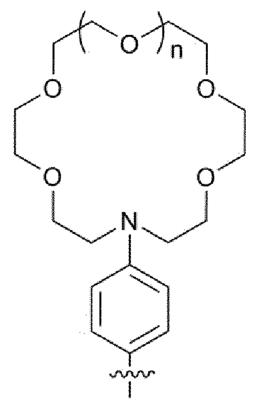
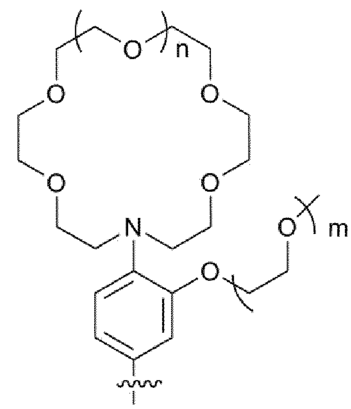
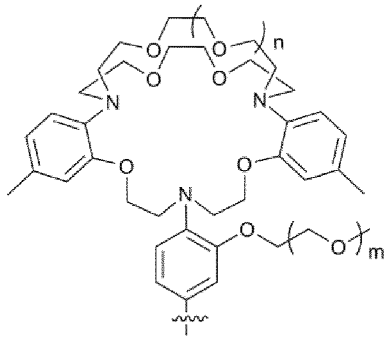

dans lesquelles

n est un nombre choisi parmi 0 et 1,
m est un nombre choisi parmi 0, 1 et 2, et
dans lesquelles le cycle phényle est éventuellement substitué par un atome d'halogène, un nitro, un amino, un hydroxyle, un alkyle inférieur, choisi parmi le méthyle, l'éthyle, le propyle, l'isopropyle, le butyle, le sec-butyle,

le tert-butyle, le pentyle, l'isopentyle, le néopentyle, l'hexyle, l'isohexyle ou le néohexyle ou un alcoxy inférieur, choisi parmi un -O-méthyle, -O-éthyle, -O-propyle, -O-isopropyle, -O-butyle, -O-sec-butyle, -O-tert-butyle, -O-pentyle, -O-isopentyle, - O-néopentyle, -O-hexyle, -O-isohexyle ou -O-néohexyle, dans lequel l'alkyle inférieur ou l'alcoxy inférieur est éventuellement substitué par un atome d'halogène, un nitro, un amino, un hydroxyle ou un alkyle inférieur, choisi parmi le méthyle, l'éthyle, le propyle, l'isopropyle, le butyle, le sec-butyle, le tert-butyle, le pentyle, l'isopentyle, le néopentyle, l'hexyle, l'isohexyle ou le néohexyle ou un alcoxy inférieur, choisi parmi un -O-méthyle, -O-éthyle, -O-propyle, -O-isopropyle, -O-butyle, -O-sec-butyle, -O-tert-butyle, -O-pentyle, -O-isopentyle, -O-néopentyle, -O-hexyle, -O-isohexyle ou -O-néohexyle, et

dans lesquelles le cycle phényle peut éventuellement faire partie d'un système aromatique condensé, qui est éventuellement substitué par un atome d'halogène, un nitro, un amino, un hydroxyle ou un alkyle inférieur, choisi parmi le méthyle, l'éthyle, le propyle, l'isopropyle, le butyle, le sec-butyle, le tert-butyle, le pentyle, l' isopentyle, le néopentyle, l'hexyle, l'isohexyle ou le néohexyle ou un alcoxy inférieur, choisi parmi un -O-méthyle, -O-éthyle, -O-propyle, -O-isopropyle, -O-butyle, -O-sec-butyle, -O-tert-butyle, -O-pentyle, -O-isopentyle, -O-néopentyle, -O-hexyle, -O-isohexyle ou -O-néohexyle, ou un phényle, éventuellement substitué par un atome d'halogène, un nitro, un amino, un hydroxyle ou un alkyle inférieur, choisi parmi le méthyle, l'éthyle, le propyle, l'isopropyle, le butyle, le sec-butyle, le tert-butyle, le pentyle, l'isopentyle, le néopentyle, l'hexyle, l'isohexyle ou le néohexyle ou un alcoxy inférieur, choisi parmi un -O-méthyle, -O-éthyle, -O-propyle, -O-isopropyle, -O-butyle, -O-sec-butyle, -O-tert-butyle, -O-pentyle, -O-isopentyle, -O-néopentyle, -O-hexyle, -O-isohexyle ou -O-néohexyle.

**3.** Composé selon la revendication 1 ou 2, dans lequel le $\pi$-lieur est choisi dans le groupe constitué par des triazoles 1,4-disubstitués, à savoir

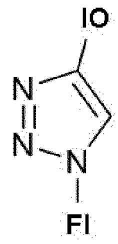

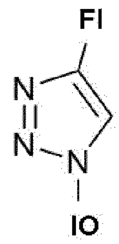

dans lesquelles IO est l'ionophore et Fl est le fluorophore.

**4.** Composé, selon la revendication 1, dans lequel la fraction fluorophore est représentée par la formule

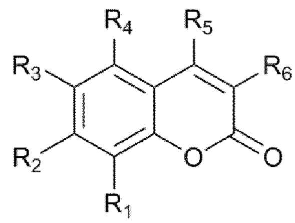

dans laquelle

$R_1$, $R_4$, $R_5$ = H, alkyle inférieur, choisi parmi le méthyle, l'éthyle, le propyle, l'isopropyle, le butyle, le sec-butyle, le tert-butyle, le pentyle, l'isopentyle, le néopentyle, l'hexyle, l'isohexyle ou le néohexyle, $CF_3$, MeO, un atome d'halogène, $NO_2$, CN,
$R_2$, $R_3$ = H, $NH_2$, N(alkyle inférieur)2, alkyle inférieur, choisi parmi le méthyle, l'éthyle, le propyle, l'isopropyle, le butyle, le sec-butyle, le tert-butyle, le pentyle, l'isopentyle, le néopentyle, l'hexyle, l'isohexyle ou le néohexyle, éventuellement substitué par un carboxyle ou un carbonyle, un diéthylamino
$R_6$ = alcynyle, azoture.

**5.** Composé selon la revendication 1, dans lequel la fraction fluorophore est choisie dans le groupe constitué par

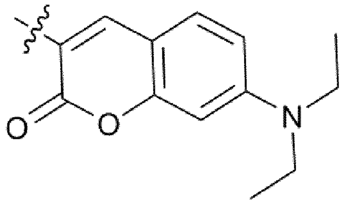

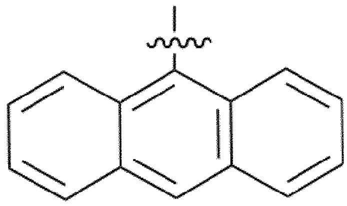

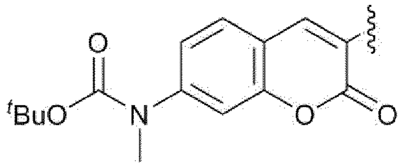

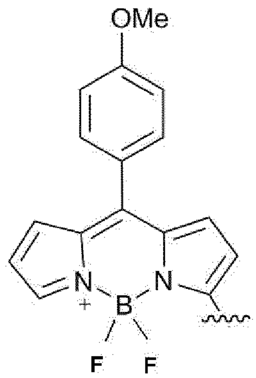

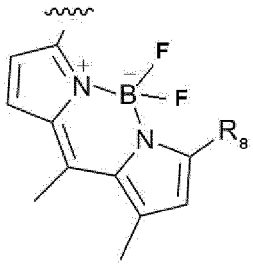

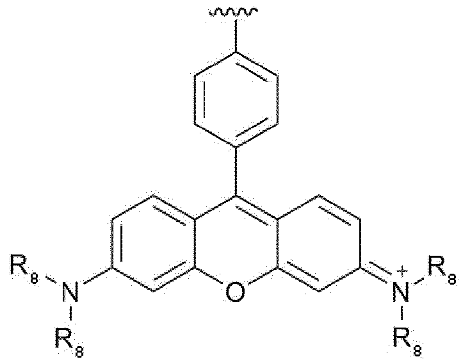

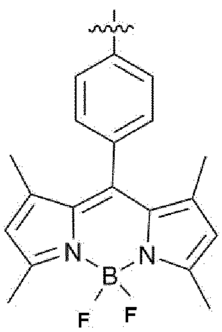

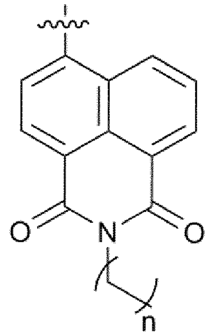

dans lesquelles

n est un nombre entier allant de 0 à 15 ;

$R_8$ est choisi parmi un hydrogène ou un alkyle inférieur, choisi parmi le méthyle, l'éthyle, le propyle, l'isopropyle,

le butyle, le sec-butyle, le tert-butyle, le pentyle, l'isopentyle, le néopentyle, l'hexyle, l'isohexyle ou le néohexyle.

**6.** Composé, selon la revendication 1, à savoir

la 3-{4-[4-(1,4,7,10,13-pentaoxa-16-azacyclooctadécan-16-yl)phényl]-1H-1,2,3-triazol-1-yl}-7-(diéthylamino)-2H-chromén-2-one,
la 3-{1-[4-(1,4,7,10,13-pentaoxa-16-azacyclooctadécan-16-yl)phényl]-1H-1,2,3-triazol-4-yl}-7-(diéthylamino)-2H-chromén-2-one,
la 7-(diéthylamino)-3-{1-[3-(2-méthoxyéthoxy)-4-(1,4,7,10,13-pentaoxa-16-azacyclooctadécan-16-yl)phényl]-1H-1,2,3-triazol-4-yl}-2H-chromén-2-one,
la 7-(diéthylamino)-3-{4-[3-(2-méthoxyéthoxy)-4-(1,4,7,10,13-pentaoxa-16-azacyclooctadécan-16-yl)phényl]-1H-1,2,3-triazol-1-yl}-2H-chromén-2-one,
la 7-(diéthylamino)-3-{4-3-[2-méthoxyéthoxy)-4{bis(4-méthylbenzo[5,6,17,18](O,N,N',O')-]-1,7,16,-triaza-cryptand-[3,2,1]-phén-4-yl]-1H-1.2.3-triazol-1yl}-2H-chromén-2-one,
la 7-(diéthylamino)-3-{4-[3-(2-méthoxyéthoxy]-4{bis(4-méthylbenzo[5,6,17,18](O,N,N',O')-]-1,7,16,-triaza-cryptand-[3,2,1]-phén-4-yl]-1H-1.2.3-triazol-1yl}-2H-chromén-2-one,
la 7-(diéthylamino)-3-{4-[3-(2-méthoxyéthoxy]-4{bis(4-méthylbenzo[5,6,17,18](O,N,N',O')-]-1,7,16,-triaza-cryptand-[3,2,1]-phén-4-yl]-1H-1.2.3-triazol-1yl}-2H-chromén-2-one,
la 3-{4-[4-(1,4,7,10-tétraoxa-13-azacyclopentadécan-13-yl)phényl]-1H-1,2,3-triazol-1-yl}-7-(diéthylamino)-2H-chromén-2-one,
la 3-{1-[4-(1,4,7,10-tétraoxa-13-azacyclopentadécan-13-yl)phényl]-1H-1,2,3-triazol-4-yl}-7-(diéthylamino)-2H-chromén-2-one,
la 7-(diéthylamino)-3-{4-[3-méthoxy-4-(1,4,7,10-tétraoxa-13-azacyclopentadécan-13-yl)phényl]-1H-1,2,3-triazol-1-yl}-2H-chromén-2-one,
la 7-(diéthylamino)-3-{1-[3-méthoxy-4-(1,4,7,10-tétraoxa-13-azacyclopentadécan-13-yl)phényl]-1H-1,2,3-triazol-4-yl}-2H-chromén-2-one,
la 7-(diéthylamino)-3-{4-[3-méthoxy-4{bis(4-méthylbenzo[5,6,17,18](O,N,N',O')-]-1,7,16,-triaza-cryptand-[3,2,1]-phén-4-yl]-1H-1.2.3-triazol-1yl}-2H-chromén-2-one,
la 7-(diéthylamino)-3-{1-[3méthoxy-4{bis(4-méthylbenzo[5,6,17,18](O,N,N',O')-]-1,7,16,-triaza-cryptand-[3,2,1]-phén-1-yl]-1H-1.2.3-triazol-1yl}-2H-chromén-2-one,
la 6-{4-[4-(1,4,7,10,13-pentaoxa-16-azacyclooctadécan-16-yl)phényl]-1H-1,2,3-triazol-1-yl}-2-éthyl-1H-benzo[de]isoquinoléine-1,3(2H)-dione,
la 6-{1-[4-(1,4,7,10,13-pentaoxa-16-azacyclooctadécan-16-yl)phényl]-1H-1,2,3-triazol-4-yl}-2-éthyl-1H-benzo[de]isoquinoléine-1,3(2H)-dione,
la 2-éthyl-6-{4-[3-(2-méthoxyéthoxy)-4-(1,4,7,10,13-pentaoxa-16-azacyclooctadécan-16-yl)phényl]-1H-1,2,3-triazol-1-yl}-1H-benzo[de]isoquinoléine-1,3(2H)-dione,
la 2-éthyl-6-{1-[3-(2-méthoxyéthoxy)-4-(1,4,7,10,13-pentaoxa-16-azacyclooctadécan-16-yl)phényl]-1H-1,2,3-triazol-4-yl}-1H-benzo[de]isoquinoléine-1,3(2H)-dione,
la 2-éthyl-6-{4-[3-(2-méthoxyéthoxy)-4{bis(4-méthylbenzo[5,6,17,18](O,N,N',O')-]-1,7,16,-triaza-cryptand-[3,2,2]-phén-1-yl]-1H-1.2.3-triazol1-yl}-1H-benzo[de]isoquinoléine-1,3(2H)-dione,
la 2-éthyl-6-{1-[3-(2-méthoxyéthoxy)-4{bis(4-méthylbenzo[5,6,17,18](O,N,N',O')-]-1,7,16,-triaza-cryptand-[3,2,2]-phén-4-yl]-1H-1.2.3-triazol1-yl}-1H-benzo[de]isoquinoléine-1,3(2H)-dione,
la 6-{4-[4-(1,4,7,10-tétraoxa-13-azacyclopentadécan-13-yl)phényl]-1H-1,2,3-triazol-1-yl}-2-éthyl-1H-benzo[de]isoquinoléine-1,3(2H)-dione,
la 6-{1-[4-(1,4,7,10-tétraoxa-13-azacyclopentadécan-13-yl)phényl]-1H-1,2,3-triazol-4-yl}-2-éthyl-1H-benzo[de]isoquinoléine-1,3(2H)-dione,
la 2-éthyl-6-{4-[3-méthoxy-4-(1,4,7,10-tétraoxa-13-azacyclopentadécan-13-yl)phényl]-1H-1,2,3-triazol-1-yl}-1H-benzo[de]isoquinoléine-1,3(2H)-dione,
la 2-éthyl-6-{1-[3-méthoxy-4-(1,4,7,10-tétraoxa-13-azacyclopentadécan-13-yl)phényl]-1H-1,2,3-triazol-4-yl}-1H-benzo[de]isoquinoléine-1,3(2H)-dione,
la 2-éthyl-6-{4-[3-méthoxy-4{bis(4-méthylbenzo[5,6,17,18](O,N,N',O')-]-1,7,16,-triaza-cryptand-[3,2,1]-phén-1-yl]-1H-1.2.3-triazol1-yl}-1H-benzo[de]isoquinoléine-1,3(2H)-dione,
la 2-éthyl-6-{1-[3-méthoxy-4{bis(4-méthylbenzo[5,6,17,18](O,N,N',O')-]-1,7,16,-triaza-cryptand-[3,2,1]-phén-4-yl]-1H-1.2.3-triazol1-yl}-1H-benzo[de]isoquinoléine-1,3(2H)-dione,
le 7-{4-[4-(1,4,7,10,13-pentaoxa-16-azacyclooctadécan-16-yl)phényl]-1H-1,2,3-triazol-1-yl}-5,5-difluoro-1,3,9,10-tétraméthyl-5H-dipyrrolo[1,2-c:1',2'-f][1,3,2]diazaborinin-4-ium-5-uide,
le 7-{1-[4-(1,4,7,10,13-pentaoxa-16-azacyclooctadécan-16-yl)phényl]-1H-1,2,3-triazol-4-yl}-5,5-difluoro-1,3,9,10-tétraméthyl-5H-dipyrrolo[1,2-c:1',2'-f][1,3,2]diazaborinin-4-ium-5-uide,

le 5,5-difluoro-7-{4-[3-(2-méthoxyéthoxy)-4-(1,4,7,10,13-pentaoxa-16-azacyclooctadécan-16-yl)phényl]-1H-1,2,3-triazol-1-yl}-1,3,9,10-tétraméthyl-5H-dipyrrolo[1,2-c:1',2'-f][1,3,2]diazaborinin-4-ium-5-uide,
le 5,5-difluoro-7-{1-[3-(2-méthoxyéthoxy)-4-(1,4,7,10,13-pentaoxa-16-azacyclooctadécan-16-yl)phényl]-1H-1,2,3-triazol-4-yl}-1,3,9,10-tétraméthyl-5H-dipyrrolo[1,2-c:1',2'-f][1,3,2]diazaborinin-4-ium-5-uide,
le 5,5-difluoro-7{4-[3-(2-méthoxyéthoxy]-4(bis(4-méthylbenzo[5,6,17,18](O,N,N',O')-]-1,7,16,-triaza-cryptand-[3,2,2]-phén-4-yl]-1H-1.2.3-triazol-1-yl}-4-1,3,9,10-tétraméthyl-5H-dipyrrolo[1,2-c:1',2'-f][1,3,2]diazaborinin-4-ium-5-uide,
le 5,5-difluoro-7{1-[3-(2-méthoxyéthoxy]-4(bis(4-méthylbenzo[5,6,17,18](O,N,N',O')-]-1,7,16,-triaza-cryptand-[3,2,2]-phén-1-yl]-1H-1.2.3-triazol-1-yl}-4-1,3,9,10-tétraméthyl-5H-dipyrrolo[1,2-c:1',2'-f][1,3,2]diazaborinin-4-ium-5-uide,
le 7-{4-[4-(1,4,7,10-tétraoxa-13-azacyclopentadécan-13-yl)phényl]-1H-1,2,3-triazol-1-yl}-5,5-difluoro-1,3,9,10-tétraméthyl-5H-dipyrrolo[1,2-c:1',2'-f][1,3,2]diazaborinin-4-ium-5-uide,
le 7-{1-[4-(1,4,7,10-tétraoxa-13-azacyclopentadécan-13-yl)phényl]-1H-1,2,3-triazol-4-yl}-5,5-difluoro-1,3,9,10-tétraméthyl-5H-dipyrrolo[1,2-c:1',2'-f][1,3,2]diazaborinin-4-ium-5-uide,
le 5,5-difluoro-7-{4-[3-méthoxy-4-(1,4,7,10-tétraoxa-13-azacyclopentadécan-13-yl)phényl]-1H-1,2,3-triazol-1-yl}-1,3,9,10-tétraméthyl-5H-dipyrrolo[1,2-c:1',2'-f][1,3,2]diazaborinin-4-ium-5-uide,
le 5,5-difluoro-7-{1-[3-méthoxy-4-(1,4,7,10-tétraoxa-13-azacyclopentadécan-13-yl)phényl]-1H-1,2,3-triazol-4-yl}-1,3,9,10-tétraméthyl-5H-dipyrrolo[1,2-c:1',2'-f][1,3,2]diazaborinin-4-ium-5-uide,
le 5,5-difluoro-7-{4-[3-méthoxy-4(bis(4-méthylbenzo[5,6,17,18](O,N,N',O')-]-1,7,16,-triaza-cryptand-[3,2,1]-phén-4-yl]-1H-1.2.3-triazol-1-yl}-4-1,3,9,10-tétraméthyl-5H-dipyrrolo[1,2-c:1',2'-f][1,3,2]diazaborinin-4-ium-5-uide,
le 5,5-difluoro-7-{1-[3-méthoxy-4(bis(4-méthylbenzo[5,6,17,18](O,N,N',O')-]-1,7,16,-triaza-cryptand-[3,2,2]-phén-1-yl]-1H-1.2.3-triazol-1-yl}-4-1,3,9,10-tétraméthyl-5H-dipyrrolo[1,2-c:1',2'-f][1,3,2]diazaborinin-4-ium-5-uide,
le 10-(4-{4-[4-(1,4,7,10,13-pentaoxa-16-azacyclooctadécan-16-yl)phényl]-1H-1,2,3-triazol-1-yl}phényl)-5,5-difluoro-1,3,7,9-tétraméthyl-5H-dipyrrolo[1,2-c:1',2'-f][1,3,2]diazaborinin-4-ium-5-uide,
le 10-(4-{1-[4-(1,4,7,10,13-pentaoxa-16-azacyclooctadécan-16-yl)phényl]-1H-1,2,3-triazol-4-yl}phényl)-5,5-difluoro-1,3,7,9-tétraméthyl-5H-dipyrrolo[1,2-c:1',2'-f][1,3,2]diazaborinin-4-ium-5-uide,
le 5,5-difluoro-10-(4-{4-[3-(2-méthoxyéthoxy)-4-(1,4,7,10,13-pentaoxa-16-azacyclooctadécan-16-ylphényl]-1H-1,2,3-triazol-1-yl}phényl)-1,3,7,9-tétraméthyl-5H-dipyrrolo[1,2-c:1',2'-f][1,3,2]diazaborinin-4-ium-5-uide,
le 5,5-difluoro-10-(4-{1-[3-(2-méthoxyéthoxy)-4-(1,4,7,10,13-pentaoxa-16-azacyclooctadécan-16-yl)phényl]-1H-1,2,3-triazol-4-yl}phényl)-1,3,7,9-tétraméthyl-5H-dipyrrolo[1,2-c:1',2'-f][1,3,2]diazaborinin-4-ium-5-uide,
le 5,5-difluoro-10-(4-{4-[3-(2-méthoxyéthoxy)-4(bis(4-méthylbenzo[5,6,17,18](O,N,N',O')-]-1,7,16,-triaza-cryptand-[3,2,2]-phén-4-yl]-1H-1.2.3-triazol1-yl}1,3,7,9-tétraméthyl-5H-dipyrrolo)(1,2-c:1',2'-f)(1,3,2)diazaborinin-4-ium-5-uide,
le 5,5-difluoro-10-(4-{1-[3-(2-méthoxyéthoxy)-4(bis(4-méthylbenzo[5,6,17,18](O,N,N',O')-]-1,7,16,-triaza-cryptand-[3,2,2]-phén-4-yl]-1H-1.2.3-triazol-4-yl}1,3,7,9-tétraméthyl-5H-dipyrrolo)(1,2-c:1',2'-f)(1,3,2)diazaborinin-4-ium-5-uide,
le 10-(4-{4-[4-(1,4,7,10-tétraoxa-13-azacyclopentadécan-13-yl)phényl]-1H-1,2,3-triazol-1-yl}phényl)-5,5-difluoro-1,3,7,9-tétraméthyl-5H-dipyrrolo[1,2-c:1',2'-f][1,3,2]diazaborinin-4-ium-5-uide,
le 10-(4-{1-[4-(1,4,7,10-tétraoxa-13-azacyclopentadécan-13-yl)phényl]-1H-1,2,3-triazol-4-yl}phényl)-5,5-difluoro-1,3,7,9-tétraméthyl-5H-dipyrrolo[1,2-c:1',2'-f][1,3,2]diazaborinin-4-ium-5-uide,
le 5,5-difluoro-10-(4-{4-[3-méthoxy-4-(1,4,7,10-tétraoxa-13-azacyclopentadécan-13-yl)phényl]-1H-1,2,3-triazol-1-yl}phényl)-1,3,7,9-tétraméthyl-5H-dipyrrolo[1,2-c:1',2'-f][1,3,2]diazaborinin-4-ium-5-uide,
le 5,5-difluoro-10-(4-{1-[3-méthoxy-4-(1,4,7,10-tétraoxa-13-azacyclopentadécan-13-yl)phényl]-1H-1,2,3-triazol-4-yl}phényl)-1,3,7,9-tétraméthyl-5H-dipyrrolo[1,2-c:1',2'-f][1,3,2]diazaborinin-4-ium-5-uide,
le 5,5-difluoro-10-(4-[3-méthoxy-4{bis(4-méthylbenzo[5,6,17,18](O,N,N',O')-]-1,7,16,-triaza-cryptand-[3,2,1]-phén-4-yl]-1H-1.2.3-triazol-1-yl}-(1,3,7,9-tétraméthyl-5H-dipyrrolo)(1,2-c:1',2'-f)(1,3,2)diazaborinin-4-ium-5-uide, et
le 5,5-difluoro-10-(1-[3-méthoxy-4{bis(4-méthylbenzo[5,6,17,18](O,N,N',O')-]-1,7,16,-triaza-cryptand-[3,2,1]-phén-1-yl]-1H-1.2.3-triazol-1-yl}-(1,3,7,9-tétraméthyl-5H-dipyrrolo)(1,2-c:1',2'-f)(1,3,2)diazaborinin-4-ium-5-uide.

**7.** Complexe constitué d'un composé selon la revendication 1 et d'un cation de métal alcalin.

**8.** Complexe selon la revendication 7, dans lequel le cation de métal alcalin est choisi dans le groupe constitué par $Na^+$ et $K^+$.

**9.** Procédé de détermination de cations métalliques dans un échantillon, comprenant les étapes de

a) mise en contact des cations métalliques avec au moins un composé selon la revendication 1,
b) formation d'un complexe selon la revendication 7, après quoi une fluorescence et/ou une luminescence apparait ou change,
c) mesure de la fluorescence et/ou la luminescence résultante.

**10.** Procédé selon la revendication 9, dans lequel les cations métalliques sont $Na^+$ ou $K^+$, ou une combinaison de ceux-ci.

**11.** Procédé selon la revendication 9, dans lequel le ou les composés selon la revendication 1 complexent sélectivement les cations métalliques à déterminer.

**12.** Utilisation d'un composé selon la revendication 1 pour la détermination qualitative et/ou quantitative de cations métalliques dans un échantillon.

*Fig.1*

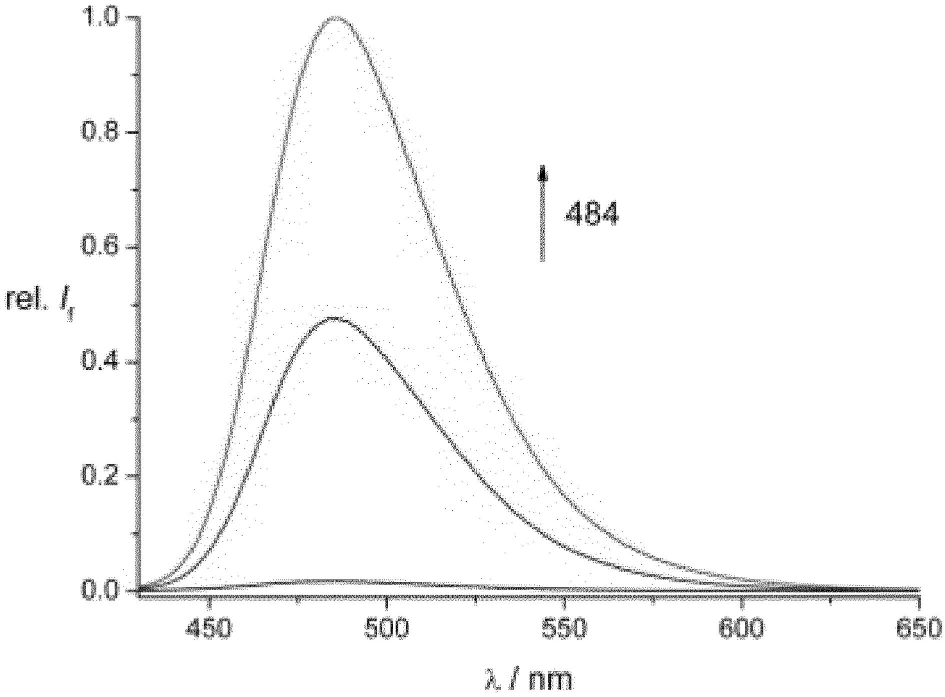

***Fig. 2a***

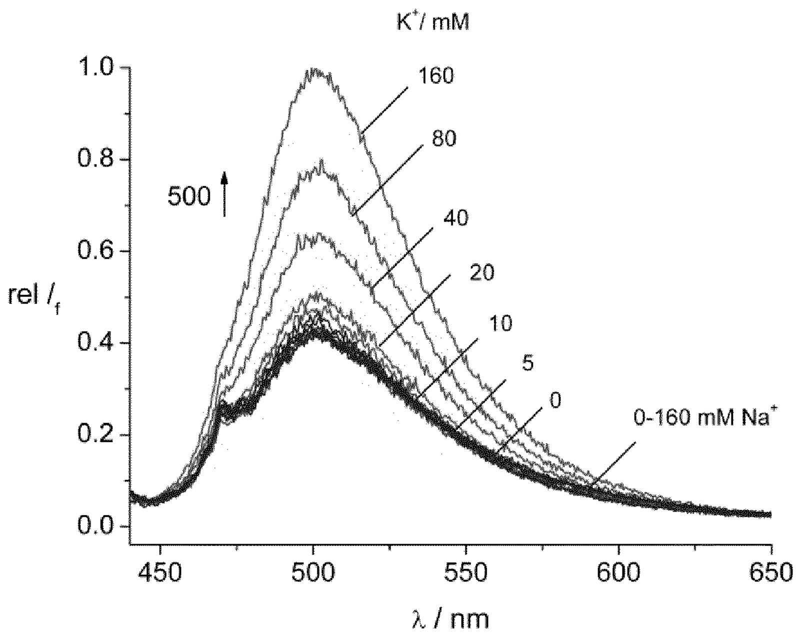
K⁺/ mM
1.0
0.8
0.6
0.4
0.2
0.0
500
rel $I_f$
160
80
40
20
10
5
0
0-160 mM Na⁺
450
500
550
600
650
λ / nm

***Fig. 2b***

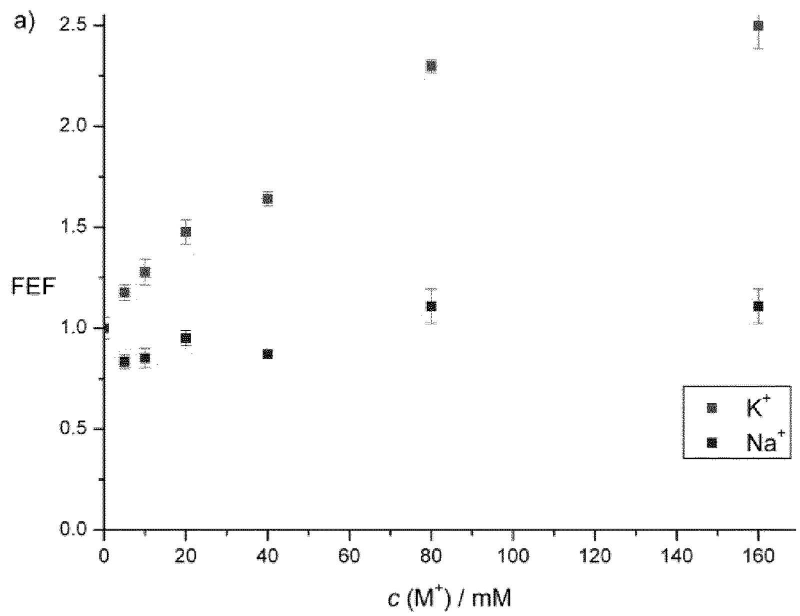
a)
2.5
2.0
1.5
1.0
0.5
0.0
FEF
K⁺
Na⁺
0
20
40
60
80
100
120
140
160
c (M⁺) / mM

*Fig. 3*

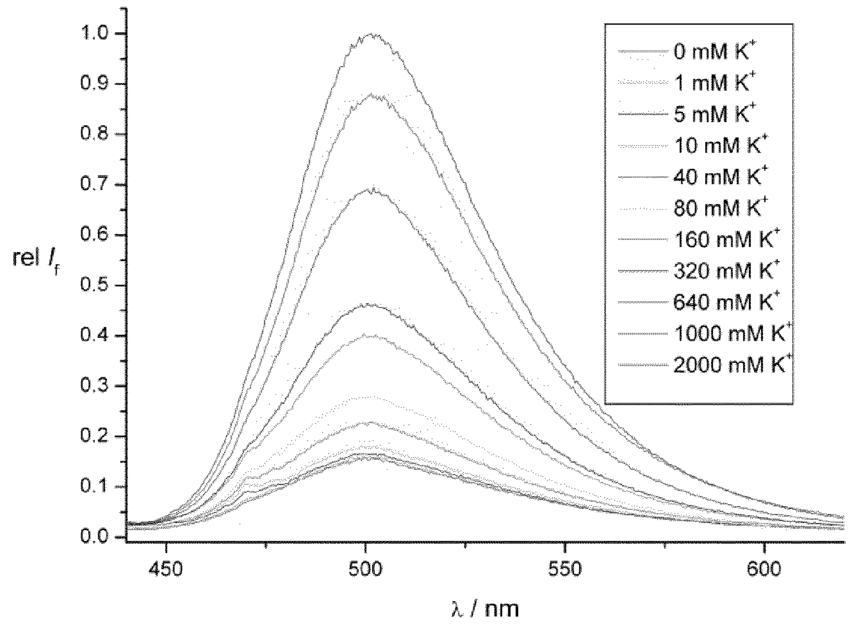
rel $I_f$
λ / nm
0 mM K+
1 mM K+
5 mM K+
10 mM K+
40 mM K+
80 mM K+
160 mM K+
320 mM K+
640 mM K+
1000 mM K+
2000 mM K+

*Fig. 4*

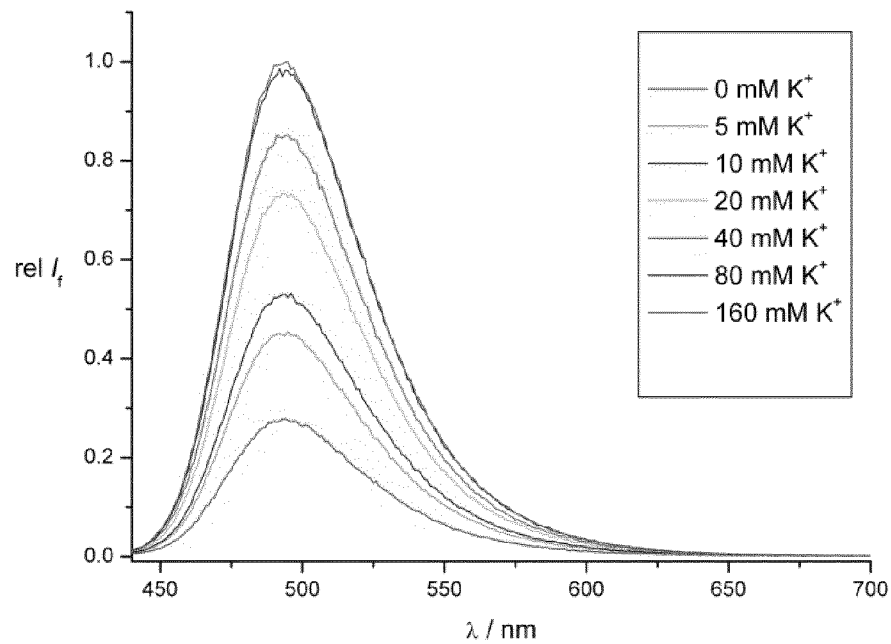
rel $I_f$
λ / nm
0 mM K+
5 mM K+
10 mM K+
20 mM K+
40 mM K+
80 mM K+
160 mM K+

***Fig. 5a***

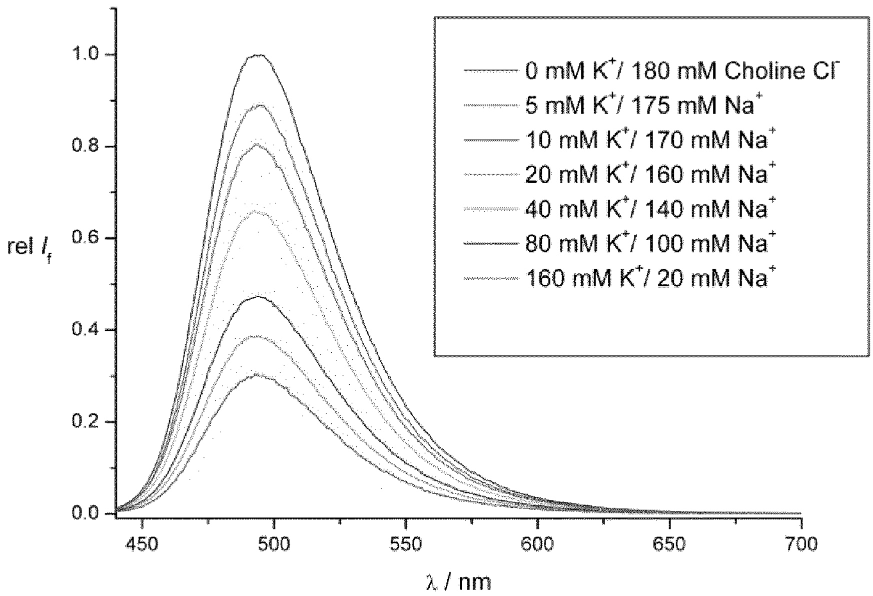
0 mM K+/ 180 mM Choline Cl-
5 mM K+/ 175 mM Na+
10 mM K+/ 170 mM Na+
20 mM K+/ 160 mM Na+
40 mM K+/ 140 mM Na+
80 mM K+/ 100 mM Na+
160 mM K+/ 20 mM Na+
rel $I_f$
λ / nm

***Fig. 5b***

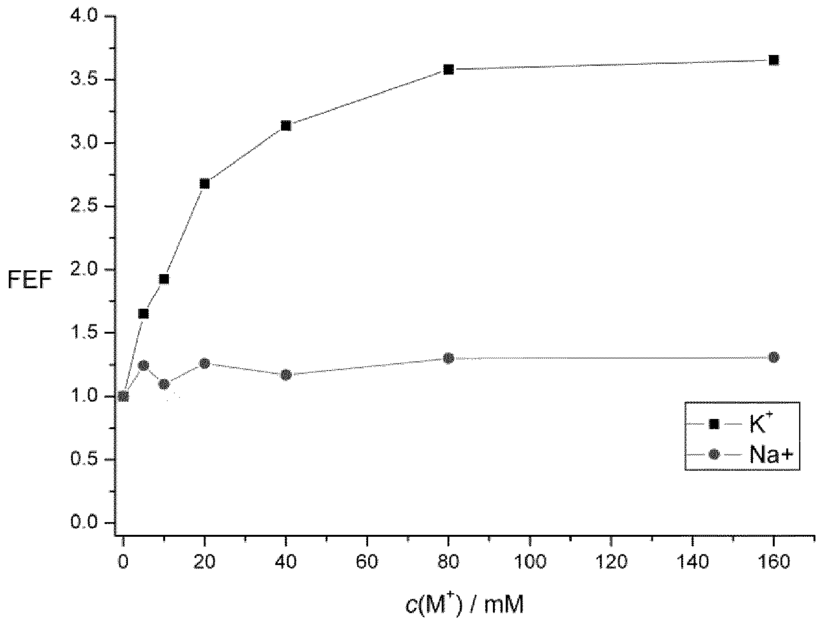
FEF
K+
Na+
c(M+) / mM

*Fig. 6*

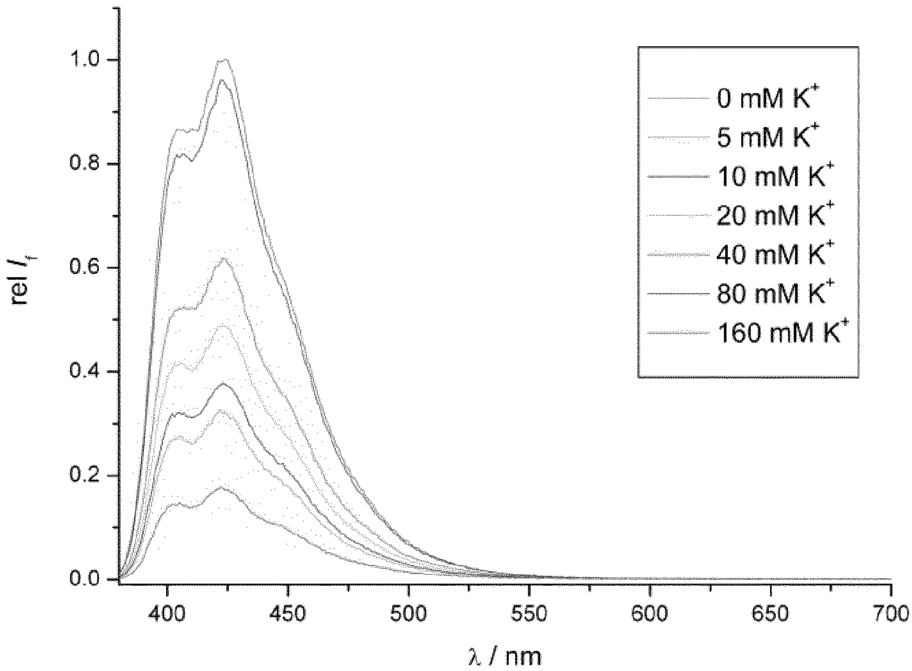
rel $I_f$
1.0
0.8
0.6
0.4
0.2
0.0
400
450
500
550
600
650
700
λ / nm
0 mM K+
5 mM K+
10 mM K+
20 mM K+
40 mM K+
80 mM K+
160 mM K+

*Fig. 7a*

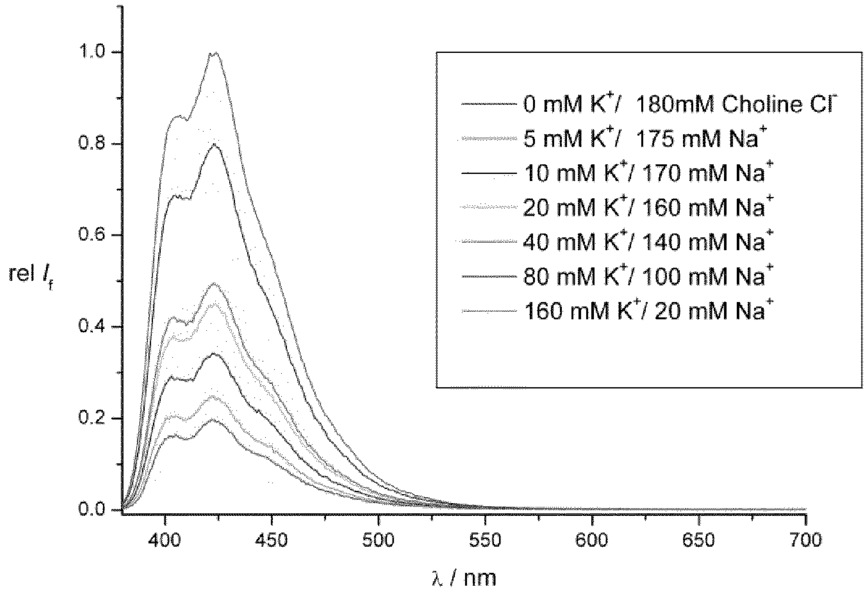
1.0
0.8
0.6
0.4
0.2
0.0
rel $I_f$
0 mM $K^+$/ 180mM Choline $Cl^-$
5 mM $K^+$/ 175 mM $Na^+$
10 mM $K^+$/ 170 mM $Na^+$
20 mM $K^+$/ 160 mM $Na^+$
40 mM $K^+$/ 140 mM $Na^+$
80 mM $K^+$/ 100 mM $Na^+$
160 mM $K^+$/ 20 mM $Na^+$
400
450
500
550
600
650
700
λ / nm

*Fig. 7b*

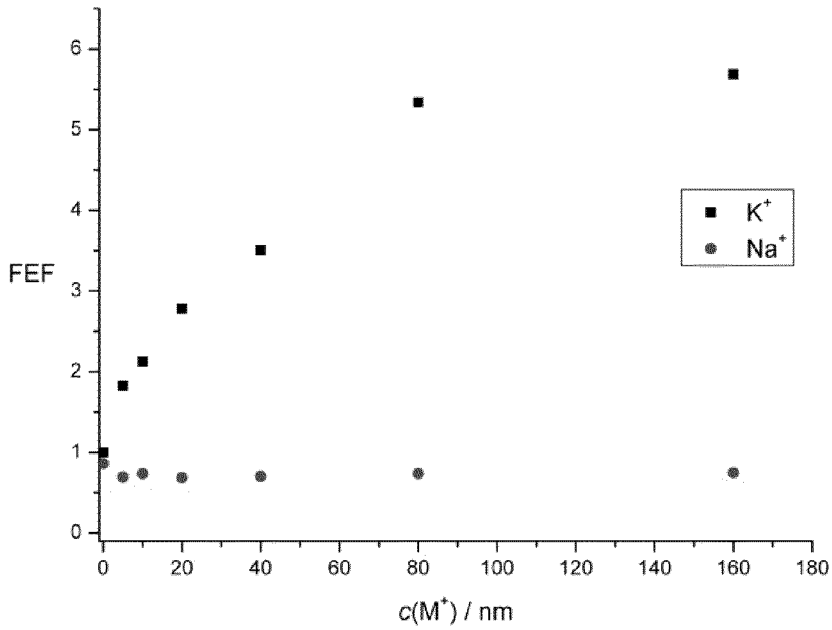
6
5
4
3
2
1
0
FEF
$K^+$
$Na^+$
0
20
40
60
80
100
120
140
160
180
$c(M^+)$ / nm

*Fig. 8*

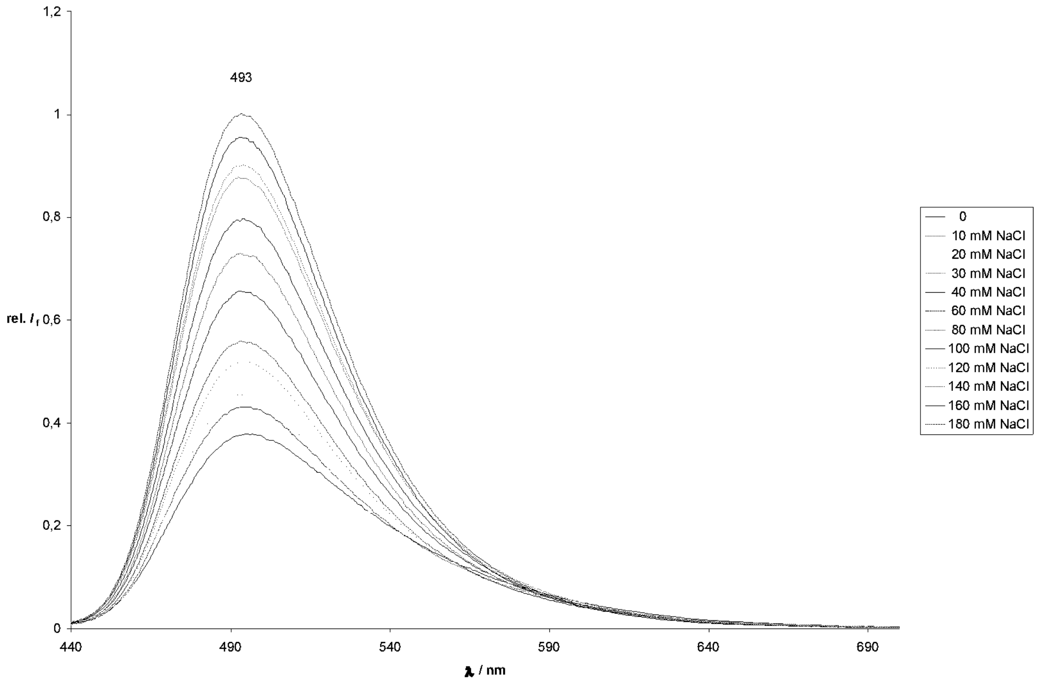

493
rel. $I_f$
λ / nm
0
10 mM NaCl
20 mM NaCl
30 mM NaCl
40 mM NaCl
60 mM NaCl
80 mM NaCl
100 mM NaCl
120 mM NaCl
140 mM NaCl
160 mM NaCl
180 mM NaCl


*Fig. 9*

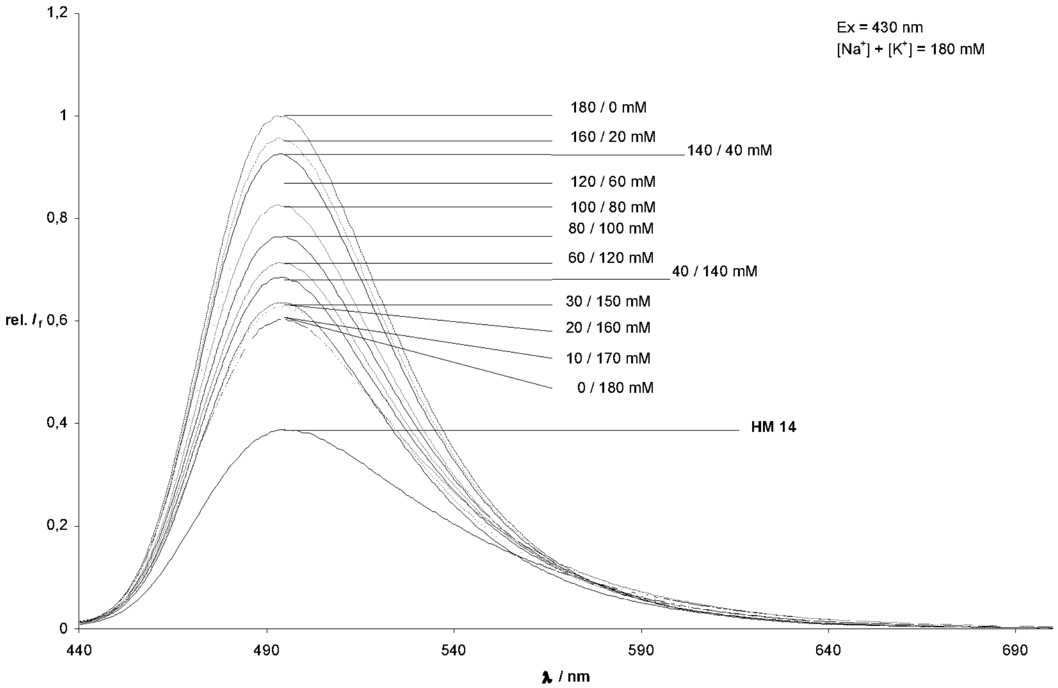

Ex = 430 nm
[Na+] + [K+] = 180 mM
180 / 0 mM
160 / 20 mM
140 / 40 mM
120 / 60 mM
100 / 80 mM
80 / 100 mM
60 / 120 mM
40 / 140 mM
30 / 150 mM
20 / 160 mM
10 / 170 mM
0 / 180 mM
HM 14
rel. $I_f$
λ / nm

*Fig. 10*

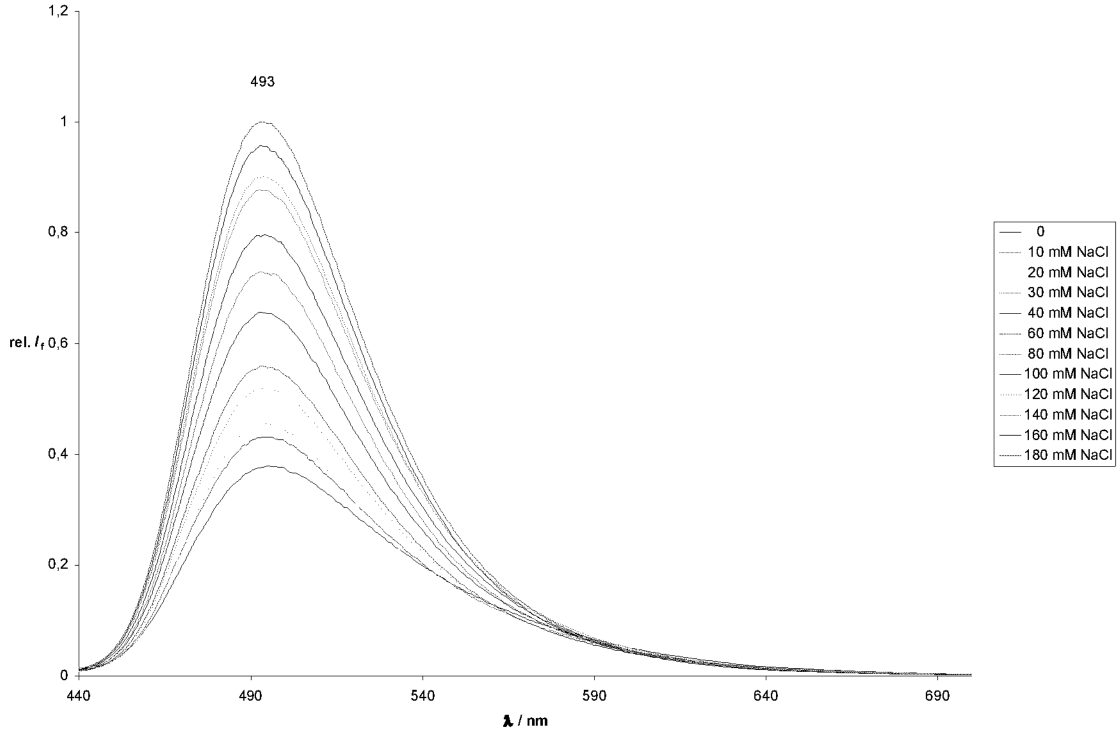
493
rel. $I_f$
1,2
1
0,8
0,6
0,4
0,2
0
440
490
540
590
640
690
λ / nm
0
10 mM NaCl
20 mM NaCl
30 mM NaCl
40 mM NaCl
60 mM NaCl
80 mM NaCl
100 mM NaCl
120 mM NaCl
140 mM NaCl
160 mM NaCl
180 mM NaCl

*Fig. 11*

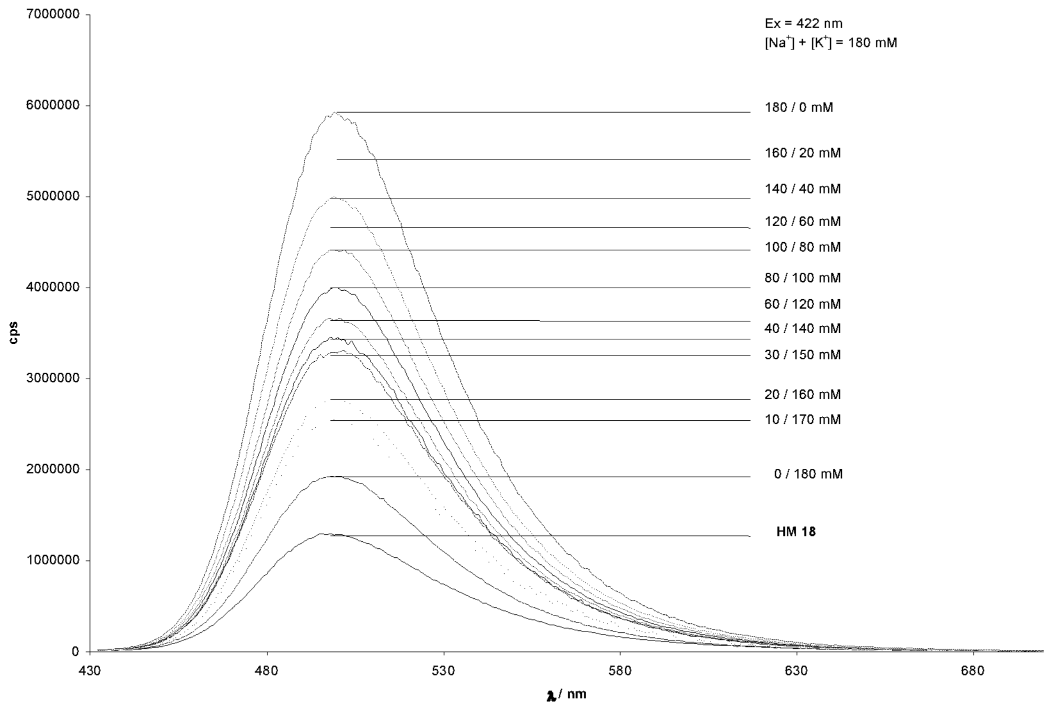
Ex = 422 nm
$[Na^+] + [K^+]$ = 180 mM
cps
7000000
6000000
5000000
4000000
3000000
2000000
1000000
0
430
480
530
580
630
680
λ / nm
180 / 0 mM
160 / 20 mM
140 / 40 mM
120 / 60 mM
100 / 80 mM
80 / 100 mM
60 / 120 mM
40 / 140 mM
30 / 150 mM
20 / 160 mM
10 / 170 mM
0 / 180 mM
HM 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0881487 A2
- WO 20070044866 A2
- US 6211359 B1
- US 20070259443 A1
- US 20070259444 A1


### Non-patent literature cited in the description


- **E. TAMANINI ; A. KATEWA ; L. M. SEDGER ; M. H. TODD ; M. WATKINSON.** *Inorg. Chem,* 2009, vol. 48, 319
- **E. TAMANINI ; K. FLAVIN ; M. MOTEVALLI ; M. H. TODD ; M. WATKINSON.** *Inorg. Chem.,* 2010, vol. 49, 3798
- **S.HUANG ; R. J. CLARK ; L. ZHU.** *Org. Lett.,* 2007, vol. 9, 4999
- **S. MAISONNEUVE ; Q. FANG ; J. XIE.** *Tetrahedron,* 2008, vol. 64, 8716
- **K.VARAZO ; F. XIE ; D. GULLEDGE ; Q. WANG.** *Tetrahedron Lett.,* 2008, vol. 49, 5293
- **H.-C. HUNG ; C.-W. CHENG ; Y.-Y. WANG ; Y.-J. CHEN ; W.-S. CHUNG.** *Eur. J. Org. Chem.,* 2009, vol. 15, 6360
- **D. MAITY ; T. GOVINDARAJU.** *Inorg. Chem.,* 2010, vol. 49, 7229
- **P. D. JAROWSKI ; Y.-L. WU ; B. SCHWEIZER ; F. DIEDERICH.** *Org. Lett.,* 2008, vol. 10, 3347
- **J. D. LEWIS ; J. M. MOORE.** *Dalton Trans.,* 2004, 1376
- **K. SIVAKUMAR ; F. XIE ; Q. WANG.** *Org. Lett.,* 2004, vol. 6, 4603
- **J. W. SIBERT ; V. LYNCH.** *Inorg. Chem.,* 2007, vol. 46, 10913
- **J. W. SIBERT ; P. B. FORSHEE.** *Inorg. Chem.,* 2002, vol. 41 (23), 5928
- **D.-N. LEE ; G.-J. KIM ; H.-J. KIM.** *Tetrahedron Lett.,* 2009, vol. 50, 4766
- **E. TAMANINI ; S. E. J. RIGBY ; M. MOTEVALLI ; M. H. TODD ; M. WATKINSON.** *Chem. Eur., J.,* 2009, vol. 15, 3720
- **H. HE ; M. A. MORTELLARO ; M. J. P. LEINER ; R. J. FRAATZ ; J. K. TUSA.** *J. Am. Chem. Soc.,* 2003, vol. 125, 1468
- **P. PADMAWAR ; X. YAO ; O. BLOCH ; G. T. MANLEY ; A. S. VERKMAN.** *Nat. Methods.,* 2005, vol. 2, 825
- **R. D. CARPENTER ; A. S. VERKMAN.** *Org. Lett.,* 2010, vol. 12, 1160
- **H. HE ; M. A. MORTELLARO ; M. J. P. LEINER ; S. T. YOUNG ; R. J. FRAATZ ; J. K. TUSA.** *Anal. Chem.,* 2003, vol. 75, 549
- **K. SIVAKUMAR ; F. XIE ; B. M. CASH ; S. LONG ; H. N. BARNHILL ; Q. WANG.** *Org. Lett.,* 2004, vol. 6, 4603-4606
- **J.-S. WU ; W.-M. LIU ; X.-Q. ZHUANG ; F. WANG ; S.-T. LEE.** *Org. Lett.,* 2007, vol. 9, 33-36
- **D.-N. LEE ; G.-J. KIM ; H.-J. KIM.** *Tetrahedron Lett.,* 2000, vol. 50, 4766-4768
- **D. JIMENEZ ; R. MARTÍNEZ-MÁÑEZ ; F. SANCENÓN ; E.GARCÍA-BREIJO.** *Eur. J. Inorg. Chem.,* 2005, 2393-2403
- **J. P. DIX ; F. VÖGTLE.** *Chem. Ber.,* 1980, vol. 113, 457
- **J. D.LEWIS ; J. M. MOORE.** *Dalton Trans.,* 2004, 1376-1385
- **J. W. SIBERT ; V. LYNCH.** *Inorg. Chem.,* 2007, vol. 46, 10913-10925
- **P. A. S. SMITH ; J. H. BOYER.** *Org. Synth.,* 1963, vol. 4, 74
- **E. TAMANINI ; S. E. J. RIGBY ; M. MOTEVALLI ; M. H. TODD ; M. WATKINSON.** *Chem. Eur. J.,* 2009, vol. 15, 3720-3728
- **M. HIROKAZU ; S. FURUYOSHI ; Y. NAKATSUJI ; M. OKAHARA.** *Bull. Chem. Soc. Jpn.,* 1983, vol. 56, 212-218
- **R. WORTMANN ; C. GLANIA ; P. KRÄMER ; R. MATSCHINER ; J. J. WOLFF ; S. KRAFT ; B. TREPTOW ; E. BARBU ; D. LÄNGLE ; G. GÖRLITZ.** *Chem. Eur. J.,* 1997, vol. 3, 1765-1773
- *Synlett,* 1996, 521-522
- *Chem. Commun.,* 2011, vol. 47, 4685-4687
- **HUIMIN GUO ; MARIA L. MURO-SMALL ; SHAOMIN JI ; JIANZHANG ZHAO ; FELIX N. CASTELLANO.** *Inorg. Chem.,* 2010, vol. 49, 6802-6804
- **T. GUNNLAUGSSON ; PAUL. E. KRUGER ; G. M. HUSSEY.** *J. of. Org. Chem.,* 2005, vol. 70 (25), 10875-10878
- **Q. XIAO ; T. BROWN.** *Tetrahedron,* 2007, vol. 63, 3483

- **K.-C. CHANG ; I.-H. SU ; A. SENTHILVELAN ; W.-S. CHUNG.** *Org. Lett.,* 2007, vol. 9 (17), 3363-3366
- **A. TORRADO ; B, IMPERIALI.** *J. Org. Chem.,* 1996, vol. 61, 8940-8948
- **LIU et al.** *Journal of Polymer Science: Part A: Polymer Chemistry,* 2001, vol. 39, 1495-1504
- **KREICBERGA. E. ; JECS. V. KAMPARS.** Serija 1: Materialzinatne un Lietiska Kimija. Rigas Tehniskas Universitates Zinatniskie Raksti, 2005, vol. 10, 46-53
- **H. HE ; J. K. TUSA.** *J. Am. Chem. Soc.,* 2003, vol. 125, 1468-1469
- **D. JIMÉNEZ ; E.GARCÍA-BREIJO.** *Eur. J. Inorg. Chem.,* 2005, 2393-2403
- **T. GUNNLAUGSSON ; M. NIEUWENHUYZEN.** *J. Chem. Soc. Trans.,* 2002, vol. 1, 1954-1962
- **H. GUO ; F. N. CASTELLANO.** *Inorg. Chem.,* 2010, vol. 49, 6802-6804